# EUROPEAN PATENT APPLICATION

(11) **EP 2 071 336 A1**
(43) Date of publication of application: **17.06.2009**
(21) Application number: 07076092.1
(22) Date of filing: 13.12.2007
(51) Int. Cl.: G01N 33/574, C12Q 1/40

(54) **Serum proteomic for finding diagnostic markers and for monitoring therapeutical intervention in treatment of hepatocellular carcinoma**

(71) Applicant: Fraunhofer-Gesellschaft zur Förderung der angewandten Forschung e.V., 80686 München (DE)
(72) Inventor: Jürgen Borlak, 31275 Lehrte OT Immense, Berlin (DE); Guiseppe Gazzana,, 30625 Hannover, Ber4lin (DE)
(74) Representative: Baumbach, Friedrich

(57) **Abstract**

The invention is directed to biomarkers for determining the EGFR kinase activity in a subject, and the use thereof for predicting and monitoring therapeutic intervention in cancer patients. Areas of application are the life sciences: biology, biochemistry, biotechnology, medicine and medical technology.

The biomarkers are selected from a first group consisting of Amy 1, Apo Al, Carbx, Casp, AFP, ApoM, SAP, Fib-a, Fib-b, Fib-g, ApoE, A2MG, A2MG isoform, Serpin, Clusterin, MHC-fB, SAP isoform, or from a second group consisting of Gpx3, properidin, MUP1, HMW-K, Lifr-p, Orm 1, MBL-A, MBP-C, wherein the biomarkers are regulated by EGF overexpression in a subject.

## Description

The invention is directed to biomarkers for determining the EGFR kinase activity in a subject, and the use thereof for predicting and monitoring therapeutic intervention in cancer patients. Areas of application are the life sciences: biology, biochemistry, biotechnology, medicine and medical technology.

The epidermal growth factor receptor (EGFR) plays an important role in various tumor diseases. Its hyperactivity can cause cancer of numerous organs, e.g. epithelial tumors of the lung, colon, breast, head and neck, ovarian, and liver (HCC). The hepatocellular carcinoma (HCC), for instance, ranks fifth among the most common malignant tumors worldwide and is the third leading cause of cancer-related deaths. Thereby, the number of HCC diseases in Europe and the United States steadily increases, as exemplarily demonstrated in the north east of Germany, where the number of HCC-incidences of males arose from 3.6% in 1976 to 5.7% in 2002 . Chronic liver disease, which lead to cirrhosis and infection with hepatitis C are important risk factors for HCC. Overall, the formation of HCC is a multistage process, whereby the latest findings from cancer research evaluate the dysregulation of tumor progenitor genes as a predisposing or initial event leading to an epigenetic modification of progenitor or stem cells. Then, the risk of malignant transformation becomes dramatically increased by mutations in such tumor suppressor- and proto-oncogenes .Finally, the malignant transformation and the invasive tumor growth is massively promoted by epigenetic instability. Despite numerous findings the molecular basis of hepatocellular carcinoma remains insufficiently known.

Recently, the consequences of excessive EGF-signal transmission in liver cancer has been reported. This growth factor binds to and induces EGFR activity. Activated EGFR phosphorylates many proteins which are networked by signal transduction chains and therefore favour the emergence and further development of tumor malignancies. In particular, various tyrosine residues are phosphorylated, which subsequently act as docking sites for a number of proteins. In this way, EGFR is involved in diverse signaling pathways, including the mitogen-activated protein kinases (MAPK). This in turn has implications for the fate of the cell, leading to exaggerated proliferation, the lack of differentiation and migration of transformed tumor cells.

Since the EGF-receptor tyrosine kinase (RTK) plays a key role in malignant tumor diseases, therapeutic antibodies and small molecules, also termed as "EGFR kinase modulators", that counter an increased activity of EGFR are frequently used for the treatment of cancer. However, besides to the EGF-signalling cascade further signalling pathways are involved in the formation and growth of cancer such as, e.g., *Wnt-*β*-Catenin, Hedgehog* and other receptor tyrosine kinases.
Thus, there is a need for new prognostic and predictive biomarkers and methods for easily identifying the indication "EGFR hyperactivity" or "EGFR overexpression", repectively, in a subject suffering from or being susceptile to cancer, for accurately predicting and monitoring the response of the subject to a treatment with EGFR modulators, thereby enabeling an individualized cancer treatment of the subject for enhancing its chances of survival.

The aim of the present invention is therefore to provide biomarkers, compositions and a kit, as well as a method for a fast, easy and efficient qualification or quantification of the EGFR kinase activity status of a subject suffering from or being susceptible to cancer, in particular for predicting and monitoring the response of a cancer patient to the treatment with an EGFR activity modulator.

To this end, the implementation of the embodiments and actions as described in the claims provides appropriate means to fulfill these demands in a satisfying manner.

The invention is based on the surprising finding that biomarkers selected from a first group consisting of Amy 1, Apo Al, Carbx, Casp, AFP, ApoM, SAP, Fib-a, Fib-b, Fib-g, ApoE, A2MG, A2MG isoform, Serpin, Clusterin, MHC-fB, SAP isoform, or from a second group consisting of Gpx3, properidin, MUP1, HMW-K, Lifr-p, Orm 1, MBL-A, MBP-C, are regulated by EGF overexpression in subjects suffering from or being susceptilbe to cancer.

The biomarkers according to the invention concern gene products of mammalia, preferably gene products of the genome of *mus musculus* or *homo sapiens,* in particular the respective gene products of *homo sapiens* are preferred.

Within the context of the invention, the term "subject" is directed to a mammal, in particular to a mouse or a human being suffering from or being susceptible to cancer, more particular to a human cancer patient or a transgenic cancer mouse, such as a HCC patient or a EGF-transgenic mouse may be.

The invention further concerns a composition for qualifying the EGFR kinase activity in a subject suffering from or being susceptible to cancer, in particular by an *in vitro* body fluid analysis, wherein the composition comprises an effective amount of at least one biomarker selected from the first group of said biomarkers or an effective amount of at least one biomarker selected from the second group of said biomarkers.

In one embodiment of the invention, the biomarker is preferably selected from a first group consisting of Amy 1, Apo Al, Carbx, Casp, Fib-a, Fib-b, Fib-g, Clusterin, MHC-fB, SAP isoform or from a second group consisting of HMW-K, Lifr-p, Orm 1, MBL-A, MBP-C.

In another preferred embodiment, the biomarker is selected from a first group consisting of AFP, ApoE, ApoM, or from a second group consisting of Gpx3, A2MG, A2MG isoform, SAP.

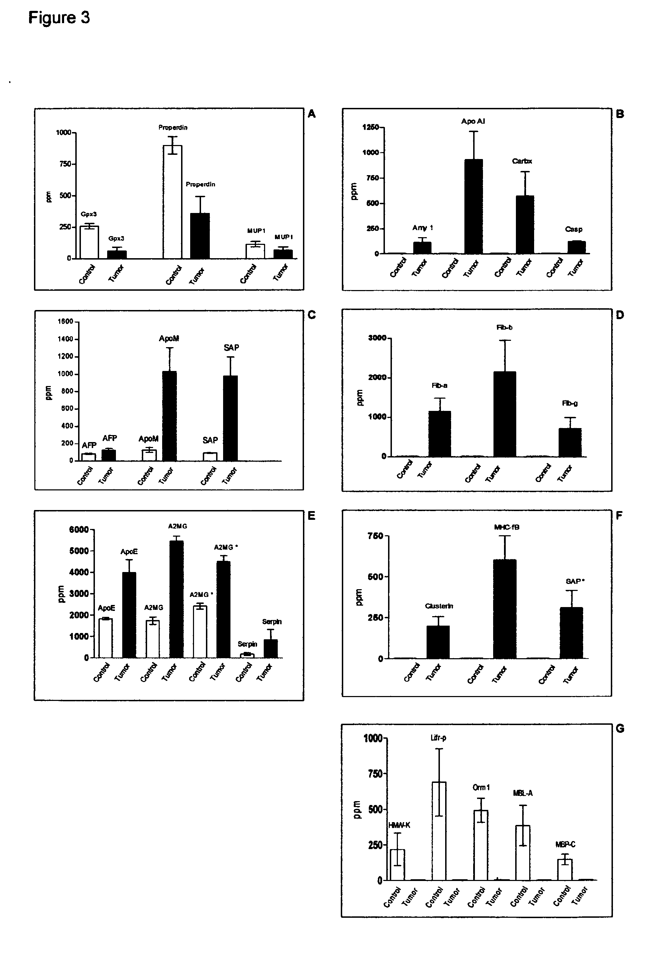

In particular, it is preferred, if the composition according to the invention comprises an effective amount of at least one biomarker selected from the first group of said biomarkers and an effective amount of at least one biomarker selected from the second group of said biomarkers, wherein the combination
(a) biomarker selected from the group consisting of Amy 1, Apo Al, Carbx, Casp, Fib-a, Fib-b, Fib-g, Clusterin, MHC-fB, SAP isoform and biomarker selected from the group consisting of HMW-K, Lifr-p, Orm 1, MBL-A, MBP-C or the combination
(b) biomarker selected from the group consisting of AFP, ApoE, ApoM and biomarker selected from the group consisting of Gpx3, A2MG, A2MG isoform, SAP is particularly preferred.
   In another preferred embodiment the composition further comprises an effective amount of a biomarker selected from the group of EGF, thus allowing an easy calibration of the system.
   In yet another preferred embodiment, the composition according to the invention further comprises an effective amount of a protease, in particular of trypsin, thus enabling a further enhancement of the system sensitivity.
   The composition according to the invention, in particular the protease digest thereof, may be preferably used for producing a vaccine for the immunization of an animal in order to produce polyclonal antibodies specific for the at least one biomarker.
   Another aspect of the invention concerns the use of the composition according to the invention for the production of a diagnostic agent, in particular of a diagnostic standard for *in vitro* body fluid analyses.
   The term "body fluid" according to the invention is directed to any body fluid of a subject, in particular to blood, plasma, serum or urine, whereas serum is the preferred body fluid within the context of the invention. The term "diagnostic agent" as used herein relates to any solution, suspension or solid formulation, containing said composition in an acceptable amount for diagnostic purposes.
   In particular, the composition is used for the production of a diagnostic agent for qualifying the EGFR kinase activity in a subject suffering from or being susceptible to cancer, preferably cancer of the liver, lung, breast, colon, prostate, bladder, head and neck, ovary or brain, in particular in a subject suffering from or being susceptible to HCC.
   In a further preferred embodiment, the composition according to the invention is used for the production of a diagnostic agent for predicting or monitoring the response of a cancer patient to a method of treating cancer comprising administering an EGFR kinase modulator to the patient.
   In yet another aspect, the invention provides a kit for qualifying the EGFR kinase activity in a subject suffering from or being susceptible to cancer, in particular for predicting or monitoring the response of a cancer patient to a method of treating cancer comprising administering an EGFR kinase modulator, wherein the kit comprises at least one standard (1) indicative of the body fluid level of a biomarker selected from the first group of said biomarkers in normal individuals or individuals having cancer associated with increased EGFR kinase activity and/or at least one standard (2) indicative of the body fluid level of a biomarker selected from the second group of said biomarkers in normal individuals or individuals having cancer associated with increased EGFR kinase activity, and instructions for the use of the kit.
   In a preferred embodiment of the kit, the standard (1) comprises an indicative amount of at least one biomarker selected from the first group of said biomarkers and/or the at least one standard (2) comprises an indicative amount of at least one biomarker selected from the second group of said biomarkers.
   In another preferred embodiment, the kit comprises a mixture of the at least one standard (1) and the at least one standard (2), in particular a composition according to the invention comprising an effective amount of at least one Figure 3 from the first group of said biomarkers and an effective amount of at least one biomarker selected from the second group of said biomarkers, wherein the set of biomarkers according to combination (a) or combination (b), as described herein, is particularly preferred.
   In yet another preferred embodiment, the kit according to the invention further comprises a lysis buffer, wherein the lysis buffer comprises (a) at least one buffer component, (b) at least one chaotrope, (c) at least one detergens, (d) at least one reducing agent (e) at least one carrier ampholyte, and (f) at least one ribonuclease, Preferably, the lysis buffer is an aqueous solution of (a) at least one buffer compound selected from the group consisting of Tris and HEPES, (b) at least one chaotrope selected from the group consisting of urea and thiourea, (c) at least one detergens selected from the group consisting of CHAPS and SDS, (d) at least one reducing agent selected from the group consisting of DTT and TCEP, (e) at least one carrier ampholyte selected from the group consisting of biolyte 5-7 and biolyte 3-10, and (f) at least one ribonuclease selected from the group consisting of endonuclease and exonuclease, wherein an aqueous solution of (a) Tris; (b) urea and thiourea, (c) CHAPS, (d) DTT, (e) biolyte 3-10, and (f) endonuclease, is particularly preferred.

In one preferred embodiment, the kit according to the invention further comprises at least one antibody specific for a biomarker selected from the first group of said biomarkers and/or at least one antibody specific for a biomarker selected from the second group of said biomarkers, and reagents effective to detect said biomarker(s) in a serum sample, such as buffers for dissolving or equilibrating the standard (1) and/or the standard (2), or an enzyme substrate for imaging enzyme labels may be. In particular, a kit is preferred, comprising at least one antibody specific for a biomarker selected from the group consisting of Amy 1, Apo Al, Carbx, Casp, Fib-a, Fib-b, Fib-g, Clusterin, MHC-fB, SAP isoform and/or at least one antibody specific for a biomarker selected from the group consisting of HMW-K, Lifr-p, Orm 1, MBL-A, MBP-C.
More particular, it is preferred, if the at least one antibody is polyclonal, thus allowing a further enhancement of the system sensitivity.
Advantageously, the kit further comprises at least one labelled secondary antibody specific for the at least one antibody, thus allowing a fast screening of the binding of the at least one antibody to the at least one biomarker, in particular if the at least one biomarker or the digest thereof is immobilized to a solid phase support, such as nitrocellulose may be.

In a further aspect, the invention provides a method of qualifying the EGFR kinase activity in a subject, comprising determining in a body fluid sample of a subject suffering from or being susceptible to cancer at least one biomarker selected from the first group of said biomarkers and/or at least one biomarker selected from the second group of said biomarkers, wherein the body fluid level of the at least one biomarker of said first group being significantly higher and/or the body fluid level of the at least one biomarker of said second group being significantly lower than the level of said biomarker(s) in the body fluid of subjects without cancer, in particular without cancer associated with increased activity of EGFR, is indicative of induced EGFR kinase activity in the subject.

In particular, it is preferred, if the method comprises determining at least one biomarker selected from the first group of said biomarkers and at least one biomarker selected from the second group of said biomarkers, wherein the body fluid level of the at least one biomarker of said first group being significantly higher and the body fluid level of the at least one biomarker of said second group being significantly lower than the level of said biomarkers in the body fluid of subjects without cancer, in particular without cancer associated with increased activity of EGFR, is indicative of induced EGFR kinase activity in the subject, preferably if a combination of a biomarker selected from the group consisting of Amy 1, Apo Al, Carbx, Casp, Fib-a, Fib-b, Fib-g, Clusterin, MHC-fB, SAP isoform and a biomarker selected from the group consisting of HMW-K, Lifr-p, Orm 1, MBL-A, MBP-C or a combination of a biomarker selected from the group consisting of AFP, ApoE, ApoM and a biomarker selected from the group consisting of Gpx3, A2MG, A2MG isoform, SAP is determined.

Preferably, the method according to the invention is carried out for predicting the response of a cancer patient to a method of treating cancer comprising administering an EGFR kinase modulator, wherein the body fluid level of the at least one biomarker of said first group being significantly higher and/or the body fluid level of the at least one biomarker of said second group being significantly lower than the level of said biomarker(s) in the body fluid of subjects without cancer, in particular without cancer associated with increased activity of EGFR, is indicative that the subject will respond therapeutically to a method of treating cancer comprising administering an EGFR kinase modulator.

In one embodiment, the method is implemented for monitoring the therapeutically response of a cancer patient to a method of treating cancer comprising administering an EGFR kinase modulator, wherein the body fluid level of the at least one biomarker of said first group before and after the treatment and/or the body fluid level of the at least one biomarker of said second group before and after the treatment is determined, and a significant decrease of said body fluid level(s) of the at least one biomarker of said first group and/or a significant increase of said body fluid level(s) of the at least one biomarker of said second group after the treatment is indicative that the cancer patient therapeutically responds to the administration of the EGFR kinase modulator.

In a preferred embodiment, the method is implemented by performing an immunoassay, such as an enzyme immunoassay (EIA), a radio immunoassay (RIA) or a fluorescence immunoassay (FIA) may be, in particular by using the kit according to the invention and/or by performing a western blot. Preferably, at least one antibody specific for a biomarker selected from the group consisting of Amy 1, Apo Al, Carbx, Casp, Fib-a, Fib-b, Fib-g, Clusterin, MHC-fB, SAP isoform and/or at least one antibody specific for a biomarker selected from the group consisting of HMW-K, Lifr-p, Orm 1, MBL-A, MBP-C is used for the immunoassay and/or reagents effective to detect said biomarker(s) in a serum sample, such as a blocking buffer for reducing unspecific antibody binding or an enzyme substrate for imaging enzyme labelled antibodies may be, is used for the immunoassay.

In another preferred embodiment, the method is implemented by performing a peptide mass fingerprinting, in particular by using the kit described herein.

Within the context of peptide mass fingerprinting, the method preferably comprises the steps of
- isolating a serum sample from a blood sample of a subject suffering from or being susceptible to cancer,
- adding lysis buffer to the serum sample;
- separating the proteins of the lysed serum sample by 2-DE gel electrophoresis;
- excising from the gel at least one sample containing a protein of interest;
- adding digesting buffer to the at least one excised sample, and
- determining the amount of the at least one protein of interest by analyzing the at least one digest mixture by mass spectrometry.

In one embodiment of the method, the subject is a human patient or a non-human transgenic animal, in particular suffering from or being susceptible to cancer, more particular suffering from or being susceptible to cancer of the liver, lung, breast, colon, prostate, bladder, head and neck, ovary or brain, such as a transgenic mouse, in particular a mouse whose genome comprises a non natural IgEGF sequence, may be.

In another embodiment of the method, the serum sample is isolated by centrifuging the blood sample;

In yet another embodiment of the method, the 2-DE is performed by using two different pH gradients, preferably by using the pH gradients 3-10 and 4-7.

In a further embodiment of the method, the lysis buffer comprises (a) at least one buffer component, (b) at least one chaotrope, (c) at least one detergens, (d) at least one reducing agent (e) at least one carrier ampholyte, and (f) at least one ribonuclease. Preferably, the lysis buffer used is an aqueous solution of (a) at least one buffer compound selected from the group consisting of Tris and HEPES, (b) at least one chaotrope selected from the group consisting of urea and thiourea, (c) at least one detergens selected from the group consisting of CHAPS and SDS, (d) at least one reducing agent selected from the group consisting of DTT and TCEP, (e) at least one carrier ampholyte selected from the group consisting of biolyte 5-7 and biolyte 3-10, and (f) at least one ribonuclease selected from the group consisting of endonuclease and exonuclease, wherein an aqueous solution of (a) Tris; (b) urea and thiourea, (c) CHAPS, (d) DTT, (e) biolyte 3-10, and (f) endonuclease, is particularly preferred.the lysis buffer comprises (a) at least one buffer component, (b) at least one chaotrope, (c) at least one detergens, (d) at least one reducing agent (e) at least one carrier ampholyte, (f) at least one ribonuclease is particularly preferred.

In yet a further embodiment of the method, the protein of interest is a biomarker selected from the first group of said biomarkers or is a biomarker selected from the second group of said biomarkers, in particular is selected from the first group consisting of Amy 1, Apo Al, Carbx, Casp, Fib-a, Fib-b, Fib-g, Clusterin, MHC-fB, SAP isoform or from the second group consisting of HMW-K, Lifr-p, Orm 1, MBL-A, MBP-C, or more preferably is selected from the first group consisting of AFP, ApoE, ApoM or from the second group consisting of Gpx3, A2MG, A2MG isoform, SAP.

In another embodiment of the method the digesting buffer comprises a bicarbonate compound and a protease, wherein the digesting buffer preferably is an aqueous solution of at least one bicarbonate compound selected from the group consisting of ammonium bicarbonate and sodium bicarbonate and of at least one serine protease, in particular selected from the group consisting of trypsin, chymotrypsin and elastase, or, in particular preferred, the digesting buffer is an aqueous solution of ammonium bicarbonate and trypsin.

In yet another embodiment of the method, the mass spectrometry is selected from the group consisting of MALDI-TOF and ESI-TOF, preferably the mass spectrometry is performed by MALDI-TOF.

In a further embodiment of the method, a tandem mass spectrometer is used for the peptide mass fingerprinting, wherein a MALDI-TOF/TOF spectrometry is particularly preferred for putting the method into practice.

In yet a further embodiment of the method, a matrix is used for the mass spectrometry selected from the group consisting of 3,5-dimethoxy-4-hydroxycinnamic acid, α-cyano-4-hydroxycinnamic acid and 2,5-dihydroxybenzoic acid, wherein α-cyano-4-hydroxycinnamic acid is particularly preferred as the matrix.

In another preferred embodiment of the method, the serum sample is calibrated or the serum samples are equilibrated to a predefined protein concentration by adding the lysis buffer, thus allowing an easy adaption of the system to different purposes.

In particular, it is preferred, if the method further comprises the steps of
- determining the protein concentration of the serum sample, in particular by the Bradford method; or
- freezing and thawing the serum sample before the lysis buffer is added; and/or
- staining the gel after the 2-DE, in particular by using coomassie blue; and/or
- destaining the exised sample; or
- shrinking, in particular by adding acetonitrile, and drying of the excised sample before the digesting buffer is added; or
- using a peptide calibration standard for the mass spectrometry,
wherein preferably a combination of said steps, in particular two of said steps, more preferably three of said steps, in particular four or five of said steps, most preferably all of said steps are implemented.

Yet another aspect of the invention concerns a procedure to screen for and to identify drugs against cancer associated with an increased EGFR kinase activity, wherein the procedure comprises determining in a body fluid sample of a transgenic cancer mouse being treated with a compound to be tested, in particular of a mouse whose genome comprises a non natural IgEGF sequence, at least one biomarker selected from the first group of said biomarkers and/or at least one biomarker selected from the second group of said biomarkers, and wherein the body fluid level of the at least one biomarker of said first group being significantly lower and/or the body fluid level of the at least one biomarker of said second group being significantly higher than the level of said biomarker(s) in the body fluid of an untreated transgenic cancer mouse is indicative of the therapeutic effect of said compound as an EGFR kinase modulator.

In a preferred embodiment, the procedure is implemented by using the method according the invention, in particular by using the method comprising an immunoassay or a peptide mass fingerprinting as described herein.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

Epidermal growth factor (EGF) is an important mitogen for hepatocytes. It's targeted overexpression induced hepatocellular carcinomas (HCC), as recently reported by us (Borlak et al. 2005). Early detection of disease is essential for successful therapy and overall survival. In particular, the efforts in identifying serum biomarkers of liver cancer in a transgenic disease model that mimics effectively the consequence of exaggerated EGF signalling are described. A reference 2-DE map of mouse serum proteins is reported. About 180 proteins were detected per gel and 130 proteins were identified by 2-DE-MALDI-MS analysis. Serum proteins of healthy non-transgenic and HCC tumor bearing mice were compared and 25 regulated proteins were identified, of which n= 7 reached statistical significance (p<0.05). Furthermore, several fragments of fibrinogens and of the alpha-2-macroglobulin were identified to be disease associated. Also immunoglobulins were found to be repressed or absent in serum samples of tumor bearing mice, and this included, amongst others, the Ig K and L class. In contrast, amyloid component P and apolipoprotein M were highly significantly increased by 10- and 8- fold, respectively in serum samples of HCC-mice. Overall, the findings provide a rationale for further clinical evaluation of the herein identified biomarker candidates.

In the following, the findings with an EGF transgenic disease model of liver cancer are reported. This is an important growth factor mitogen for hepatocytes. Its targeted overexpression promoted hepatocellular carcinogenesis as recently reported by us.⁸ In general, the EGF gene codes for a 53 amino acid protein to stimulate proliferation of epidermal cells and a variety of other cell types through binding to the EGF receptor. This single-pass transmembrane receptor functions as a tyrosine kinase. Once activated, the EGFR becomes autophosphorylated to initiate signalling through tyrosine phosphorylation of other proteins.⁹ A total of four different EGF receptors (Her1, Her2, Her3, Her4) have been identified so far. Specifically, ligand binding induces either homo- and/or heterodimerisation. It is of considerable importance that EGFR connects to other signalling cascades as well, notably the MAP kinase pathway, to ultimately cause phosphorylation of transcription factors such as c-Fos, c-Jun and ELK-1, thereby fostering cell proliferation.⁹ EGFR is over expressed in a number of solid tumours and the expression level correlates well with tumour progression, resistance to chemotherapy and survival. Consequently, EGFR is an obvious target for the rational design of novel anticancer agents, i.e. inhibitors of the receptor kinase activity and/or antagonistic antibodies.¹⁰ Previously, targeted overexpression of a secretable form of EGF (IgEGF) has been reported to result in multiple highly malignant HCCs, with 100% fatalities around 7-8 months after birth .¹¹ This transgenic mouse line mimics effectively the consequence of an altered EGF and/or TNFα signalling. Recently, other mice models were successfully established to study the tumor biology of HCC in non-viral- and viral disease ¹²⁻¹³. Through application of gene chip analysis it was possible to identify networks of EGF-regualted genes at various stages of tumour development.⁸ Here, the efforts to examine serum proteins of non-transgenic healthy and EGF overexpressing transgenic mice are reported. A reference 2-DE map of mouse serum proteins, consisting of more than 130 proteins, was created . After 2-DE analysis and gel image matching, 25 serum proteins were identified by peptide mass fingerprinting as being disease associated. These proteins are involved in a variety of cellular and metabolic pathways, amongst them: the glutathione peroxidase 3 (Gpx3) and the serum amyloid component P (SAP), their expression being up-regulated by up to 10-fold in tumour bearing mice.
Overall, this study aimed at identifying disease associated serum proteins in an EGF liver cancer disease model.

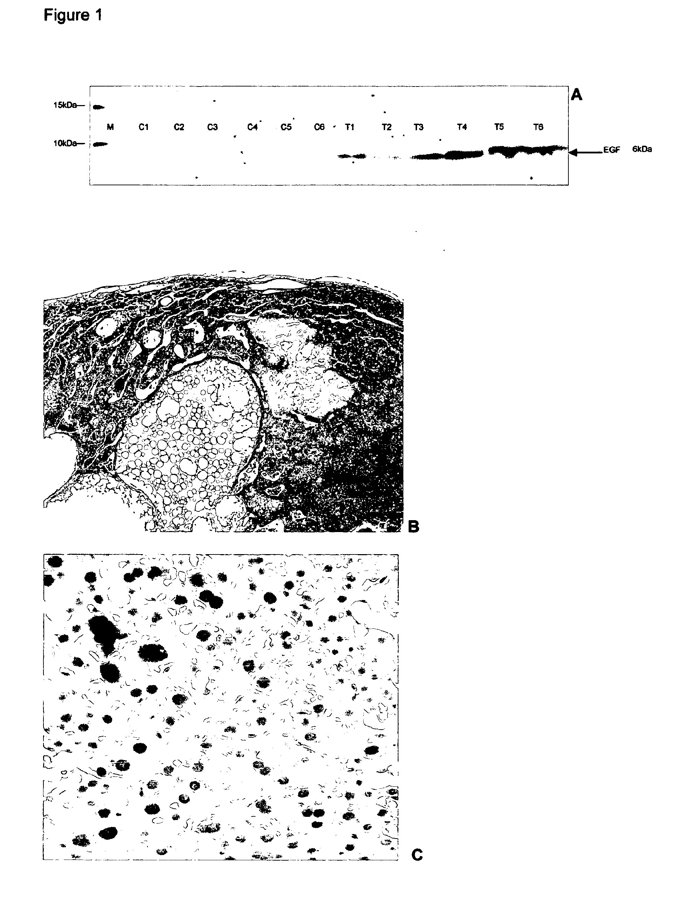

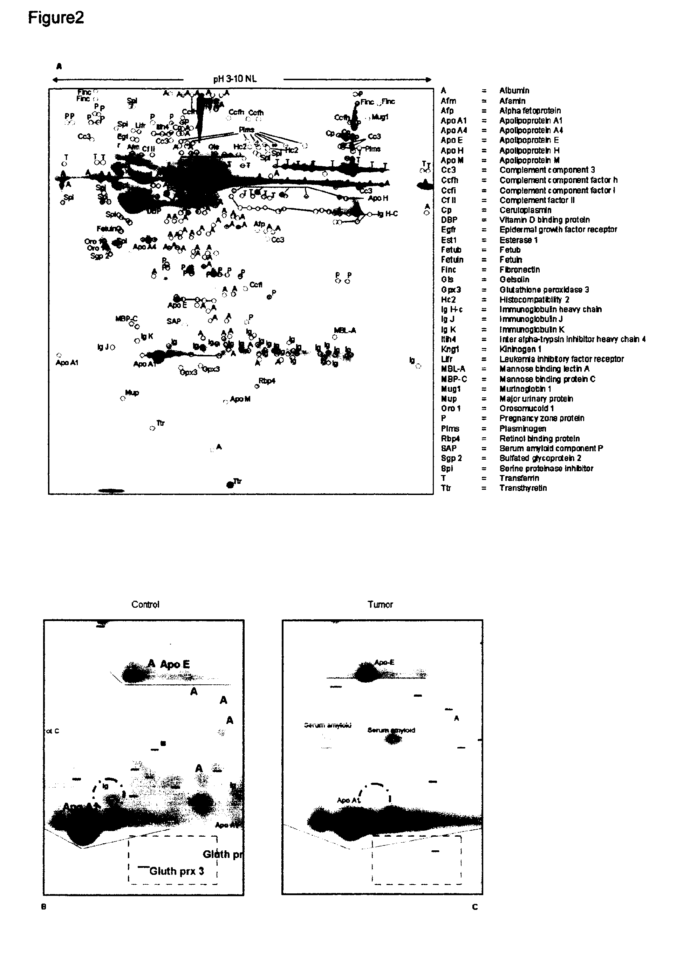

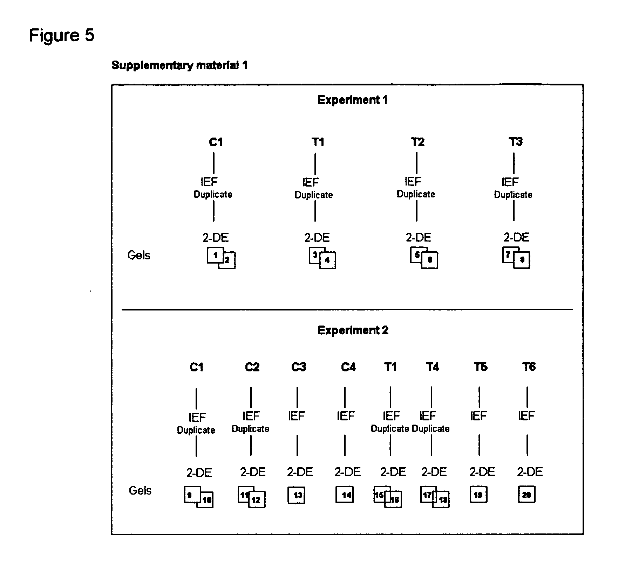

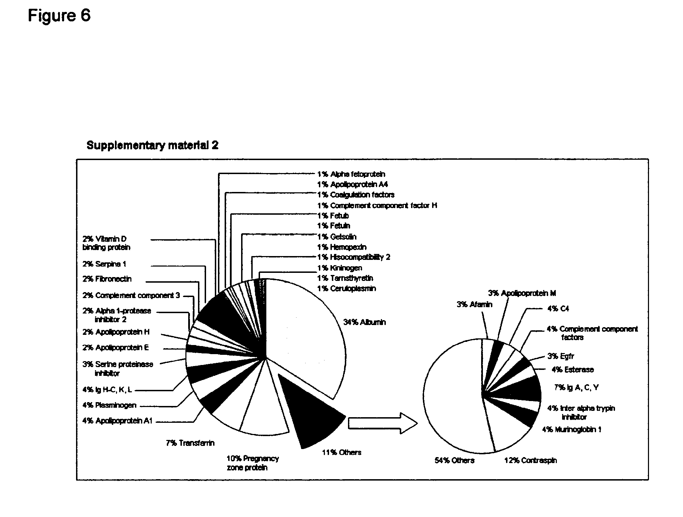

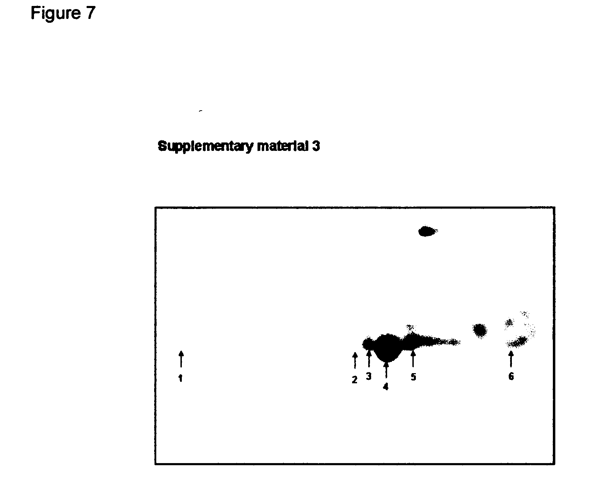

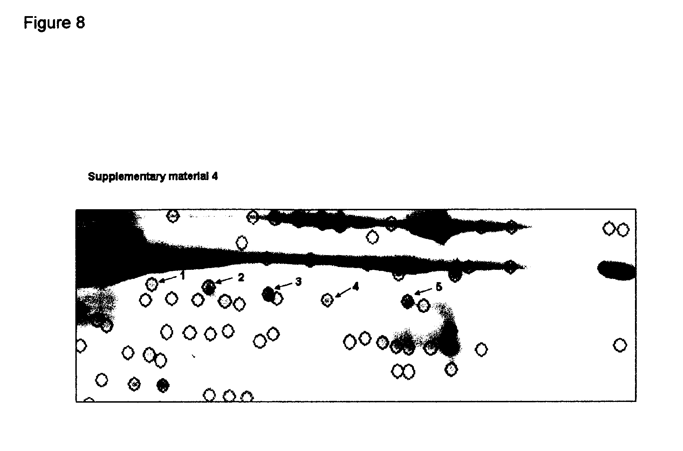

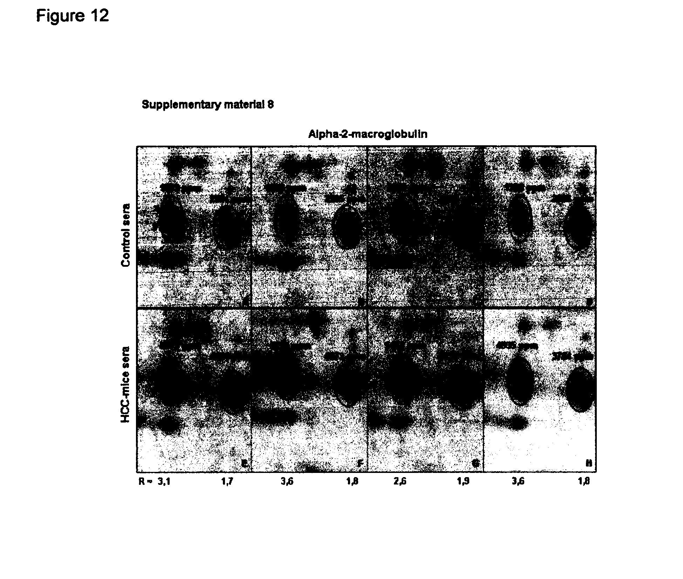

The serum proteome of healthy and HCC tumor bearing mice was investigated . As depicted in Figure 1A, transgenic mice overexpressed EGF by approximately 26-fold. Furthermore, tumors were confirmed by histopathology, of which an example is given in Fig. 1B. Essentially, highly differentiated tumors were observed which were less differentiated at advanced stages of disease (see also Borlak et al., 2005 for further details).⁸ A total of 14 2-DE gels (n = 5 independent serum control and n = 9 independent serum of HCC mice) were processed for MALDI-TOF-MS analysis (see supplementary material 1, Fig. 5). This enabled the built up of a reference 2-D map of mouse serum proteins consisting of 130 proteins (Fig. 2A). 25 proteins were found to be differentially regulated of which n= 7 were statistically significant (Table 1, Fig. 2B , C). Albumin is the most abundant serum protein ; its quantification of the 2-DE gels gave a relative volume of approximately 34 % (see supplementary material 2, Fig. 6). Other high abundance proteins include alpha-2-macroglobulin with a relative volume of 10 %, transferrin (7 %), apolipoprotein A1 (4 %), plasminogen (4 %) and several immunoglobulins (Igs) such as H-C, K and L (4 %). Less abundant proteins were apolipoprotein E, apolipoprotein H, fibronectin, gelsolin, kininogen and ceruloplasmin. Several spots were identified at low molecular weights as albumin or fragments of it (Figure 2 A) and more than 8 spots as alpha-2-macroglobulin (A2MG) (fragments) with mass ranges of 37-40 kDa (A2mG has a Mr of ∼ 165 kDa). At least 2 of these fragments were up-regulated in serum of tumour bearing mice and were increased by 3-fold (see table 1, Fog. 3E, supplementary material 8, Fig. 12). Furthermore, two isoforms were identified with identical pl but different Mr as well.
Additionally, the apolipoproteins Apo A-I, Apo E, Apo H, ApoM and ApoJ were identified. 6 different spots in the serum proteome maps were observed, which were identified by MALDI-TOF as Apo A-I (see supplementary material 3, Fig. 7) and at least 5 spots of apo H, also known as beta-2-glycoprotein I (see supplementary material 4, Fig. 8).

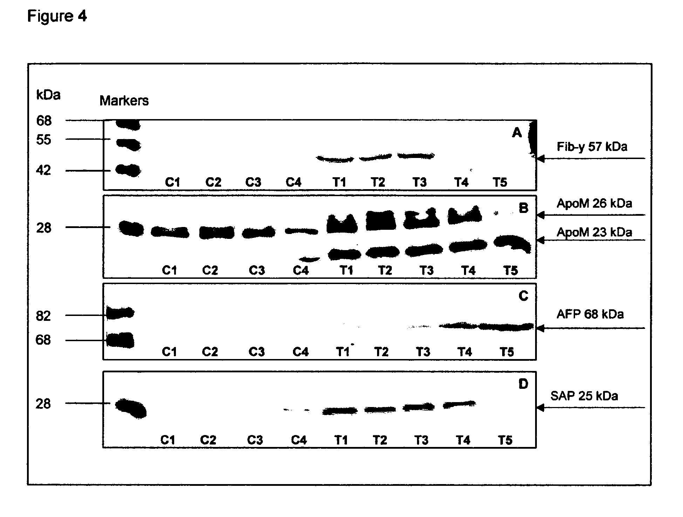

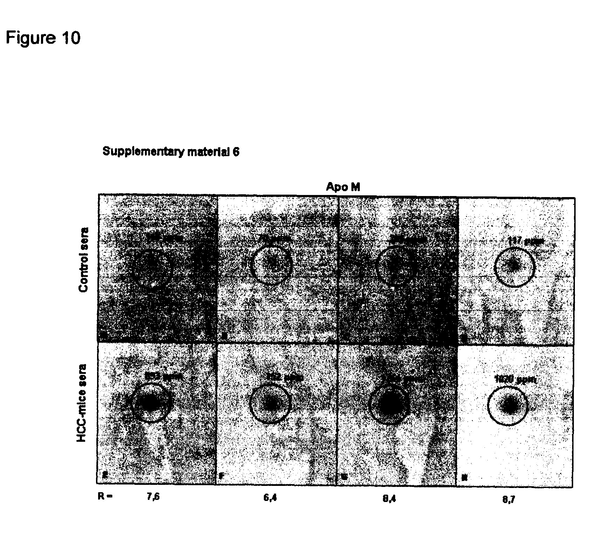

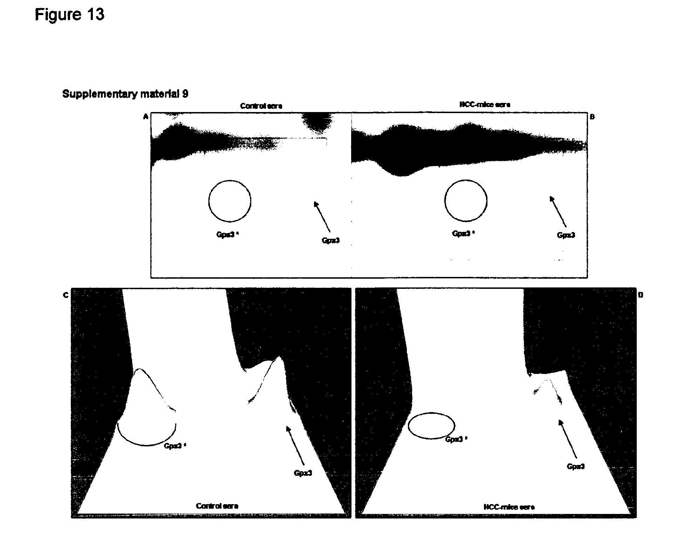

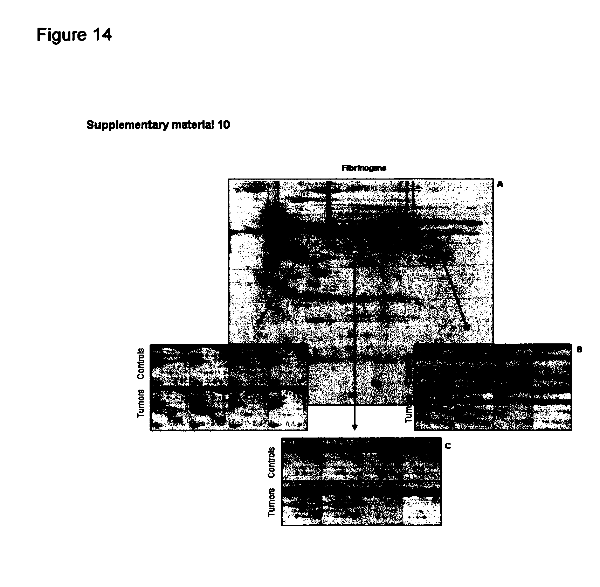

After in gel digestion, approximately 2500 spots were selected for MALDI-TOF-PMF analysis. In the supplementary table 1 accession number, protein name, theoretical pl and Mr together with Mascot score, identified peptides and sequence coverage are given. Furthermore, biological function, frequency of identification and protein expression in serum of tumor and / or healthy mice are summarized as well (see supplementary table 1). Among the 130 annotated proteins, 25 were differentially expressed (Table 1), of which 7 were up-regulated (apolipoprotein E, alpha-2-macroglobulin, alpha-2-macroglobulin *, alfafetoprotein, apolipoprotein M, serum amyloid component P, serine proteinase inhibitor) (Fig. 3C, 3E), where as 3 proteins were down-regulated (glutathione peroxidase 3, properdin, major urinary protein 1) (Fig. 3A). Moreover, 10 proteins were found in serum of tumor bearing mice only (amylase 1, apo A-I, carboxylesterase, caspase, clusterin, fibrinogen-α, fibrinogen-β, fibrinogen-γ, major histocompatibility complex factor B, serum amyloid component P*), but 5 proteins were exclusively identified in serum sample of healthy control animals (mannose binding lectin A, orosomucoid 1, HMW-kininogen II, leukemia inhibitory factor receptor, mannose binding protein-C) (Fig. 3G).
In tumor bearing mice, most of the up-regulated serum proteins are apolipoproteins. Among them, apoM was up-regulated by 8-fold (Fig. 3C , 4B, supplementary material 6, Fig. 10). ApoM is seen as an important biomarker candidate and its significance is discussed later on. Furthermore, apolipoproteins are closely associated with amyloid fibrillogenesis. Indeed, serum amyloid A, apolipoprotein (apo) All and apo A1 are deposited as biochemically distinct forms of amyloid.¹⁴ In serum samples of HCC bearing mice, the serum amyloid component P (SAP) was strongly increased. This protein belongs to the group of acute-phase reactants (APRs) (Fig. 3C, 4D). Here SAP is evidenced to be highly disease associated in HCC and a putative isoform (SAP*) in sera of tumour bearing mice is identified (see table 1, supllemantary material 7) with a lower theoretical pl value.
In addition, several isoforms of alpha-2-macroglobulin were found (see Fig. 2A , supplementary material 8, Fig. 12) to be induced in HCC. To the best of our knowledge, the overexpression of the newly identified fragments have not been reported for HCC so far. In general, the serum concentration of A2MG is about 2 g/l in adults but displays little variation with age or in acute and chronic disease. It therefore may qualify as a bonafide candidate serum biomarker for HCC.
Further, decreased concentration of proteins of the acute phase response in serum of HCC mice were observed . This group of proteins, designated as negative acute phase proteins, included major urinary protein 1 (MUP1), glutathione peroxidase 3 (Gpx3), properdin, and several immunoglobulins (Igs). (see table 1 , Fig. 3A). Specifically, plasma glutathione peroxidase 3 (Gpx3), a glycosylated protein, was repressed when serum samples from healthy non-transgenic and tumor bearing mice were compared. In 2D gels two different spots are visible e.g. Gpx3* and Gpx3 while only one isoform was repressed in serum samples of tumour bearing mice (Gpx3) (see Fig. 3A , supplementary material 9, Fig. 13).
In the present study, also cancer-related fibrinogen deposition in serum of HCC bearing mice is identified (see table 1, Fig. 3D, supplementary material 10, Fig. 14). Plasmin-generated fragments of fibrinogen alpha, beta and gamma (Fga, Fgb, Fgg) were already observed in various types of solid tumor types. In fact, elevated levels of Fgg and acute phase proteins were reported for plasma proteins of prostate-, lung-, and breast-cancer patients.¹⁵⁻¹⁶ As of today overexpression of FGG was reported for two hepatoma cell lines, e.g. SMMC-7721 and HepG2.¹⁷ Up-regulation of Fgg in EGF induced HCC is now evidenced, as well. Specifically, not all tumor samples expressed the three fibrinogens (see Figure 4A , supplementary material 10, Fig. 14), but this agrees well with the findings of Gerner et al., who reported Fgg to be present in 58 % of the cases (14 / 24 analyzed plasma cancer samples.¹⁵⁻¹⁶ Elevated plasma levels of Fgg, as observed in serum of EGF transgenic mice may be viewed as indicators for tumor-associated fibrin deposition and fibrinolysis. Fibrinolysis is mediated mainly via the plasma protease plasmin, which exerts pleiotropic effects.¹⁸⁻²⁰ Furthermore, the complement system is known to contain at least 30 different proteins, which are primarily formed in the liver and circulate in their inactive form. These proteins, when activated, produce various complexes that play a major role in the natural defense mechanisms of the human body. Several proteins of the complement system were identified to be regulated. This included the mannose binding lectine A and the mannose binding protein C (present in serum of healthy non-transgenic mice only), and the major histocompatibility complex factor B (MHC-fB) which was found to be up regulated. MHC-fB was identified in healthy and HCC serum samples, but an activated form/fragment of this protein was found in serum of HCC mice only in the range of Mw 65-68 kDa (see table 1, Fig. 3F). These fragments could be the result of proteolitic processes in tumours.
Also, orosomucoid 1, also known as alpha 1-acid glycoprotein, was identified in sera of health non tumor bearing animals (see table 1, Fig. 3G). This protein is synthesised by the liver, and functions in modulating the activity of the immune system during the acute-phase reaction. Several studies report an up regulation of this protein in patients with HCC and suggest orosomucoid as a useful marker for discriminating the stage of inflammatory reactions.²¹⁻²³ Finally, an up regulation of the oncofetal protein Afp was observed in sera of HCC-mice (Fig. 3C , 4C). Indeed, Afp is routinely assayed for liver damage and its malignancies.

To confirm results by different means, 4 proteins regulated in all gels were selected for Western Blot analysis. A total of n = 5 serum samples of tumor bearing mice were probed for Fib-γ, ApoM, Afp (Fig. 4 A,B,C), where as n= 4 serum samples was used for SAP (Fig. 4 D). Fib-γ was expressed only in 3 out of the 5 serum samples analyzed.

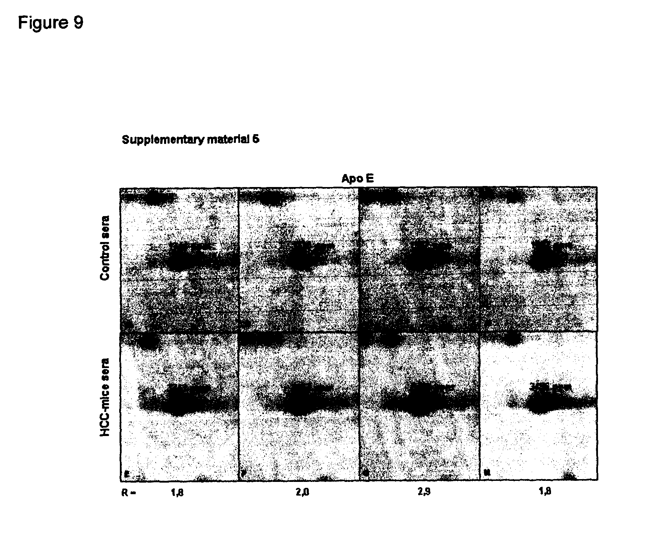

Serum biomarkers of HCC were searched for and a serum proteome map of EGF induced HCC is reported. Comparison between sera of healthy and tumour animals revealed significant differences in expression of several proteins. A total of 25 proteins was identified as differentially expressed. Specifically, proteins of the acute phase response were found to be down regulated. This group of proteins included mannose binding lectin a (MBL-A), major urinary protein 1 (MUP 1), orosomucoid 1 (Orm1), glutathione peroxidase 3 (Gpx3) and several immunoglobulins (Igs) (see table 1, supplememntary material 11). In contrast, Apo E was up regulated in serum of HCC mice.Notably, Yokoyama and coworkers observed increased expression of apo E in 88 % of HCC tumor tissue without an increase of apo E gene expression and / or elevated serum level.¹⁹ These results do not agree with the findings as serum levels of apo E were increased by 2-fold (Fig. 3E and supplementary material 5, Fig. 9). An overexpression of ApoE has already been observed in brain ²⁴, breast ²⁵, ovarian²⁶, prostate²⁷ and HCC tumor tissues.²⁸

Another up-regulated apolipoprotein was apoM. This protein is mainly associated with high-density lipoprotein (HDL) in human plasma, and a small proportion in triglyceride-rich (TGRLP) and low-density lipoproteins (LDL). During embryogenesis, apo M is over expressed in livers of 3-5 month-old human embryos and continues to be strongly expressed throughout embryogenesis, but thereafter returns to much lower levels. Apo M is found as an important biomarker candidate. Furthermore, there is evidence for platelet-activating factor (PAF) to be involved in the up-regulation of apo M in HepG2 cells, but Luo and coworkers reported decreased apo M mRNA transcript levels in HepG2 cells in response to epidermal growth factor (EGF) treatment.²⁹⁻³⁰ It is of considerable importance that apo M gene expression may also be regulated by hepatocyte nuclear factor-1α (HNF-1α), which was found to be repressed in tumor tissue of EGF transgenic mice (results of Western Blot not shown).³¹ Furthermore, the apo M gene is located within the histocompatibility complex III (HMC-III) region of chromosome 6 and many genes in this region code for immune response.³⁰ Whether apo M is co-regulated by the host defense system requires additional research.
In general, apolipoproteins are important in maintaining the structural integrity of lipoprotein particles thereby facilitating the solubilisation of lipids. Additionally, they play a crucialrole in lipoprotein receptor recognition and regulation of lipoprotein metabolism. In humans, about 60 % of the protein content in high-density lipoprotein (HDL) is represented by apo A-I and about 20 % by apo A-II. Other apolipoproteins include apo A-IV, apo C, apo D and apo E. Most of these proteins are expressed as different isoforms. Recently, it was suggested that apo A-I exists in six isoforms, 4 of them displaying differences in glycosylation pattern.³² Likewise, apo-H displays genetic polymorphism, with three alleles, namely APOH*1, APOH*2, APOH*3, at a single locus on chromosome 17.³³ This protein has been identified as a structural component of chylomicrons, very low-density lipoproteins (VLDL), low-density lipoproteins (LDL) and HDL. In human plasma, 35 % of apo-H is associated with chylomicrons.³⁴ Various properties have been ascribed to this protein, e.g. function as an antigen of antiphospholipid antibodies, or acute-phase reactant and may have some involvement in the HBV infection of hepatocytes as well_{.}³⁵⁻³⁶

Our study identified A2MG to be strongly upregulated in HCC. This protein is an abundant plasma protein produced predominantly by the mammalian liver. A2MG is a member of the protease inhibitor 139 (A2M) family and is able to inhibit all four classes of proteinases by a unique 'trapping' mechanism located in a 'bait region', which contains specific cleavage sites for different proteinases. When a proteinase cleaves the bait region, a conformational change of the protein is induced, which then traps the proteinase. The entrapped enzyme remains still active against low molecular weight substrates, but is poorly accessible for reaction with high molecular weight substrates. Mouse A2MG has a M*r* of ∼165 kDa; notably, more than 8 spots as A2MG fragments with mass ranges of 37-40 kDa were identified . At least 2 of these fragments were up regulated in serum of tumor bearing mice and were increased by 3-fold (see table 1, Fig. 3E , supplementary material 8, Fig. 5). Furthermore, two isoforms with identical pl but different Mw were identified as well. In breast and trophoblastic cancers, A2MG may be less useful as a tumor marker.³⁷ In contrast, serum A2MG levels in patients with ovarian carcinomas are significantly elevated.³⁸⁻³⁹ Likewise, the study also evidenced repression of glutathione peroxidase 3 (Gpx3) in HCC bearing mice. Gpx3 is one of at least 25 selenocysteine-containing protein with antioxidant properties in mammals. It is one of the five known glutathione peroxidases and is unique among members of the Gpx family as this protein is the only extracellular isoform. Gpx3 is secreted from renal proximal tubular cells and epithelial cells of the Bowman's capsule. While Gpx3 deficiency has been associated with cardiovascular disease and with renal dysfunction or infertility of males, little is known about its association with HCC.⁴⁰⁻⁴¹ Strikingly, plasma selenium concentration is decreased in patients with cirrhosis, as reported by Burk and coworkers, who investigated glutathione peroxidase (GSHPx-3) and selenoprotein P expression, in patients with cirrhosis and in healthy control subjects.⁴² Equally, studies on selenium availability and expression of selenoproteins (Gpx1, Gpx3, thioredoxin reductase and selenoprotein P mRNA) in mouse fibrosarcoma cells evidenced selenium to exert a statistically significant effect on Gpx3 transcript expression.⁴³ Whether selenium availability is reduced in HCC requires further studies.
Also, high proteolytic activity in sera of HCC bearing mice was observed. Recently, Chignard et al. detected protein fragments in HCC patient sera as well, identified as calreticulin and protein disulfide isomerase A3.⁴⁴ Generally, plasma proteases are capable of activating growth factors by cleavage of their inert proforms.⁴⁵ High proteolytic activity of proteases in plasma of cancer patients has been reported.¹⁶ This may also be linked to an overexpresion of SAP, apo M and other serum proteins in HCC bearing mice as reported in the study. An acute phase response is observed in the majority of cancer patients⁴⁶ and could represent an adaptive response to an exaggerated proteolytic activity. In fact, during tumor growth these proteins accumulate in peripheral blood and may interfere by a direct anti-apoptotic mode on tumor necrosis factor-induced apoptosis of hepatocytes as suggested by Van Molle et al, 1997.⁴⁷⁻⁴⁸

Finally, disease associated regulation of a fragment of Factor B in serum of HCC mice was found. Factor B is a serine proteinase of the antibody-independent, alternative pathway of complement activation, an important humoral response of the host defense system against invading pathogens. In addition, fragments of the factor B exert cytokine-like activities to cause B lymphocyte proliferation and differentiation, macrophage spreading and monocyte mediated cytotoxicity. The major site of MHC-fB expression is the liver, as evidenced by allotyp changes of serum MHC-fB following liver transplantion. MHC-fB is a positive acute phase reactant. It's hepatic synthesis and serum level are increased during the acute phase of the inflammatory response.⁴⁹ Overall, serum amyloid component P (SAP), apolipoprotein M (apo M), alpha-2-macroglobulin (A2MG) and fibrinogens (Fga, Fgb, Fgg) are found as beneficial candidates for HCC-diagnostics, since their serum levels were increased by 10- , 8-and 3- fold, respectively. Furthermore, fibrinogens were identified in serum samples of HCC mice only, but their expression was variable. The current available screening tests to detect early liver malignancies combine α-fetoprotein analysis and ultrasound. Even though screening for early detection of HCC has become more common, its effectiveness in disease diagnostics remains controversial.⁵⁰ There is a need to search for new, robust and specific markers for the detection of HCC at early stages of disease to allow for curative rather than palliative interventions. Since blood serum contains high concentrations of abundant proteins such as albumin, transferrin, haptoglobin and immunoglobulins, their removal may be advantageous to enable detection and identification of less abundant proteins. Unfortunately, many of the target proteins are bound to abundant proteins; thus, depletion of serum of major proteins using affinity chromatography, specific antibodies or size-exclusion filtration may also lead to removal of less abundant proteins and therefore loss of biomarkers. In conclusion, the use of 2-DE combined with MALDI-TOF-MS, analysis provided evidence for 25 differentially expressed proteins in serum of HCC bearing mice, of which n=7 reached statistical significance. Several proteins, so far unknown to be regulated in HCC, have been identified and represent beneficial biomarkers useful for implementing the invention.

Materials A UP 200S sonicator (Dr. Hielscher GmbH, Germany) was used to homogenize the samples. For the first dimension, immobilized pH-gradient (IPG) strips (17cm, pH 3-10 non linear) were purchased from Bio-Rad (Hercules, CA USA). The focusing chamber was Protean IEF Cell (Bio-Rad). For the second dimension a Protean plus Dodeca Cell (Bio-Rad) was used. Reagents: tris, urea, thiourea, CHAPS, dithiothreitol, bromophenol blue, glycerin, sodium dodecyl sulphate, glycin, temed, ammoniumperoxodisulphate, ammonium sulphate, ammonium bicarbonate, colloidal coomassie blue and acrylamide were purchased from Roth (Karlsruhe, Germany). lodacetamide was from SERVA (Heidelberg, Germany). Benzonase was purchased from Novagen (Darmstadt, Germany). Ampholytes (Biolyte 3-10) were purchased from Bio-Rad (Hercules, CA USA).

Animal care The creation of the EGF2B transgenic line was described earlier by Tönjes et al. (1995). Transgenic mice were maintained as hemizygotes in the CD2F1-(DBA/2xBalb/c) background. PCR was carried out with Platinum PCRSuperMix (InVitrogen). Annealing temperature and the number of cycles are indicated in brackets after each primer pair. The transgene was verified by PCR of DNA extracted from tail biopsies (Hogan et al., 1994) and the following forward primer (fp) and reverse primer (rp) pair was used for a transgene specific amplification: forward primer: 5'-CTAGGCCAAGGGCCTTGGGGGCTCTTGCAG
- 3'; reverse primer: 5'- CATGCGTATTTGTCCAGAGCTTCGATGTA-3' (61 °C, 32 cycles, 317 bp).
   Animals, aged 6-8 months and of the weight of 25 -33 g, were housed in Makrolon ® Type III cages. Drinking water and food (V1124-000, SSNIFF, Holand) was given *ad libitum.* Temperature and relative humidity were 22 ± 2 °C and 40-70 % respectively. Furthermore, a 12 h day and night cycle was used. For serum protein identification, mice were sacrificed with CO₂ and blood was taken from the *Vena cava.* Then, blood was centrifuged (20 min., 6000 rpm, at room temperature) and serum was immediately frozen at -80 °C. None of the serum samples were hemolytic.

Sample preparation Five micro liters of sera from n=4/group non-transgenic control and from n=6/group tumor bearing animals were used for further 2-DE analysis. The protein concentration of serum was determined by the Bradford method. For each animal 0.5 mg of protein sample, approximately 3 µL of serum, were diluted to a final volume of 350 µL with a lysis buffer containing 40 mM tris base, 5 M urea, 2 M thiourea, 4 % CHAPS, 100 mM DTT, 0.5 % (v/v) biolyte 3-10 and endonuclease (6 µL / mL).

Two-dimensional gel electrophoresis - 2-DE A total of n=8 2-DE gels for control serum samples and n=12 2-DE gels for serum of tumor bearing mice were prepared as follow: IEF was performed using precast 17 cm IPG strips 3-10 NL. 0.5 mg of protein was loaded by active rehydration (12 h, 50 V). Focusing started at 250 V for 20 min. in rapid mode, 10000 V for 5 h in linear mode and 10000 V for 50000 Vh in rapid mode. Each sample was analyzed in duplicate.
After IEF, the IPG strips were either stored at - 80°C or transferred to 10 mL equilibration buffer (6 M urea, 30 % w/v glycerin, 2% w/v SDS, 50 mM Tris-HCl pH 8.8) with 2 % w/v DTT and 0.5 % v/v bromophenol blue solution (0.25 % w/v bromophenol blue, 1.5 M Tris-HCl pH 8.8, 0.4 % w/v SDS) and incubated for 20 min. at room temperature. Strips were removed and incubate in equilibration buffer with 4 % w/v iodoacetamide and 0.5 % v/v bromophenol blue solution for further 20 min. at room temperature. Finally, strips and 10 µL SDS-PAGE molecular weight standard on filter paper were placed on top of the 20 cm x 20.5 cm 12 % second-dimension gel (12 % v/v acrylamide/bis solution, 375 mM Tris, pH 8.8, 0.1 % v/v SDS, 1/2000 TEMED, 0.05 % v/v APS). Both were fixed in place with a 0.5 % w/v agarose overlay. Gels were run in PROTEAN Plus Dodeca cell from Bio-Rad at 70 V for approximately 14 h, followed by 200 V until the bromophenol blue dye reached the bottom of the gel. The running buffer (25 mM Tris, 0.2 M glycin, 0.1 % SDS) was cooled externally to 16°C.
Gels/proteins were fixed overnight in 30 % ethanol, 2 % phosphoric acid, washed 3 x 20 min. with 2 % phosphoric acid. The gels were equilibrated with 15 % ammoniumsulfate, 18 % ethanol, 2 % phosphoric acid for 15 min and finally stained with colloidal coomassie blue for 48 h.

Gel scanning and image analysis After staining, gels were washed 10 min with pure water and scanned on a Molecular FX Scanner Bio-Rad at 100 µm resolution. Protein spots were imaged first automatically and then manually and analyzed using the PDQuest^{™} software Bio-Rad. The normalization was carried out in total density in gel mode according to the manufactures recommendations. For reproducibility, two experiments were performed, each time running 4-control and 6-tumor samples for a total of 20 gels. From them, 14 gels were chosen and used for spot excision and MS analysis. The gels with higher number of spots were selected; all spots (about 180 spots per gel) were excised for MS analysis

MALDI-MS A total of 2500 spots from 14 gels were excised using the spot cutter of Bio-Rad and placed into 96-well microtiter plates. Excised gel spots were washed with 20 µL of water for 10 min. and destained twice with 15 µL ammonium bicarbonate 50 mM for 5 min. first and then with 15 µL 50 % ammonium bicarbonate 50 mM - 50 % acetonitrile for 5 min. Finally, gel particles were covered by acetonitrile until gel pieces shrunk and left dry for 10 min. Gels/proteins were digested in situ with 4 µL of ammonium bicarbonate 50 mM containing 20 ng trypsin (Sequencing Grade Modified Trypsin Promega). After 15 min. each gel peace was re-swelled with 10 µL of ammonium bicarbonate 50 mM and incubated for 4 h at 37°C. After 4 h the reaction was stopped by adding 10 µL of trifluoro acetic acid 1 % containing 1.5 % (w/v) n-octyl-β-D-glucopyranoside (OGP) (AppliChem). For the application of the samples, 4 µL of peptide solution were loaded on a MTP Anchor Chip Target 600/384 (Bruker Daltonics) previously prepared with a saturated solution of matrix, α-cyano-4-hydroxy-cinnamic acid (α-HCCA) (Bruker Daltonics). An external calibration was performed by spotting on the 96 calibration positions of the Anchor Chip Target 1 µL of peptide calibration standards (Bruker Daltonics) containing the following peptides: angiotensin II (1046.5420 Da), angiotensin I (1296.6853 Da), substance P (1347.7361 Da), bombesin (1619.8230 Da), ACTH clip 1-17 (2093.0868 Da), ACTH clip 18-39 (2465.1990 Da), somatostatin 28 (3147.4714 Da) and OGP 1.5 % (w/v). Samples were analyzed in a MALDI-TOFFTOF spectrometer (Ultraflex, Bruker Daltonics) using an accelerating voltage of 25 kV for the Peptide Mass Fingerprint (PMF) mode. Peptide matching and protein searches were performed automatically with the MASCOT 2.0 software. For the PMF search the parameters were the following: C-carbaimidomethyl (fixed modification), M-oxidation (variable modification), monoisotopic (mass value), 100 ppm (peptide mass tolerance), 1 (max missed cleavege), mammalia (taxonomy). Five matching peptides and at least 10 % peptide coverage of the theoretical sequences was the minimal requirement for an identity assignment. The identified proteins were organized with the ProteinScape^{™} database (Protagen-Bruker Daltonics), checked individually and only mouse proteins or highly homologous sequences from other species were considered.

In conclusion, the EGF receptor plays an important role in various tumor diseases. Its hyperactivity can cause cancer of numerous organs. By detecting early stages of tumor growth a dramatic reduction in the mortality rate of cancer patients can be achieved. To date, though, diagnostic markers for liver cancer, such as alpha-Fetoprotein (AFP) and the Des-Gamma-Carboxyprothrombin (DCP) are insufficient for the definite diagnosis of tumor disease.

The studies according to the invention provide new information on the role of EGF in tumorigenesis. The serum proteomics facilitates the discovery of biomarkers and enables an improved early detection of cancers and therapeutic monitoring in the various treatment strategies.

An EGF-transgenic mouse model has been developed and by using this model the consequences of a changed EGF-signalling in the emergence of liver cancer has been investigated. The mouse model is very similar to the human hepatocellular carcinoma allowing research on the various stages of cancerogenesis.

With the help of genomic platform technologies molecular events referring to the individual stages of tumor development could be investigated. Also, the analyses of tumor specific proteins and serum proteins according to the invention provided beneficial approaches for identifying potential serum biomarkers of liver tumors.

Blood proteins are easily accessible. Therefore, great efforts are undertaken worldwide to seek serum biomarkers for monitoring the course of disease in patients. Changes in the expression of serum-proteins or -peptides are easy to measure even in the early stages of the disease to measure, well in advance before the disease phenotype associated with significant metabolic damage manifests.

A major drawback in serum proteome analytics is, however, the complexity of the sample to be analyzed. For this reason, several additional methods for removing abundant proteins, such as albumin, have been developed to facilitate an identification of the less prevalent and disease associated proteins.

However, the pre-treatment of serum samples is problematic, since proteins that only occur in small quantities are lost. Moreover, the prefractioning of serum proteins is costly in terms of time. Here, the efforts for analyzing the serum proteome while avoiding an pre-fractioning are described.

The EGF2B-transgenic mouse has already been described in a previous publication. For the serum proteome analysis blood serum was obtained both from wild type mice as well as tumor mice.

By using a lysis buffer containing thiourea the serum proteins were extracted and separated by 2D-gelelektrophoresis (2-DE) with the use of two different pH-gradients (3-10, 4-7).

Subsequently to colloidal Coomassie-blue staining the spots were cut from the 2D-gel with a spot cutter. Then, the gel samples were washed, discolored and digested with trypsin.

An over-expression of EGF in transgenic mice was confirmed by RT-PCR. As previously described in detail, HCC-disease was induced by liver specific overexpression of EGF. In the genomic studies, also signal proteins regulated by an excessive EGF-tyrosine kinase activity could be identified.
Using genome wide gene expression analysis, new candidate genes that play a role in tumor formation were identified. Some were changed depending on the degree of differentiation of the tumor, so for example the expression of TGFα and PDGFα. Increased expression of eps-15, a substrate of the EGFR was likewise observed.

In less differentiated tumors, the activation of the RhoC-kinase was particularly pronounced. In all of the tumors the expression of cell cycle-regulating proteins such as junB, c-fos, egr-1, and the survival factor IGFBP1 was significantly increased.

The oncogenomic studies revealed important information about the serum proteome analyses and were affected by the motivation to find new diagnostic biomarkers for HCC.

For overcoming the technical difficulties, the analysis of the serum proteome had to be improved so that as many proteins as possible could be identified after separation with high-resolution 2-DE by mass spectrometric methods (MALDI-MS) .

With the help of differential display, tumor-associated serum proteins were discovered in the 2D-gel electrophoresis.

In addition, it was possible to automate the MS-analysis of the trypsin-digested proteins.

The different behavior of matrix peptide mixtures in peptide mass fingerprinting has already been published in numerous studies.

It was further a goal to automate the MS and MS / MS-analyses in order to detect tumor serum proteins in a high-throughput format. For example, with the help of this method the zinc-alpha-2-Glycoprotein could be identified with an outstanding sequence coverage.

This was achieved by the use of α-cyano-4-hydroxycinnamic acid in matrix preparations. In addition, this improved mass spectroscopic method also allows access to information on posttranslational modifications such as phosphorylation and glycosylation.
With the help of this newly developed protocol, a reference list of the serum-proteome was created, which enables an important basis for studies in healthy and tumor diseased subjects.

### Figure captions

**Figure 1****.** A: Western blot representing the overexpression of EGF in livers of transgenic mouse model of HCC. (T= tumor samples; C= control samples). B: Histopathology of advanced liver cancer. C: Histopathology of control liver.

**Figure 2****.** A: Example for a map of the mouse serum proteome. (pH 3-10 NL; stain: coomassie blue; loaded sample: 500 µg); B, C: Zoom in for control and tumour serum samples, respectively. Immunoglobulins (circle) and glutathione peroxidase 3 (small rectangle) are down regulated in HCC-mice sera (right side of the panel). Two spots of serum amyloid component P (big rectangle) are up regulated (right side of the panel).

**Figure 3****.** ppm values of the 25 regulated proteins. A: down regulated proteins; C, E: up regulated proteins; B, D, F: proteins virtually present only in tumor samples; G: proteins virtually present only in control samples.

**Figure 4****.** Western blots of fibrinogen gamma (A), apoM (B), alfafetoprotein (C) and serum amyloid component P (D).All these proteins were up-regulated in serum samples of tumor bearing mice. (C= control , T= tumor).

**Table 1.** List of the 25 differentially regulated proteins. Proteins are sorted according to their name. NCBI accession number, MASCOT score, percent of sequence coverage and number of identified peptides (Match) are given. Protein function, p-value, mean fold change and frequency of in gel identification are also reported in table 1. ° Spots were present in 2 gels, but only 1 spot was cut and analyzed for MALDI mass spectrometry identification. * Isoform

**Supplementary material 1 (****Fig. 5****):** Outline of the experiments. Two experiments were carried out. In the experiment 1, n=1 control serum (non transgenic mouse) (C1) was run in duplicate and then studied for the search and identification of proteins; n = 3 tumour (T1, T2, T3) sera of bearing mice (EGF) were run in duplicate and then studied for the search and identification of proteins.
In the experiment 2, n= 4 control sera (non transgenic mice) (C1, C2, C3, C4) and n= 4 tumour sera (T1, T4, T5, T6) were run and then processed for protein identification. In summary a total of 5 gels were studied for the control samples (grey boxes) and a total of 9 gels (grey boxes) were studied for the tumour samples. (IEF: isoelectric focusing ; 2-DE: two dimensional electrophoresis

### Supplementary material 2 (Fig. 6):

Distribution of relative volumes of identified proteins.
Primary hepatocellular carcinoma (HCC) is worldwide a common neoplasm with approximately 600 000 death per year. Early detection of tumor growth is essential for therapy and overall survival. Disease-associated proteins have been searched in the sera of HCC bearing mice, which specifically developed HCC, as a result of targeted overexpression of epidermal growth factor (EGF). The picture depicts the distribution of the relative volumes of the spots in the quantification of the 2-DE gels

### Supplementary material 3 (Fig. 7)

The six spots of Apo A-I in 2-D gels.

### Supplementary material 4 (Fig. 8)

The five spots of Apo H in 2-D gels.

### Supplementary material 5 (Fig. 9)

Overexpression and ppm values of apolipoprotein E (apo E) in sera of HCC mice. R = ppm ratio (tumour / control).

### Supplementary material 6 (Fig. 10):

Overexpression and ppm values of apolipoprotein M (apo M) in sera of HCC mice. R = ppm ratio (tumour / control).

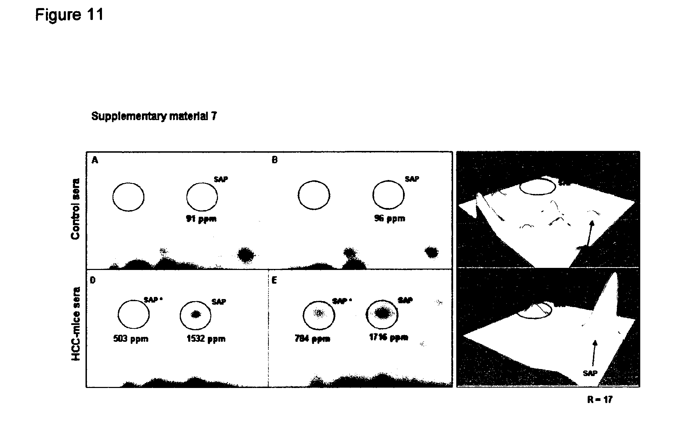

### Supplementary material 7 (Fig. 11):

Overexpression and ppm values of serum amyloid component P (SAP). (A, B): control samples. (D, E): sera of HCC mice, the second SAP isoform, SAP*, is identified in sera of HCC mice only. (C, F): 3-D view of SAP spots in control and HCC mice respectively. R = ppm ratio (tumour / control).

### Supplementary material 8 (Fig. 12)

Overexpression and ppm values of two A2MG-fragments in sera of HCC mice (E, F, G, H). The x and y isoforms (A, E). R = ppm ratio (tumour / control).

### Supplementary material 9 (Fig. 13):

Down-regulation of glutathione peroxidase 3 (Gpx3). (A, C): control samples, gpx3 is present in two isoforms, Gpx3* and Gpx3. (B, D): tumour samples, the isoform Gpx3 is down regulated, while the isoform Gpx3* is now virtually absent.

### Supplementary material 10 (Fig. 14):

(A): Expression of fibrinogens alpha, beta and gamma (Fga, Fgb, Fgg) in sera of HCC mice. (B, C, D): zoom view of the gels; Not all tumor animals carried the three fibrinogens in sera.

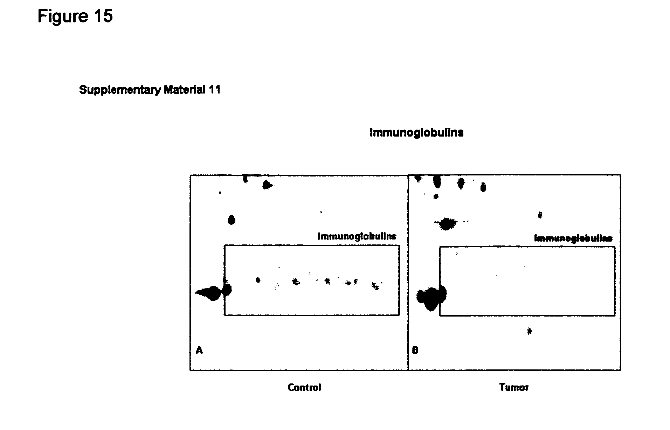

### Supplementary material 11 (Fig. 15):

Down regulation of immunoglobulins. Many immunoglobulins were virtually absent in tumor samples (B).

**Supplementary table 1.** Serum proteins A total of 2500 spots derived from 14 gels were excised and digested with trypsin (Promega). Peptides were loaded on a MTP Anchor Chip Target 600/384 (Bruker Daltonics) previously prepared with HCCA and analyzed in a MALDI-TOF-TOF spectrometer (Ultraflex I, Bruker Daltonics). Peptide matching and protein searches were performed automatically with the MASCOT 2.0 software. MASCOT scores are also reported in the table (see column "Mascot Score"). Five matching peptides (see column "N° of matched peptides") and at least 10 % peptide coverage of the theoretical sequences was the minimal requirement for an identity assignment (see column "Coverage"). The identified proteins were organized with the ProteinScape^{™} database (Bruker Daltonics) and checked individually.
In the table proteins are sorted by alphabetical order in the second column and the NCBI annotation is given in the first column. The theoretical pl, MW, and biological function are given herein. Expression of proteins and frequency of identification are reported in the column "regulation" and "gels" respectively.

**Supplementary table 2.** Sequences of the identified peptides.

**Table 1**

| **Acc. Number (NCBI)** | **Protein Name** | **Mascot Score** | **Sequence Covera ge %** | **N° of matched peptides** | **Function** | **p- value** | **Regulation (tumor / kontrol)** | **Gels** |
|---|---|---|---|---|---|---|---|---|
| gi\|191765 | Alpha fetoprotein | 234 | 39,2 | 16 | Unknown | 0,035 | 1,6 | 3 |
| gi\|14318646 | Amylase 1, salivary | 72,5 | 23,1 | 13 | Unknown | - | tumor | 3 |
| gi\|6753096 | Apolipoprotein A-I | 84,2 | 32,3 | 20 | Reverse transport of cholesterol from tissues to the liver | - | tumor | 4 |
| gi\|6753102 | Apolipoprotein E | 220 | 60,5 | 18 | Binding and transport | 0,035 | 2,2 | 12 |
| gi\|9055162 | Apolipoprotein M | 114 | 45,3 | 9 | Lipid transport. | 0,044 | 8 | 8 |
| gi\|2921308 | Carboxyl esterase precursor | 93,3 | 26,4 | 14 | Metabolism of xenobiotics | - | tumor | 2 |
| gi\|13899173 | Caspase recruitment domain protein 12 | 70,3 | 11,7 | 9 | Promotion of apoptosis. | - | tumor | 2 |
| gi\|49523333 | Clusterin (Apolipoprotein J) | 79,6 | 22,1 | 10 | Maybe binding to cells, membranes and hydrophobic proteins. | - | tumor | 4 |
| gi\|33563252 | Fibrinogen, alpha polypeptide | 204 | 47,2 | 26 | Cofactor in platelet aggregation | - | tumor | 1° |
| gi\|33859809 | Fibrinogen, beta polypeptide | 263 | 59,9 | 29 | Cofactor in platelet aggregation | - | tumor | 1° |
| gi\|19527078 | Fibrinogen, gamma polypeptide | 234 | 60,6 | 22 | Cofactor in platelet aggregation | - | tumor | 1° |
| gi\|52843238 | Glutathione peroxidase 3 | 117 | 43 | 9 | Unknown | 0,002 | 0,2 | 4 |
| gi\|40715898 | HMW-kininogen-II variant | 106 | 25,5 | 9 | Unknown | - | control | 2 |
| gi\|21594125 | Lifr protein | 95,5 | 20,4 | 10 | Unknown | - | control | 3 |
| gi\|8569601 | Major urinary protein 1 | 161 | 73,5 | 11 | Binds pheromones | 0,22 | 0,14 | 3 |
| gi\|6754654 | Mannose binding lectin (A) | 113 | 45,6 | 10 | Binds mannose and N-acetylglucosamine in a Ca++ dependent way | - | control | 5 |
| gi\|233018 | Mannose-binding protein C; MBP-C | 160 | 49,6 | 13 | Unknown | - | control | 3 |
| gi\|387437 | MHC factor B | 246 | 58,5 | 30 | . Complement system | - | tumor | 4 |
| gi\|6679182 | Orosomucoid 1 | 100 | 34,8 | 10 | Acute-phase reaction | - | control | 4 |
| gi\|53787 | Properdin (AA 5 - 441) | 143 | 32,5 | 16 | . Complement system. | 0,103 6 | 0,4 | 6 |
| gi\|34785996 | A2MG | 216 | 22,3 | 22 | Endopeptidase inhibitor. | 0,001 8 | 1,8 | 14 |
| gi\|34785996 | A2MG * | 216 | 22,3 | 22 | . Endopeptidase inhibitor. | 2,6 e-5 | 3,2 | 14 |
| gi\|6679383 | Serine (or cysteine) proteinase inhibitor, clade F, member 2; plasmin inhibitor alpha 2; alpha 2 antiplasmin; serine (or cysteine) proteinase inhibitor, clade F (alpha-2 antiplasmin, pigment epithelium derived factor), member 2 [Mus musculus] | 124 | 35 | 17 | Inhibitor for plasmin, trypsin, and chymotrypsin | 0,303 3 | 4,7 | 5 |
| gi\|38174334 | Serum amyloid P-component | 122 | 37,5 | 9 | Unknown | 0,010 3 | 10 | 7 |
| gi\|38174334 | Serum amyloid P-component * | 92,3 | 27,2 | 7 | Unknown | - | tumor | 4 |

**SUPPLEMENTARY TABLE 1**

| **Acc. Number (NCBI)** | **Protein Name** | **pl** | **Mr (kDa)** | **Mascot Score** | **Sequence Coverage %** | **N° of matched peptides** | **Function** | **Regulation (tumor / control)** | **Gels** |
|---|---|---|---|---|---|---|---|---|---|
| gi\|15277503 | ACTB protein | 5,5 | 40,2 | 249 | 61,4 | 20 | Actins are highly conserved proteins that are involved in various types of cell motility and are ubiquitously expressed in all eukaryotic cells | | 2 |
| gi\|71621 | Actin beta | 5,2 | 41,6 | 192 | 65,2 | 19 | Involved in various types of cell motility. | | 1 |
| gi\|21553101 | Afamin | 5,5 | 69,6 | 146 | 38,1 | 18 | Transport | | 5 |
| gi\|20072386 | Afm protein | 5,6 | 47,6 | 186 | 50,4 | 20 | Transport | | 11 |
| gi\|11277085 | Albumin (fragment) | 5,4 | 51,4 | 283 | 56,7 | 22 | Serum albumin, the main protein of plasma, has a good binding capacity for water, Ca(2+), Na(+), K(+), fatty acids, hormones, bilirubin and drugs. Its main function is the regulation of the colloidal osmotic pressure of blood | | 13 |
| gi\|33859506 | Albumin 1 | 5,7 | 68,7 | 421 | 65,6 | 37 | Transport | | 14 |
| gi\|42542817 | Alpha fetoprotein | 5,6 | 67,3 | 111 | 43 | 18 | Unknown | up regulated | 3 |
| gi\|191765 | Alpha fetoprotein | 5,4 | 47,2 | 234 | 39,2 | 16 | Unknown | up regulated | 9 |
| gi\|191844 | Alpha-1 protease inhibitor 2 | 5,3 | 44,8 | 166 | 60,4 | 19 | Inhibitor of serine proteases. Its primary target is elastase, but it also has a moderate affinity for plasmin and thrombin. | | 7 |
| gi\|31982171 | Alpha-2 macroglobulin MUG1 | 6 | 165,1 | 131 | 22,6 | 31 | A proteinase activates the inhibitor by specific proteolysis in the bait region, which, by an unknown mechanism leads to reaction at the cysteinyl-glutamyl internal thiol ester site and to a conformational change, whereby the proteinase is trapped and/or covalently bound to the inhibitor. | | 9 |
| | | | | | | | While in the tetrameric proteinase inhibitors steric inhibition is sufficiently strong, monomeric forms need a covalent linkage between the activated glutamyl residue of the original thiol ester and a terminal amino group of a lysine or another nucleophilic group on the proteinase, for inhibition to be effective | | |
| gi\|7304875 | Alpha-2-HS-glycoprotein (Fetuin) | 6,1 | 37,3 | 98,8 | 48,4 | 13 | Probably involved in differentiation. | | 8 |
| gi\|14318646 | Amylase 1, salivary | 6,6 | 57,6 | 72,5 | 23,1 | 13 | Unknown | tumor | 3 |
| gi\|425520 | Anti-colorectal carcinoma light chain | 6,2 | 26,5 | 88,8 | 40,7 | 6 | Unknown | | 1 |
| gi\|26345182 | Apolipoprotein A1 (Unnamed protein product) | 5,4 | 30,6 | 163 | 62,5 | 18 | Participates in the reverse transport of cholesterol from tissues to the liver for excretion by promoting cholesterol efflux from tissues and by acting as a cofactor for the lecithin cholesterol acyltransferase (LCAT). | | 12 |
| gi\|14789706 | Apolipoprotein A4 | 5,3 | 45 | 292 | 73,9 | 25 | May have a role in chylomicrons and VLDL secretion and catabolism. Required for efficient activation of lipoprotein lipase by ApoC-II; potent activator of LCAT. Apoa-IV is a major component of HDL and chylomicrons. | | 10 |
| gi\|6753096 | Apolipoprotein A-I | 5,4 | 30,5 | 84,2 | 32,3 | 20 | Participates in the reverse transport of cholesterol from tissues to the liver for excretion by promoting cholesterol efflux from tissues and by acting as a cofactor for the lecithin cholesterol acyltransferase (LCAT). | tumor | 4 |
| gi\|6753102 | Apolipoprotein E | 5,7 | 33,2 | 220 | 60,5 | 18 | Mediates the binding, internalization, and catabolism of lipoprotein particles. It can serve as a ligand for the LDL (apo B/E) receptor and for the specific apo-E receptor (chylomicron remnant) of hepatic tissues. | up regulated | 12 |
| gi\|1938223 | Apolipoprotein H (beta-2 glycoprotein I ) | 9,7 | 36,3 | 153 | 59,1 | 15 | Binds to various kinds of negatively charged substances such as heparin, phospholipids, and dextran sulfate. May prevent activation of the intrinsic blood coagulation cascade by binding to phospholipids on the surface of damaged cells. | | 12 |
| gi\|9055162 | Apolipoprotein M | 6,1 | 21,3 | 114 | 45,3 | 9 | Probably involved in lipid transport. | up regulated | 8 |
| gi\|2921308 | Carboxylesterase precursor | 5 | 61,1 | 93,3 | 26,4 | 13 | Involved in the detoxification of xenobiotics and in the activation of ester and amide prodrugs. Involved in the extracellular metabolism of lung surfactant | tumor | 2 |
| gi\|13899173 | Caspase recruitment domain protein 12 | 6,27 | 116,1 | 70,3 | 11,7 | 9 | Plays a role in the promotion of apoptosis | tumor | 2 |
| gi\|49523333 | Clusterin (Apolipoprotein J) | 5,4 | 51,7 | 79,6 | 22,1 | 10 | Not yet clear. It is known to be expressed in a variety of tissues and it seems to be able to bind to cells, membranes and hydrophobic proteins. It has been associated with programmed cell death. | tumor | 4 |
| gi\|6753798 | Coagulation factor II (prothrombin) | 6 | 70,3 | 215 | 45 | 24 | Thrombin, which cleaves bonds after Arg and Lys, converts fibrinogen to fibrin and activates factors V, VII, VIII, XIII, and, in complex with thrombomodulin, protein C. | | 10 |
| gi\|13624321 | Coagulation factor XIII, beta subunit | 6,6 | 76 | 190 | 32,8 | 19 | The B chain of factor XIII is not catalytically active, but is thought to stabilize the A subunits and regulate the rate of transglutaminase formation by thrombin. | | 3 |
| gi\|6996919 | Complememnt histocompatibility 2, complement component factor B | 7,1 | 85 | 264 | 52 | 30 | Cell proliferation and complement activation, alternative pathway. | | 12 |
| gi\|28175786 | Complement C3 precursor (HSE-MSF) | 6,4 | 186,4 | 289 | 29 | 40 | C3 plays a central role in the activation of the complement system. Its processing by C3 convertase is the central reaction in both classical and alternative complement pathways. After activation C3b can bind covalently, via its reactive thiolester, to cell surface carbohydrates or immune aggregates. | | 12 |
| gi\|220349 | Complement C4 | 9,1 | 59,2 | 217 | 47,9 | 22 | C4 plays a central role in the activation of the classical pathway of the complement system. It is processed by activated C1 which remove from the alpha chain the C4a anaphylatoxin. | | 8 |
| gi\|309119 | Complement C4b-binding protein precursor | 6 | 46,6 | 93,9 | 33,8 | 11 | Controls the pathway of complement activation. It accelerates the degradation of the C4bC2a complex. It interacts with SAP | | 1 |
| gi\|15030019 | Complement component 6 | 5,7 | 86,6 | 207 | 39 | 27 | Involved in the formation of the lytic c5b-9m complex | | 2 |
| gi\|27462724 | Complement component C1SA | 4,8 | 76,9 | 72,3 | 18,3 | 9 | C1s B chain is a serine protease that combines with C1q and C1s to form C1, the first component of the classical pathway of the complement system. C1r activates C1 s so that it can, in turn, activate C2 and C4 | | 1 |
| gi\|19072788 | Complement component factor h | 6,6 | 139 | 264 | 32 | 30 | Factor H functions as a cofactor in the inactivation of C3b by factor I and also increases the rate of dissociation of the C3bBb complex (C3 convertase) and the (C3b)NBB complex (C5 convertase) in the alternative complement pathway | | 10 |
| gi\|6671744 | Complement component factor i | 9,2 | 67,2 | 87,6 | 21,7 | 7 | Responsible for cleaving the alpha chains of C4b and C3b in the presence of the cofactors C4 binding protein and factor H respectively. | | 6 |
| gi\|9954973 | Complement D Chain D, N-Terminally Truncated C3dg Fragment | 5 | 31,1 | 75,1 | 38,3 | 10 | C3 plays a central role in the activation of the complement system. Its processing by C3 convertase is the central reaction in both classical and alternative complement pathways. After activation C3b can bind covalently, via its reactive thiolester, to cell surface carbohydrates or immune aggregates. Derived from proteolytic degradation of | | 1 |
| | | | | | | | complement C3, C3a anaphylatoxin is a mediator of local inflammatory process. It induces the contraction of smooth muscle, increases vascular permeability and causes histamine release from mast cells and basophilic leukocytes. The short isoform has B-cell stimulatory activity. | | |
| gi\|6754132 | Complement histocompatibility 2, Q region locus 10 | 5,1 | 37,3 | 217 | 53,2 | 18 | Involved in the presentation of foreign antigens to the immune system | | 9 |
| gi\|54173 | Contraspin | 5 | 46,7 | 259 | 60,5 | 22 | Contrapsin inhibits trypsin-like proteases | | 6 |
| gi\|38614350 | Cp protein | 5,5 | 121,1 | 248 | 40,5 | 36 | Ceruloplasmin is a blue, copper binding (6-7 atoms per molecule) glycoprotein found in plasma. Four possible functions are ferroxidase activity, amine oxidase activity, copper transport and homeostasis, and superoxide dismutase activity. | | 13 |
| gi\|19388017 | Cpn2 protein | 5,5 | 62 | 112 | 29,7 | 11 | The 83 kDa subunit binds and stabilizes the catalytic subunit at 37 degrees Celsius and keeps it in circulation. Under some circumstances it may be an allosteric modifier of the catalytic subunit | | 5 |
| gi\|11055360 | Epidermal growth factor receptor isoform 2 | 6,8 | 71,4 | 239 | 43,4 | 22 | cell proliferation - cellular morphogenesis -epidermal growth factor receptor signaling pathway positive regulation of cell proliferation - protein amino acid autophosphorylation - regulation of peptidyl-tyrosine phosphorylation signal transduction | | 9 |
| gi\|6679689 | Esterase 1 | 4,9 | 61,1 | 164 | 46,8 | 18 | Involved in the detoxification of xenobiotics and in the activation of ester and amide prodrugs. Involved in the extracellular metabolism of lung surfactant | | 9 |
| gi\|33563252 | Fibrinogen, alpha polypeptide | 7 | 61,3 | 204 | 47,2 | 26 | Fibrinogen has a double function: yielding monomers that polymerize into fibrin and acting as a cofactor in platelet aggregation | tumor | 1° |
| gi\|33859809 | Fibrinogen, beta polypeptide | 6,5 | 54,8 | 263 | 59,9 | 29 | Fibrinogen has a double function: yielding monomers that polymerize into fibrin and acting as a cofactor in platelet aggregation | tumor | 1° |
| gi\|19527078 | Fibrinogen, gamma polypeptide | 5,5 | 49,4 | 234 | 60,6 | 22 | Fibrinogen has a double function: yielding monomers that polymerize into fibrin and acting as a cofactor in platelet aggregation | tumor | 1° |
| gi\|1181242 | Fibronectin | 5,2 | 160,9 | 161 | 23,5 | 25 | Fibronectins bind cell surfaces and compounds including collagen, fibrin, heparin, DNA, and actin. They are involved in cell adhesion, cell motility, opsonization, wound healing, and maintenance of cell shape. Interaction with TNR mediates inhibition of cell adhesion and neurite outgrowth. | | 5 |
| gi\|46849812 | Fibronectin 1 | 5,4 | 272,5 | 349 | 31 | 59 | Fibronectins bind cell surfaces and various compounds including collagen, fibrin, heparin, DNA, and actin. Fibronectins are involved in cell adhesion, cell motility, opsonization, wound healing, and maintenance of cell shape. Interaction with TNR mediates inhibition of cell adhesion and neurite outgrowth. | | 9 |
| gi\|28916693 | Gelsolin | 5,8 | 85,9 | 255 | 47,6 | 28 | Calcium-regulated, actin modulating protein that binds to the plus (or barbed) ends of actin monomers or filaments, preventing monomer exchange (end-blocking or capping). It can promote the assembly of monomers into filaments (nucleation) as well as sever filaments already formed. | | 12 |
| gi\|52843238 | Glutathione peroxidase 3 | 9,1 | 25,4 | 117 | 43 | 9 | Unknown | down regulated | 4 |
| gi\|17512357 | Gpld1 protein | 6,3 | 93,6 | 125 | 19,4 | 16 | This protein hydrolyzes the inositol phosphate linkage in proteins anchored by phosphatidylinositol glycans (GPI-anchor) thus releasing these proteins from the membrane. | | 3 |
| gi\|1694789 | GRS protein | 5,3 | 20,1 | 70 | 44,6 | 5 | Retards apoptosis induced by IL-3 deprivation. May function in the response of hemopoietic cells to external signals and in maintaining endothelial survival during infection | | 1 |
| gi\|37719755 | GUGU alpha | 6,2 | 44,4 | 155 | 50,6 | 16 | Multifunction | | 3 |
| gi\|23956086 | Hemopexin | 9 | 51,3 | 238 | 55,7 | 23 | Binds heme and transports it to the liver for breakdown and iron recovery, after which the free hemopexin returns to the circulation | | 11 |
| gi\|50082914 | High molecular weight kininogen I isoform DeltaD5 | 4,7 | 53,2 | 89,2 | 27,7 | 11 | (1) Kininogens are inhibitors of thiol proteases; | | 3 |
| | | | | | | | (2) HMW-kininogen plays an important role in blood coagulation by helping to position optimally prekallikrein and factor XI next to factor XII; ; | | |
| | | | | | | | (3) HMW kininogen inhibits the thrombin- and plasmin-induced aggregation of thrombocytes; | | |
| | | | | | | | (4) the active peptide bradykinin that is released from HMW-kininogen shows a variety of physiological effects: (4A) influence in smooth muscle contraction, (4B) induction of hypotension, (4C) natriuresis and diuresis, (4D) decrease in blood glucose level, (4E) it is a mediator of inflammation and causes (4E1) increase in vascular permeability, (4E2) stimulation of nociceptors (4E3) release of other mediators of inflammation (e.g. prostaglandins), (4F) it has a cardioprotective effect (directly via bradykinin action, indirectly via endothelium-derived relaxing factor action) | | |
| | | | | | | | (5) LMW kininogen inhibits the aggregation of thrombocytes; | | |
| | | | | | | | (6) LMW kininogen is in contrast to HMW kininogen not involved in blood clotting | | |
| gi\|40715898 | HMW-kininogen-II variant | 6 | 51 | 86,4 | 25,8 | 12 | (1) Kininogens are inhibitors of thiol proteases; | control | 2 |
| | | | | | | | (2) HMW-kininogen plays an important role in blood coagulation by helping to position optimally prekallikrein and factor XI next to factor XII; | | |
| | | | | | | | (3) HMW kininogen inhibits the thrombin- and plasmin-induced aggregation of thrombocytes; | | |
| | | | | | | | (4) the active peptide bradykinin that is released from HMW-kininogen shows a variety of physiological effects: (4A) influence in smooth muscle contraction, (4B) induction of hypotension, (4C) natriuresis and diuresis, (4D) decrease in blood glucose level, (4E) it is a mediator of inflammation and causes (4E1) increase in vascular permeability, | | |
| | | | | | | | (4E2) stimulation of nociceptors (4E3) release of other mediators of inflammation (e.g. prostaglandins), (4F) it has a cardioprotective effect (directly via bradykinin action, indirectly via endothelium-derived relaxing factor action); | | |
| | | | | | | | (5) LMW kininogen inhibits the aggregation of thrombocytes; | | |
| | | | | | | | (6) LMW kininogen is in contrast to HMW kininogen not involved in blood clotting | | |
| gi\|31615671 | Ig (A Chain A, Crystal Structure Of Fab Fragment Of Antibody Hyhel-26 Complexed With Lysozyme) | 5,5 | 23,6 | 76,4 | 44,4 | 6 | Host defense | control | 3 |
| gi\|4930001 | Ig (A Chain A, Idiotope-Anti Idiotope Fab-Fab Complex) | 6 | 23,7 | 65,8 | 41,6 | 7 | Host defense | control | 4 |
| gi\|11514687 | Ig (A Chain A, Lyme Disease Antigen Ospa In Complex With Neutralizing Antibody Fab La-2) | 7,4 | 23,6 | 78,7 | 49,3 | 6 | Host defense | control | 1 |
| gi\|42543442 | Ig (A Chain A, S25-2- Kdo Monosaccharide Complex) | 9,4 | 24,2 | 89,2 | 44,7 | 7 | Host defense | control | 2 |
| gi\|7766934 | Ig (A Chain A, Structure Of An Activity Suppressing Fab Fragment To Cytochrome P450 Aromatase) | 6 | 23,8 | 78,2 | 42,9 | 6 | Host defense | control | 1 |
| gi\|27373551 | Ig (antibody variable domain) | 7 | 12,7 | 68,5 | 48,7 | 5 | Host defense | tumor | 1 |
| gi\|1870378 | Ig (Anti-DNA immunoglobulin light chain IgG) | 6,3 | 11,6 | 109 | 57 | 6 | Host defense | up regulated | 5 |
| gi\|12002896 | Ig (anti-human apolipoprotein A monoclonal antibody mAb(a)23L kappa light chain) | 7,4 | 23,5 | 82,4 | 44,6 | 7 | Host defense | control | 1 |
| gi\|349893 | Ig (C Chain C, Fab (Igg2a,Kappa) Fragment (26-10) Complex With Digoxin) | 7,8 | 24 | 114 | 47,5 | 8 | Host defense | control | 1 |
| gi\|47059057 | Ig (gamma-2b-immunoglobulin) | 8,9 | 51,9 | 69 | 29 | 8 | Host defense | tumor | 2 |
| gi\|1806128 | Ig (immunoglobulin constant heavy chain) | 9,3 | 51,7 | 88,4 | 41,9 | 12 | Host defense | | 5 |
| gi\|10121892 | Ig (immunoglobulin IgM MP 18-3-117 kappa light chain) | 5,2 | 26 | 76,9 | 42,1 | 7 | Host defense | control | 3 |
| gi\|196723 | Ig (immunoglobulin kappa chain VK-1) | 5,6 | 10,9 | 125 | 61,6 | 7 | Host defense | control | 2 |
| gi\|18033701 | Ig (immunoglobulin light chain constant region) | 5,5 | 11,3 | 65,6 | 70,2 | 5 | Host defense | control | 1 |
| gi\|13399686 | Ig (L Chain L, 64m-2 Antibody Fab Complexed With D(5ht)(6-4)t) | 6 | 23,9 | 115 | 70,5 | 10 | Host defense | control | 5 |
| gi\|3212470 | Ig (L Chain L, Anti Taq Fab Tp7) | 5,7 | 23,1 | 104 | 58,6 | 5 | Host defense | control | 2 |
| gi\|5542523 | Ig (L Chain L, Bactericidal Antibody Against Neisseria Meningitidis) | 9,3 | 24,1 | 107 | 66,2 | 11 | Host defense | control | 2 |
| gi\|16975338 | Ig (L Chain L, Crystal Structure Of Immunoglobulin Fab Fragment Complexed With 17-Beta-Estradiol) | 6,6 | 23,6 | 102 | 69,2 | 11 | Host defense | control | 2 |
| gi\|18655521 | Ig (L Chain L, Crystal Structure Of The Fab Fragment Of The Mouse Anti- Human Fas Antibody Hfe7a) | 4,8 | 23,9 | 81,1 | 56,4 | 8 | Host defense | | 2 |
| gi\|1942810 | Ig (L Chain L, Fab Fragment Of A Neutralizing Antibody Directed Against An Epitope Of Gp41 From Hiv-1) | 6,4 | 24,2 | 124 | 80 | 12 | Host defense | control | 2 |
| gi\|7546516 | Ig (L Chain L, Fab Fragment Of Neutralising Monoclonal Antibody 4c4 Complexed With G-H Loop From Fmdv) | 5,6 | 23,7 | 72,4 | 37 | 5 | Host defense | control | 1 |
| gi\|17943084 | Ig (L Chain L, Structural Basis For Disfavored Elimination Reaction In Catalytic Antibody 1d4) | 7,8 | 24,2 | 118 | 59,1 | 8 | Host defense | control | 2 |
| gi\|2624743 | Ig (L Chain L, Structure Of A Catalytic Antibody, Igg2a Fab Fragment) | 7,3 | 24 | 69,9 | 45,7 | 8 | Host defense | control | 1 |
| gi\|32263981 | Ig (mAb immunoglobulin light chain variable region) | 9,9 | 11,6 | 80,9 | 59,8 | 7 | Host defense | control | 1 |
| gi\|18568342 | Ig (monoclonal antibody BBK-2 light chain) | 6,5 | 26,1 | 77,8 | 51,7 | 8 | Host defense | control | 1 |
| gi\|52382 | Ig (mu(b) immunoglobulin heavy chain) | 6,4 | 50 | 153 | 40,7 | 19 | Host defense | control | 1 |
| gi\|7439167 | Ig (PC4436 monoclonal antibody 13-1 heavy chain) | 8,7 | 49,3 | 92,6 | 29,1 | 8 | Host defense | | 2 |
| gi\|15824610 | Ig (Pterin-mimicking anti-idiotope kappa chain variable region) | 7,8 | 12 | 75,4 | 77,5 | 5 | Unknown | control | 1 |
| gi\|780265 | Ig gamma-1 chain C region (15C5) (fragment) | 9 | 17,3 | 70,5 | 38,8 | 8 | Host defense | | 3 |
| gi\|1799551 | Ig gamma-chain, secrete type | 9,4 | 36,2 | 88,5 | 31 | 9 | Host defense | | 1 |
| gi\|223428 | Ig H-C allotype gamma2b | 7,8 | 36,6 | 174 | 56 | 19 | Host defense | | 9 |
| gi\|224008 | Ig J chain | 4,4 | 15,8 | 111 | 42,4 | 9 | Host defense | control | 2 |
| gi\|12832551 | Ig K (unnamed protein product) | 6,4 | 23,2 | 98 | 43,1 | 8 | Host defense | control | 2 |
| gi\|896077 | Ig kappa chain | 7,6 | 8,7 | 90,1 | 74,7 | 6 | Host defense | control | 3 |
| gi\|896089 | Ig kappa chain | 9,5 | 10,5 | 82 | 82,3 | 6 | Host defense | control | 1 |
| gi\|896103 | Ig kappa chain | 4,3 | 10,3 | 64,7 | 57,9 | 5 | Host defense | control | 3 |
| gi\|896107 | Ig kappa chain | 4,6 | 9,1 | 71,6 | 71,8 | 5 | Host defense | control | 1 |
| gi\|7513693 | Ig kappa chain (Mab03-1) | 6,4 | 23,6 | 104 | 58,9 | 11 | Host defense | control | 1 |
| gi\|110429 | Ig kappa chain V region (9.42 | 9,1 | 10,4 | 73,4 | 58,5 | 6 | Host defense | control | 1 |
| gi\|49258884 | Ig kappa chain V region (D444) | 9,3 | 11,8 | 70,1 | 50,9 | 7 | Host defense | control | 3 |
| gi\|930226 | Ig kappa chain V region (hybridoma NC19-E11) | 5,1 | 11 | 90 | 55,6 | 8 | Host defense | control | 2 |
| gi\|125796 | Ig kappa chain V-III region PC 2880/PC 1229 | 5,2 | 12 | 73,7 | 66,7 | 6 | Host defense | down regulated | 1 |
| gi\|197064 | Ig kappa chain V-region (V-Jk1) protein | 7,3 | 11,9 | 92,3 | 99,1 | 7 | Host defense | control | 1 |
| gi\|1613779 | Ig kappa light chain | 5,5 | 23,9 | 69,6 | 38,3 | 10 | Host defense | control | 1 |
| gi\|284924 | Ig light chain V region (clone 185-c1) | 6,1 | 10,7 | 86,9 | 75,5 | 6 | Host defense | tumor | 1 |
| gi\|494164 | Igg2a Fab Fragment (Fab 179) | 5,7 | 23,9 | 65,7 | 39,6 | 5 | Host defense | control | 1 |
| gi\|229901 | Igg2b, Kappa | 5,8 | 23,7 | 108 | 70,1 | 12 | Host defense | control | 5 |
| gi\|62204734 | Inter alpha-trypsin inhibitor, heavy chain 4 | 6,1 | 104 | 220 | 37 | 29 | Unknown | | 5 |
| gi\|16418335 | Leucine-rich alpha-2-glycoprotein | 6 | 37,4 | 71 | 23,4 | 8 | Unknown | | 3 |
| gi\|21594125 | Lifr protein (leukemia inhibitory factor receptor) | 6,5 | 81,3 | 95,5 | 20,4 | 10 | The WSXWS motif appears to be necessary for proper protein folding and thereby efficient intracellular transport and cell-surface receptor binding | control | 3 |
| gi\|8569601 | Major urinary protein 1 | 4,9 | 20,6 | 161 | 73,5 | 11 | Binds pheromones | down regulated | 3 |
| gi\|6754654 | Mannose binding lectin (A) | 7,3 | 25,4 | 113 | 45,6 | 10 | Binds mannose and N-acetylglucosamine in a Ca++ dependent way. Is capable of host defense against pathogens, by activating the classical complement pathway independently of the antibody | control | 5 |
| gi\|233018 | Mannose binding protein C | 4,9 | 25,9 | 160 | 49,6 | 11 | Binds mannose and N-acetylglucosamine. Host defense against pathogens, by activating the complement pathway independently of the antibody. | control | 3 |
| gi\|2118830 | MHC class I histocompatibility antigen H-2 Q10 alpha chain | 5,1 | 37 | 240 | 53,7 | 21 | Involved in the presentation of foreign antigens to the immune system | | 9 |
| gi\|387437 | MHC factor B | 8,7 | 54 | 246 | 58,5 | 30 | Factor B which is part of the alternate pathway of the complement system is cleaved by factor D into 2 fragments: Ba and Bb. Bb, a serine protease, then combines with complement factor 3b to generate the C3 or C5 convertase | tumor | 4 |
| gi\|55217 | Murine valosin-containing protein | 5,1 | 89,3 | 91,6 | 19,1 | 12 | Necessary for the fragmentation of Golgi stacks during mitosis and for their reassembly after mitosis. Involved in the formation of the transitional endoplasmic reticulum (tER). The transfer of membranes from the endoplasmic reticulum to the Golgi apparatus occurs via 50-70 nm transition vesicles which derive from part-rough, part-smooth transitional elements of the endoplasmic reticulum (tER). | | 1 |
| | | | | | | | Vesicle budding from the tER is an ATP-dependent process. The ternary complex containing UFD1 L, VCP and NPL4 binds ubiquitinated proteins and is necessary for the export of misfolded proteins from the ER to the cytoplasm, where they are degraded by the proteasome. The NPL4-UFD1L-VCP complex regulates spindle disassembly at the end of mitosis and is necessary for the formation of a closed nuclear envelope | | |
| gi\|6679182 | Orosomucoid 1 | 5,4 | 23,9 | 100 | 34,8 | 10 | Appears to function in modulating the activity of the immune system during the acute-phase reaction | control | 4 |
| gi\|31982113 | Plasminogen [Mus musculus] | 6,1 | 90,8 | 449 | 61,5 | 53 | Plasmin dissolves the fibrin of blood clots and acts as a proteolytic factor in a variety of other processes including embryonic development, tissue remodeling, tumor invasion, and inflammation; in ovulation it weakens the walls of the Graafian follicle. It activates the urokinase type plasminogen activator, collagenases and several complement zymogens, such as C1 and C5. It cleaves fibrin, fibronectin, thrombospondin, laminin and von Willebrand factor. Its role in tissue remodeling and tumor invasion may be modulated by CSPG4 | | 10 |
| gi\|2144495 | PLMS plasmin | 6 | 90,8 | 352 | 51,4 | 38 | Plasmin dissolves the fibrin of blood clots and acts as a proteolytic factor in a variety of other processes including embryonic development, tissue remodeling, tumor invasion, and inflammation; in ovulation it weakens the walls of the Graafian follicle. It activates the urokinase type plasminogen activator, collagenases and several complement zymogens, such as C1 and C5. It cleaves fibrin, fibronectin, thrombospondin, laminin and von Willebrand factor. Its role in tissue remodeling and tumor invasion may be modulated by CSPG4 | n.v.o. | 8 |
| gi\|6680608 | Pregnancy zone protein | 6,2 | 165,8 | 113 | 13,7 | 14 | Endopeptidase inhibitor activity | | 8 |
| gi\|53787 | Properdin (AA 5 - 441) | 8,4 | 47,5 | 143 | 32,5 | 16 | A positive regulator of the alternate pathway of complement. It binds to and stabilizes the C3- and C5 convertase enzyme complexes. | up regulated | 6 |
| gi\|49868 | Put. beta-actin (aa 27-375) | 5,7 | 39,2 | 136 | 44,4 | 16 | Involved in various types of cell motility. | | 1 |
| gi\|34785996 | Pzp protein | 6,2 | 165,9 | 187 | 28,2 | 32 | Unknown | up regulated | 14 |
| gi\|33859612 | Retinol binding protein 4 | 5,6 | 23,2 | 147 | 51,7 | 14 | Delivers retinol from the liver stores to the peripheral tissues. In plasma, the RBP-retinol complex interacts with transthyretin, this prevents its loss by filtration through the kidney glomeruli. | | 9 |
| gi\|6678083 | Serine (or cysteine) proteinase inhibitor, clade A, member 1 a | 5,3 | 45,9 | 149 | 62,7 | 20 | Inhibitor of serine proteases. Can inhibit trypsin and chymotrypsin; relatively ineffective against elastase | | 6 |
| gi\|15029662 | Serine (or cysteine) proteinase inhibitor, clade A, member 1 a | 5,3 | 46 | 143 | 51,6 | 17 | Inhibitor of serine proteases. Its primary target is elastase, but it also has a moderate affinity for plasmin and thrombin. | | 8 |
| gi\|6678085 | Serine (or cysteine) proteinase inhibitor, clade A, member 1d | 5,2 | 46 | 171 | 47 | 14 | Inhibitor of serine proteases. Can inhibit trypsin and chymotrypsin; relatively ineffective against elastase | | 1 |
| gi\|6678087 | Serine (or cysteine) proteinase inhibitor, clade A, member 1 e | 5,4 | 45,9 | 154 | 41,6 | 19 | Does not inhibit elastase or chymotrypsin. No target protease has been identified to date | | 5 |
| gi\|18044689 | Serine (or cysteine) proteinase inhibitor, clade A, member 3K | 5 | 46,9 | 99,1 | 40 | 13 | serine-type endopeptidase inhibitor activity | | 1 |
| gi\|18252782 | Serine (or cysteine) proteinase inhibitor, clade C (antithrombin), member 1 | 6,1 | 52 | 195 | 61,7 | 25 | Most important serine protease inhibitor in plasma that regulates the blood coagulation cascade. AT-III inhibits thrombin as well as factors IXa, Xa and XIa. Its inhibitory activity is greatly enhanced in the presence of heparin. | | 7 |
| gi\|6679383 | Serine (or cysteine) proteinase inhibitor, clade F, member 2 | 5,8 | 54,9 | 106 | 34,6 | 17 | Serine-type endopeptidase inhibitor activity | up regulated | 5 |
| gi\|15929675 | Serpina1a protein | 5,4 | 45,6 | 219 | 62 | 23 | Inhibitor of serine proteases. Can inhibit trypsin and chymotrypsin; relatively ineffective against elastase. | | 12 |
| gi\|14602605 | Serpina1a protein | 5,3 | 46 | 224 | 62 | 24 | Inhibitor of serine proteases. Can inhibit trypsin and chymotrypsin; relatively ineffective against elastase. | | 7 |
| gi\|38174334 | Serum amyloid P-component | 6,2 | 26,2 | 122 | 37,5 | 9 | Unknown | up regulated | 7 |
| gi\|20330802 | Transferrin | 6,8 | 76,7 | 159 | 40 | 47 | Transferrins are iron binding transport proteins which can bind two Fe(3+) ions in association with the binding of an anion, usually bicarbonate. It is responsible for the transport of iron from sites of absorption and heme degradation to those of storage and utilization. Serum transferrin may also have a further role in stimulating cell proliferation. | | 13 |
| gi\|19354093 | Transthyretin | 5,5 | 15,8 | 155 | 96,6 | 11 | Thyroid hormone-binding protein. Probably transports thyroxine from the bloodstream to the brain. | | 8 |
| gi\|111243 | Vitamin D-binding protein | 5,2 | 53,1 | 288 | 65,3 | 30 | Multifunction | | 12 |
| gi\|1083568 | Zinc-alpha 2-glycoprotein | 5,7 | 33,5 | 94,6 | 40 | 10 | Stimulates lipid degradation in adipocytes and causes the extensive fat losses associated with some advanced cancers | | 1 |

**Supplementary Table 2**

| **NCBI Number** | **Protein** | **Identified Peptides** |
|---|---|---|
| gi\|15277503 | ACTB protein | AGFAGDDAPR |
| | | AVFPSIVGRPR |
| | | DLYANTVLSGGTTMYPGIADR |
| | | DSYVGDEAQSKR |
| | | GYSFTTTAER |
| | | GYSFTTTAEREIVR |
| | | HQGVMVGMGQK ox ox |
| | | HQGVMVGMGQKDSYVGDEAQSK ox ox |
| | | IIAPPERK |
| | | IKIIAPPER |
| | | ILTERGYSFTTTAER |
| | | IWHHTFYNELR |
| | | KDLYANTVLSGGTTMYPGIADR ox |
| | | LCYVALDFEQEMATAASSSSLEK cam ox |
| | | LDLAGRDLTDYLMK |
| | | LDLAGRDLTDYLMK ox |
| | | QEYDESGPSIVHR |
| | | QEYDESGPSIVHRK |
| | | SYELPDGQVITIGNER |
| | | TTGIVMDSGDGVTHTVPIYEGYALPHAILR ox |
| | | VAPEEHPVLLTEAPLNPK |
| gi\|71621 | Actin beta | AGFAGDDAPR |
| | | AVFPSIVGRPR |
| | | DDDIAALVVDNGSGMCK cam ox |
| | | DLYANTVLSGGTTMYPGIADR ox |
| | | DSYVGDEAQSKR |
| | | EITALAPSTMK ox |
| | | GYSFTTTAER |
| | | GYSFTTTAEREIVR |
| | | HQGVMVGMGQK ox |
| | | IIAPPERK |
| | | IKIIAPPER |
| | | IWHHTFYNELR |
| | | KDLYANTVLSGGTTMYPGIADR ox |
| | | LCYVALDFEQEMATAASSSSLEK cam ox |
| | | LDLAGRDLTDYLMK ox |
| | | QEYDESGPSIVHR |
| | | QEYDESGPSIVHRK |
| | | SYELPDGQVITIGNER |
| | | TTGIVMDSGDGVTHTVPIYEGYALPHAILR ox |
| | | VAPEEHPVLLTEAPLNPK |
| gi\|21553101 | Afamin | AAPQLPMEELVSLSK |
| | | ANVGFLPPFPTLDPEEK |
| | | SLAMVQQECNQFQELGKDTLQR ox cam |
| | | CWADNTLPECSK cam cam |
| | | DRCWADNTLPECSK cam cam |
| | | DSDPDKFFAEFIYEYSR |
| | | EACIINANKDDRPEGLSLR cam |
| | | FTAAREECSEVQEPESCFSPESSK cam cam |
| | | FTESENVCQER cam |
| | | HFLVKFTK |
| | | IGFKDLTTLLEDVSSMYEGCCEGDVVHCIR cam cam |
| | | cam |
| | | KANVGFLPPFPTLDPEEK |
| | | RLCFFYNK cam |
| | | TINPAVDHCCK cam cam |
| | | TINPAVDHCCKTDFAFR cam cam |
| | | VLNSINVAVFSK |
| | | VMLDYRDR |
| | | VMLDYRDR ox |
| | | VYMDFLEDCCSR ox cam cam |
| gi\|20072386 | Afm protein | ANVGFLPPFPTLDPEEK |
| | | ASSSYQRNVCGALIK cam |
| | | ATCFQAKAAPITQYLK cam |
| | | CWADNTLPECSK cam cam |
| | | DDRPEGLSLREAK |
| | | DSDPDKFFAEFIYEYSR |
| | | EACIINANKDDRPEGLSLR cam |
| | | EAKFTESENVCQER cam |
| | | FTESENVCQER cam |
| | | ITKVYMDFLEDCCSR ox cam cam |
| | | KANVGFLPPFPTLDPEEK |
| | | LCFFYNKK cam |
| | | NVCGALIKFGPK cam |
| | | QFQELGKDTLQR |
| | | RHPDLSTPELLR |
| | | SLAMVQQECK ox cam |
| | | SQSQVVNHICSK cam |
| | | SQSQVVNHICSKQDSISSK cam |
| | | VLNSINVAVFSK |
| | | VMLDYRDR |
| | | VYMDFLEDCCSR ox cam cam |
| gi\|11277085 | Albumin (fragment) | AETFTFHSDICTLPEKEK cam |
| | | APQVSTPTLVEAAR |
| | | CCAEANPPACYGTVLAEFQPLVEEPK cam cam cam |
| | | CCAEANPPACYGTVLAEFQPLVEEPKNLVKcam |
| | | cam cam |
| | | CCSGSLVER cam cam |
| | | CCTLPEDQRLPCVEDYLSAILNR cam cam cam |
| | | DVFLGTFLYEYSR |
| | | ENPTTFMGHYLHEVAR |
| | | HPDYSVSLLLR |
| | | LGEYGFQNAILVR |
| | | LPCVEDYLSAILNR cam |
| | | LQTCCDKPLLK cam cam |
| | | LQTCCDKPLLKK cam cam |
| | | LSQTFPNADFAEITK |
| | | LSQTFPNADFAEITKLATDLTK |
| | | NYAEAKDVFLGTFLYEYSR |
| | | QEPERNECFLQHK cam |
| | | RHPDYSVSLLLR |
| | | RPCFSALTVDETYVPK cam |
| | | TNCDLYEKLGEYGFQNAILVR cam |
| | | VCLLHEKTPVSEHVTK cam |
| | | YMCENQATISSK cam |
| | | YTQKAPQVSTPTLVEAAR |
| gi\|33859506 | Albumin 1 | SEIAHRYNDLGEQHFK |
| | | AETFTFHSDICTLPEKEK cam |
| | | AFKAWAVAR |
| | | AHCLSEVEHDTMPADLPAIAADFVEDQEVCK cam ox |
| | | cam |
| | | APQVSTPTLVEAAR |
| | | APQVSTPTLVEAARNLGR |
| | | CCAEANPPACYGTVLAEFQPLVEEPK cam cam cam |
| | | CCSGSLVER cam cam |
| | | CCTLPEDQR cam cam |
| | | CCTLPEDQRLPCVEDYLSAILNR cam cam cam |
| | | DDNPSLPPFERPEAEAMCTSFK ox cam |
| | | DVFLGTFLYEYSR |
| | | DVFLGTFLYEYSRR |
| | | EAHKSEIAHR |
| | | ECCHGDLLECADDRAELAK cam cam cam |
| | | EFKAETFTFHSDICTLPEK cam |
| | | ENPTTFMGHYLHEVAR ox |
| | | ENYGELADCCTK cam cam |
| | | HPDYSVSLLLR |
| | | LCAIPNLRENYGELADCCTK cam cam cam |
| | | LGEYGFQNAILVR |
| | | LPCVEDYLSAILNR cam |
| | | LQTCCDKPLLK cam cam |
| | | LQTCCDKPLLKK cam cam |
| | | LSQTFPNADFAEITK |
| | | LVQEVTDFAK |
| | | QEPERNECFLQHK cam |
| | | RHPDYSVSLLLR |
| | | RPCFSALTVDETYVPK cam |
| | | RPCFSALTVDETYVPKEFK cam |
| | | SLHTLFGDKLCAIPNLR cam |
| | | TNCDLYEKLGEYGFQNAILVR cam |
| | | TVMDDFAQFLDTCCK cam cam |
| | | YMCENQATISSK ox cam |
| | | YNDLGEQHFK |
| | | YNDLGEQHFKGLVLIAFSQYLQK |
| | | YTQKAPQVSTPTLVEAAR |
| gi\|42542817 | Alpha fetoprotein | ALQTMKQELLINLVK |
| | | AQDQEVCFTEEGPK cam |
| | | AQDQEVCFTEEGPKLISK cam |
| | | CSQSGNLPGCQDNLEEELQK cam cam |
| | | CSQSGNLPGCQDNLEEELQKHIEESQALSK cam |
| | | cam |
| | | DETYAPPPFSEDK |
| | | EGSMLNEHVCSVIR cam |
| | | ELREGSMLNEHVCSVIR ox cam |
| | | FIYEVSRR |
| | | LQNLFLIGYTR |
| | | NPFMYAPAILSLAAQYDK ox |
| | | NSGDGCLESQLSVFLDEICHETELSNK cam cam |
| | | QKPELTEEQLAAVTADFSGLLEK |
| | | QSCALYQTLGDYKLQNLFLIGYTR cam |
| | | TAPASVPPFQFPEPAESCK cam |
| | | TAPASVPPFQFPEPAESCKAHEENR cam |
| | | THPNLPVSVILR |
| | | VMTYICSQQNILSSK cam |
| | | YGLSGCCSQSGVER cam cam |
| gi\|191765 | Alpha fetoprotein | APQVSTPTLVEAAR |
| | | CCSGSLVER cam cam |
| | | CCTLPEDQRLPCVEDYLSAILNR cam cam cam |
| | | DVFLGTFLYEYSR |
| | | HPDYSVSLLLR |
| | | LGEYGFQNAILVR |
| | | LPCVEDYLSAILNR cam |
| | | LQTCCDKPLLK cam cam |
| | | LQTCCDKPLLKK cam cam |
| | | LSQTFPNADFAEITK |
| | | LSQTFPNADFAEITKLATDLTK |
| | | NYAEAKDVFLGTFLYEYSR |
| | | RHPDYSVSLLLR |
| | | TNCDLYEKLGEYGFQNAILVR cam |
| | | VCLLHEKTPVSEHVTK cam |
| | | YMCENQATISSK ox cam |
| | | YTQKAPQVSTPTLVEAAR |
| gi\|191844 | Alpha-1 protease | AVLTIDETGTEAAAATVFEAVPMSMPPILR |
| | inhibitor 2 | DQSPASHEIATNLGDFAISLYR |
| | | FDHPFLFIIFEEHTQSPIFVGK |
| | | FLEEAKNHYQAEVFSVNFAESEEAK |
| | | IFNNGADLSGITEENAPLK |
| | | IFNNGADLSGITEENAPLKLSK |
| | | IVEAVKELDQDTVFALANYILFK |
| | | KPFDPENTEEAEFHVDK |
| | | KVINDFVEK |
| | | LSISGDYNLK |
| | | LSISGDYNLKTLMSPLGITR |
| | | MQHLEQTLNK |
| | | MQHLEQTLNKELISK |
| | | NHYQAEVFSVNFAESEEAK |
| | | NHYQAEVFSVNFAESEEAKK |
| | | SFQHLLQTLNRPDSELQLSTGNGLFVNNDLK |
| | | TLMSPLGITR |
| | | VINDFVEKGTQGK |
| | | WKKPFDPENTEEAEFHVDK |
| gi\|31982171 | Alpha-2 | ADSHFRHGIPFFVK |
| | macroglobulin MUG1 | AHFSVMGDILSSAIR |
| | | ALMAYAFALAGNQNK |
| | | ALMAYAFALAGNQNKR ox |
| | | ALSCLESSWK cam |
| | | DGSYSAFGDQNGER |
| | | EGNTWLTAFVLK |
| | | ESGEKEEHSFTVMEFVLPR |
| | | ESVVFVQTDKPVYKPGQSVK |
| | | FALEIPVEFSMVPMAK ox ox |
| | | FSTSQSLPASQTR |
| | | HTNLVPHGTEKDVYR |
| | | HTSSWLVTPK |
| | | HVAYAVYSLSK |
| | | LPIICFDYGMVPISAPR cam ox |
| | | LQVTASPQSLCGLR cam |
| | | LSEDQEDCILYSSWLAEK cam |
| | | LSSSDEEDFLYVDIKGPTHEFSK |
| | | LVDIKGDPIPNEK |
| | | MLRPLNELLPLAYIEDPKK |
| | | MLSGFIPLKPTVK |
| | | MSLVLPPTVVK |
| | | MSLVLPPTVVKDSAR |
| | | NKESVVFVQTDKPVYKPGQSVK |
| | | NLFDELVLDKDLFQCVSFIIPR cam |
| | | QMSFSLAAEPIQGPYK ox |
| | | QQNSNGGFSSTQDTVVALDALSK |
| | | SLDEEAIKENNSIHWK |
| | | SQKTTLVTIQSTGSFSQK |
| | | TTLVTIQSTGSFSQK |
| | | YTYGKPVPGHVK |
| gi\|7304875 | Alpha-2-HS- | ANLMHNLGGEEVSVACK cam |
| | glycoprotein (Fetuin) | AQNVPLPVSTLVEFVIAATDCTAK cam |
| | | CNLLAEK |
| | | CNLLAEKQHGFCK cam cam |
| | | ELACDDPEAEQVALLAVDYLNNHLLQGFK cam |
| | | EVTDPAKCNLLAEK cam |
| | | HAFSPVASVESASGETLHSPK |
| | | QLTEHAVEGDCDFHILK cam |
| | | QLTEHAVEGDCDFHILKQDGQFR cam |
| | | QVLNQIDK |
| | | QVLNQIDKVK |
| | | TYHDLR |
| | | VGQPGAAGPVSPMCPGR cam |
| gi\|14318646 | Amylase 1, salivary | AHFSISNSAEDPFIAIHAESKI |
| | | AILDKLHNLNTK |
| | | ALVFVDNHDNQR |
| | | DFPGVPYSGFDFNDGK |
| | | GHGAGGASILTFWDAR |
| | | LSGLLDLALEKDYVR |
| | | NWGEGWGLMPSDR ox |
| | | SGNEDEFRDMVNR |
| | | TAIVHLFEWR |
| | | TASGGIENYQDAAQVR |
| | | VADYMNHLIDIGVAGFR ox |
| | | VMSSYYWPR |
| | | WVDIAKECER cam |
| gi\|425520 | Anti-colorectal | ADAAPTVSIFPPSSEQLTSGGASVVCFLNNFYPK |
| | carcinoma light chain | cam |
| | | ASQDINSYLSWFQQKPGK |
| | | HNSYTCEATHK cam |
| | | MTQSPSSMYASLGER ox |
| | | RADAAPTVSIFPPSSEQLTSGGASVVCFLNNFYPK |
| | | cam |
| | | TPAQFLGILLLWFPGMK ox |
| gi\|26345182 | Apolipoprotein A1 | ARPALEDLR |
| | (Unnamed protein | DFANVYVDAVKDSGR |
| | product) | DFWDNLEKETDWVR |
| | | HSLMPMLETLK ox |
| | | LAELKSNPTLNEYHTR |
| | | LSPVAEEFRDR |
| | | MRTHVDSLR |
| | | QKLQELQGR |
| | | QKVQPYLDEFQK |
| | | SNPTLNEYHTR |
| | | TQLAPHSEQMR |
| | | TQLAPHSEQMRESLAQR ox |
| | | TQVQSVIDKASETLTAQ |
| | | VAPLGAELQESARQK |
| | | VKDFANVYVDAVK |
| | | VQPYLDEFQK |
| | | VQPYLDEFQKK |
| | | WKEDVELYR |
| gi\|14789706 | Apolipoprotein A4 | ALVQQLEQFR |
| | | ALVQQLEQFRQQLGPNSGEVESHLSFLEK |
| | | ATIDQNLEDLRR |
| | | EAVEQFQKTDVTQQLSTLFQDK |
| | | EKVNSFMSTLEK |
| | | FLKAAVLTLALVAITGTR |
| | | GSPDQPQALPLPEQAQEQAQEQAQEQVQPKPLES |
| | | LGDASTYADGVHNK |
| | | LNHQMEGLAFQMK |
| | | LNHQMEGLAFQMKK |
| | | LQEHLKPYAVDLQDQINTQTQEMK |
| | | LQLTPYIQR |
| | | LVPFVVQLSGHLAQETER |
| | | MMPHANKVTQTFGENMQK ox |
| | | NLAPLVEDVQSK |
| | | NMEELKGHLTPR |
| | | QLEQQVEEFRR |
| | | QQLGPNSGEVESHLSFLEK |
| | | SLAPLTVGVQEK |
| | | SLAPLTVGVQEKLNHQMEGLAFQMK |
| | | TDVTQQLSTLFQDK |
| | | TDVTQQLSTLFQDKLGDASTYADGVHNK |
| | | TVEPMGEMFNK |
| | | VKGNTEGLQK |
| | | VNSFMSTLEK |
| gi\|6753096 | Apolipoprotein A-I | AQSVIDKASETLTAQ |
| | | ARPALEDLR |
| | | DFANVYVDAVK |
| | | DFWDNLEKETDWVR |
| | | HSLMPMLETLK ox ox |
| | | LAELKSNPTLNEYHTR |
| | | LQELQGR |
| | | LSPVAEEFR |
| | | LSPVAEEFRDR |
| | | MRTHVDSLR ox |
| | | QEMNKDLEEVK ox |
| | | QKLQELQGR |
| | | THVDSLR |
| | | TQLAPHSEQMR |
| | | TQLAPHSEQMRESLAQR ox |
| | | VAPLGAELQESARQK |
| | | VKDFANVYVDAVK |
| | | VQPYLDEFQK |
| | | VQPYLDEFQKK |
| | | WKEDVELYR |
| gi\|6753102 | Apolipoprotein E | AQAFGDRIR |
| | | EHMEEVRSK |
| | | ELEEQLGPVAEETR |
| | | ELEEQLGPVAEETRAR |
| | | ERLGPLVEQGR |
| | | GRLEEVGNQAR |
| | | GWFEPIVEDMHR ox |
| | | LGADMEDLRNR |
| | | LGKEVQAAQAR |
| | | LGPLVEQGRQR |
| | | LGQYRNEVHTMLGQSTEEIR |
| | | LKGWFEPIVEDMHR |
| | | LQAEIFQAR |
| | | MEEQTQQIR |
| | | NEVHTMLGQSTEEIRAR ox |
| | | QWANLMEK |
| | | SKMEEQTQQIR |
| | | TANLGAGAAQPLRDR |
| gi\|1938223 | Apolipoprotein H | ATFGCHETYKLDGPEEAECTK cam cam |
| | (beta-2 glycoprotein) I | ATVLYQGMR ox |
| | | CLPHFAMIGNDTVMCTEQGNWTR ox cam cam |
| | | CPFPPRPENGYVNYPAKPVLLYK cam |
| | | CPFPPRPENGYVNYPAKPVLLYKDK cam |
| | | CSYTVEAHCR cam cam |
| | | DGTIEIPSCFKEHSSLAFWK cam |
| | | FTCPLTGMWPINTLR cam |
| | | ITCPPPPVPK cam |
| | | KATVLYQGMR |
| | | KCSYTVEAHCR cam cam |
| | | RFTCPLTGMWPINTLR cam |
| | | TGTWSFLPTCR cam |
| | | TSYDPGEQIVYSCKPGYVSR cam |
| | | VCPFAGILENGIVR cam |
| | | WSPDIPACAR cam |
| gi\|9055162 | Apolipoprotein M | CVEEFQSLTSCLDFK cam cam |
| | | CVEEFQSLTSCLDFKAFLVTPR cam cam |
| | | FLLYNRSPHPPEK |
| | | LTEGKGNMELR |
| | | QLQLRATIR |
| | | SPHPPEKCVEEFQSLTSCLDFK cam cam |
| | | TDLFSSSCPGGIMLK cam ox |
| | | TDLFSSSCPGGIMLKETGQGYQR cam |
| | | TEGRPDMKTDLFSSSCPGGIMLK ox cam |
| gi\|2921308 | Carboxylesterase | DAGVSTYMYEFR |
| | precursor | FAPPQPAEPWSFVK |
| | | MNEETASLLLR |
| | | MNEETASLLLR ox |
| | | MNEETASLLLRR ox |
| | | MVMKFWANFAR ox ox |
| | | RFHSELNISESMIPAVIEK ox |
| | | SELILDMFGDIFFGIPAVLMSR ox ox |
| | | SFNTVPYIVGFNK |
| | | SLRDAGVSTYMYEFR |
| | | VLGKYISLEGFEQPVAVFLGVPFAKPPLGSLR |
| | | YISLEGFEQPVAVFLGVPFAKPPLGSLR |
| | | YRPSFVSDK |
| gi\|49523333 | Clusterin | ASGIIDTLFQDR |
| | (Apolipoprotein J) | ASGIIDTLFQDRFFAR |
| | | ELHDPHYFSPIGFPHK |
| | | ELHDPHYFSPIGFPHKRPHFLYPK |
| | | HTCMKFYAR cam ox |
| | | QQSQVLDAMQDSFAR ox |
| | | RPHFLYPK |
| | | SLLNSLEEAKK |
| | | TLIEKTNAER |
| | | YINKEIQNAVQGVK |
| gi\|6753798 | Coagulation factor II | DNLSPPLGQCLTER cam |
| | (prothrombin) | DTTEKELLDSYIDGR |
| | | EECSVPVCGQEGR cam cam |
| | | ENLDRDIALLK |
| | | ETFMDCLEGR cam |
| | | ETFMDCLEGR ox cam |
| | | GIAPWQVMLFR |
| | | GIAPWQVMLFRK ox |
| | | GKYGFYTHVFR |
| | | IRITDNMFCAGFK cam |
| | | ITDNMFCAGFK cam |
| | | KPVPFSDYIHPVCLPDKQTVTSLLR cam |
| | | LYQGNLAVTTLGSPCLPWNSLPAK cam |
| | | NPDSSTTGPWCYTTDPTVR cam |
| | | REECSVPVCGQEGR cam cam |
| | | RGDACEGDSGGPFVMK cam |
| | | SGGSKDNLSPPLGQCLTER cam |
| | | SPQELLCGASLISDR cam |
| | | TFGLGEADCGLRPLFEK cam |
| | | TLSKYQDFDPEVK |
| | | TTDAEFHTFFNEK |
| | | WYQMGIVSWGEGCDRK cam |
| | | YNWRENLDR |
| | | YTVCDSVRKPR cam |
| gi\|13624321 | Coagulation factor | CNEYYLLK cam |
| | XIII, beta subunit | CNEYYLLKGSETSR cam |
| | | CTAEGWSPNPR cam |
| | | DAYISETSIAGSVLR |
| | | DIVAYTCTAGYYTTTGK cam |
| | | GDVRYPMCIR cam |
| | | GMCASPPVIR cam |
| | | IAQYYYTFK |
| | | ILHGDLIDFVCK cam |
| | | LIENGYFHPVK |
| | | LSFFCLAGYATESGKQEEQIR cam |
| | | QCDFPTVENGR cam |
| | | QGYNLSPSIPLSEISAQCNR cam |
| | | QTGEAECQANGWSLTPQCNK cam cam |
| | | RDAYISETSIAGSVLR |
| | | SFYFPMSVDK |
| | | SFYFPMSVDKK ox |
| | | TTGGKDEEVVHCLSAGWSSQPSCR cam cam |
| | | VKDIVAYTCTAGYYTTTGK cam |
| gi\|6996919 | Complememnt | AEGIPEFYDYDVALVK |
| | histocompatibility 2, | ALLDIGRDPK |
| | complement | ALRLPQTATCK cam |
| | component factor B | CLTNLIEKVASYGVRPR cam |
| | | CPRPQDFENGEFWPR cam |
| | | DVKALFVSEQGK |
| | | FIQVGVISWGVVDVCR cam |
| | | GDSGGPLIVHKR |
| | | GGSFQLLQGGQALEYLCPSGFYPYPVQTR cam |
| | | GNDYHKQPWQAK |
| | | HVIIIMTDGLHNMGGNPVTVIQDIR ox |
| | | KDNEHHVFK |
| | | LEDIVTYHCSR cam |
| | | LKYGQTLRPICLPCTEGTTR cam cam |
| | | LNQISYEDHKLK |
| | | NPREDYLDVYVFGVGPLVDSVNINALASK |
| | | QHKEQLLPVK |
| | | QQLVPSYAR |
| | | RDLEIEEVLFHPK |
| | | RQQLVPSYAR |
| | | SLSLCGMVWEHK cam |
| | | SLSLCGMVWEHKK cam |
| | | SRFIQVGVISWGVVDVCR cam |
| | | STGSWSDLQTR |
| | | VKDASEVVTPR |
| | | VKDMEDLENVFYQMIDETK ox ox |
| | | VSDERSSDADWVTEK |
| | | WDGQTAICDDGAGYCPNPGIPIGTR cam cam |
| | | YGLLTYATVPK |
| | | YGQTLRPICLPCTEGTTR cam cam |
| gi\|28175786 | Complement C3 | AAVFNHFISDGVKK |
| | precursor (HSE-MSF) | AFYEHAPK |
| | | AVMVSFQSGYLFIQTDK ox |
| | | C47H50N3O7S2 (H+-Ion) |
| | | DNHLAPGQQTTLR |
| | | DNHLAPGQQTTLRIEGNQGAR |
| | | DSCIGTLVVKGDPR cam |
| | | EPGQDLVVLSLPITPEFIPSFR |
| | | EVVADSVWVDVKDSCIGTLVVK cam |
| | | FFKPAMPFDLMVFVTNPDGSPASK ox ox |
| | | GDPRDNHLAPGQQTTLR |
| | | IEGNQGAR |
| | | IFTVDNNLLPVGK |
| | | IRAFYEHAPK |
| | | IRYYTYLVMNK ox |
| | | ISLAHSLTR |
| | | KVLMEGVRPSNADALVGK |
| | | LESEETIVLEAHDAQGDIPVTVTVQDFLKR |
| | | LSINTPNSR |
| | | LVAYYTLIGASGQR |
| | | MELKPGDNLNVNFHLR ox |
| | | NVDGTAFVIFGVQDGDKK |
| | | NYAGVFMDAGLAFK |
| | | QIFSAEFEVK |
| | | QPLTITVR |
| | | QVLTSEKTVLTGASGHLR |
| | | SGIPIVTSPYQIHFTK |
| | | SLYVSVTVILHSGSDMVEAER ox |
| | | TIYTPGSTVLYR |
| | | TMEAHPYSTMHNSNNYLHLSVSR ox ox |
| | | TSQGLQTEQR |
| | | TVLTGASGHLR |
| | | TVVILIETPDGIPVKR |
| | | VEPTETFYYIDDPNGLEVSIIAK |
| | | VGLVAVDKGVFVLNK |
| | | VLMEGVRPSNADALVGK |
| | | VVIEDGVGDAVLTR |
| | | VVIEDGVGDAVLTRK |
| | | YVTVVANFGETVVEK |
| | | YYTYLVMNK |
| gi\|220349 | Complement C4 | APHIQLVAQSPWLR |
| | | ATETQGVNLLFSSR |
| | | ATETQGVNLLFSSRR |
| | | FALMDEQGKR |
| | | FVSSAFSLDLSR |
| | | GHIFVQTDQPIYNPGQR |
| | | GLETQAKLVEGR |
| | | GTGFLSIEPLDPR |
| | | HLVPGAHFLLQALVQEMSGSEASNVPVK ox |
| | | KYVLPNFEVK |
| | | LLLFSPSVVNLGTPLSVGVQLLDAPPGQEVK |
| | | LLVSAGSLYPAIAR |
| | | LSSGDDFVLLSLEVPLEDVR |
| | | LTVQAPPSRGTGFLSIEPLDPR |
| | | MRPSTDFLTITVENSHGLR ox |
| | | NTAFKATETQGVNLLFSSR |
| | | RAPHIQLVAQSPWLR |
| | | RGHIFVQTDQPIYNPGQR |
| | | SCGLFDLRR cam |
| | | TFLRGLETQAK |
| | | YIYGKPVQGVAYTR |
| | | YVLPNFEVK |
| gi\|309119 | Complement C4b- | ATYTHRDSVR |
| | binding protein | GKGVAWSNPFPECVIVK cam |
| | precursor | HSGTEDFYPYNHGISYTCDPGFR cam |
| | | IICSQPNILHGVIVSGYK cam |
| | | IICSQPNILHGVIVSGYKATYTHR cam |
| | | ITLVTYECDKGYR cam |
| | | MMVYCKPSGEWEISVSCAK ox cam cam |
| | | TMQYVPNSHDVK ox |
| | | TVPVWSSSPPTCEK cam |
| | | WKGTAPQCK cam |
| | | YECLPGYGR cam |
| gi\|15030019 | Complement | ALVHLPLEYNSAVYSR |
| | component 6 | ATDLQLSDVFLK |
| | | CLPDRTWSQGDVECQR cam cam |
| | | ECNNPAPQR cam |
| | | FDPCQCAPCPNNGRPR cam cam cam |
| | | FFPIPIFHFSEK |
| | | FLCDSGRCIPSK cam cam |
| | | FLYMEIHKEDTCTK cam |
| | | GEVLDNSFTGGICK cam |
| | | LSGTECLCVCQSGTYGENCER cam cam cam cam |
| | | NEDSLSVDER |
| | | NEHSHYSSAFNK |
| | | NEHSHYSSAFNKVIK |
| | | NIPCAVTKR cam |
| | | NKFLCDSGR cam |
| | | NKNEDSLSVDER |
| | | QELQNSGLTEEEAQNCVQYETK cam |
| | | QRQVVVNDYYWK |
| | | QVVVNDYYWK |
| | | SQQAAALAWEK |
| | | SVLRPSQFGGQPCTEPLVTFQPCVPSK cam cam |
| | | TKFFPIPIFHFSEK |
| | | TRECNNPAPQR cam |
| | | TSCLKPVVQDVLTISPFQR cam |
| | | TVVSQGDVECQR cam |
| | | VYQIGESIELTCPR cam |
| | | YGGSFLQGSEK |
| gi\|27462724 | Complement | CIPACGVPTEPFQVHQR cam cam |
| | component C1SA | IFGGQPAKIENFPWQVFFNHPR |
| | | ISDPLMYVGTMSVR ox ox |
| | | LQVVFTSDFSNEER |
| | | LRYHGDPISCAK cam |
| | | NQQFGPYCGNGFPGPLTIR cam |
| | | SPNSPIIEEFQFPYNK |
| | | WVNDQLGIELPR |
| | | YHGDPISCAK cam |
| gi\|19072788 | Complement | CDNGFSPPSGYSWDYLR cam |
| | component factor h | CIEKIPCSQPPTIEHGSINLPR cam cam |
| | | CLPVTELENGR cam |
| | | CSPPYILNGIYTPHR cam |
| | | CTAQGWEPEVPCVR cam cam |
| | | CTAQGWEPEVPCVRK cam cam |
| | | CTLKPCEFPQFK cam cam |
| | | CTLKPCEFPQFKYGR cam cam |
| | | CTPTGWIPVPR cam |
| | | CVATDQLEKCR cam cam |
| | | CVEILCTPPR cam cam |
| | | GDAVCTGSGWSSQPFCEEKR cam cam |
| | | HTEKIYSHSGEDIEFGCK cam |
| | | IIHRSDDEIR |
| | | IKTCSASDIHIDNGFLSESSSIYALNR cam |
| | | IPCSQPPTIEHGSINLPR cam |
| | | IVSGAAETDQEYYFGQVVR |
| | | KPCGHPGDTPFGSFR cam |
| | | KVEYSHGEWK |
| | | LYYEESLRPNFPVSIGNK |
| | | LYYEESLRPNFPVSIGNKYSYK |
| | | NGKWVASNPSR |
| | | SCDMPVFENSITKNTR cam ox |
| | | SDDEIRYECNYGFYPVTGSTVSK cam |
| | | SYRTGEQVTFR |
| | | TCGDIPELEHGSAK cam |
| | | TSCPPPPQIPNTQVIETTVK cam |
| | | VGDLLEFSCHSGHR cam |
| | | VGPDSVQCYHFGWSPGFPTCK cam cam |
| | | YRVGDLLEFSCHSGHR cam |
| gi\|6671744 | Complement | ECELPNSVPACVPWSPYLFQPNDR cam cam |
| | component factor i | ITCGGIYIGGCWILTAAHCVRPSR cam cam cam |
| | | SPSASDLPQEELVDQK |
| | | VANYFDWISYHVGR |
| | | VANYFDWISYHVGRSLVSQHNV |
| | | VIGGKPANVGDYPWQVAIKDGQR |
| | | VIVHEKYNGATFQNDIALIEMK |
| gi\|9954973 | Complement D Chain | DFDSVPPWR |
| | D, N-Terminally | DICEGQVNSLPGSINK cam |
| | Truncated C3dg | EADVSLTAFVLIALQEAR |
| | Fragment | FLNTAKDR |
| | | GYTQQLAFK |
| | | KGYTQQLAFK |
| | | QKPDGVFQEDGPVIHQEMIGGFR |
| | | RQEALELIK |
| | | WLILEK |
| | | WLNDER |
| gi\|6754132 | Complement | AKGNEQSFHVSLR |
| | histocompatibility 2, | APWMEQEGPEYWER |
| | Q region locus 10 | APWMEQEGPEYWERETQR |
| | | AYLEAECVEWLLR cam |
| | | FDSDAETPRMEPR |
| | | FIIVGYVDDTQFVR |
| | | FIIVGYVDDTQFVRFDSDAETPR |
| | | GNEQSFHVSLR |
| | | GYLQYAYDGR |
| | | GYLQYAYDGRDYIALNEDLK |
| | | KWEQAGAAEYYR |
| | | TDPPKTHVTHHPGSEGDVTLR |
| | | THVTHHPGSEGDVTLR |
| | | TWTAADVAAIITR |
| | | WASVVVPLGK |
| | | WEQAGAAEYYR |
| | | YFETSVSRPGLGEPR |
| | | YLELGKETLLR |
| gi\|54173 | Contraspin | ALYQTEAFTADFQQPTEAK |
| | | AVLDVAETGTEAAAATGVIGGIR |
| | | AVLDVAETGTEAAAATGVIGGI RK |
| | | AVLPAVCFNRPFLIVIYHTSAQSILFMAK cam ox |
| | | DLQILAEFHEK |
| | | DLQILAEFHEKTR |
| | | EVFTEQADLSGITEAK |
| | | EVFTEQADLSGITEAKK |
| | | FSIASDYRLEEDVLPEMGIK |
| | | GKTMEEILEGLK ox |
| | | HFRDEELSCSVLELK cam |
| | | ISFDPQDTFESEFYLDEKR |
| | | KLISELDDGTLMVLVNYIYFK ox |
| | | KLSVSQVVHK |
| | | KTLFSSQIEELNLPK |
| | | LISELDDGTLMVLVNYIYFK ox |
| | | MQQVEASLQPETLR |
| | | MQQVEASLQPETLRK |
| | | NIVFSPLSISAALALVSLGAK |
| | | NQDKNIVFSPLSISAALALVSLGAK |
| | | TLFSSQIEELNLPK |
| | | TRALYQTEAFTADFQQPTEAK |
| gi\|38614350 | Cp protein | AEDEHLGILGPPIHANVGDK |
| | | AEDEHLGILGPPIHANVGDKVK |
| | | AEVEDKVYVHLK |
| | | AGLQAFFQVR |
| | | ALYFEYTDGTFSK |
| | | DCNKPSPEDNIQDR cam |
| | | DIFTGLIGPMKICK cam |
| | | DLYSGLIGPLIVCR cam |
| | | DTANLFPHK |
| | | EMGPTYADPVCLSK ox cam |
| | | ETFTYEWTVPK |
| | | EYTDGSFTNR |
| | | EYTDGSFTNRK |
| | | GQHPLSIQPMGVSFTAENEGTYYGPPGR ox |
| | | IYHSHVDAPK |
| | | IYHSHVDAPKDIASGLIGPLILCK cam |
| | | IYTFHAHGVTYTK |
| | | KALYFEYTDGTFSK |
| | | KLISVDTEQSNFYLQNGPDR |
| | | LISVDTEQSNFYLQNGPDR |
| | | MAYREYTDGSFTNR ox |
| | | MFGNLQGLTMHVK ox ox |
| | | MFTTAPDQVDKEDEDFQESNK ox |
| | | MYYSGVDPTKDIFTGLIGPMK |
| | | NMATRPYSIHAHGVK |
| | | NMATRPYSIHAHGVK ox |
| | | QFEDFTVYLGER |
| | | QKYTVNQCQR cam |
| | | RAEDEHLGILGPPIHANVGDK |
| | | RDTANLFPHK |
| | | SGAGREDSACIPWAYYSTVDR cam |
| | | SLTLLMNPDTK ox |
| | | TESSTVVPTLPGEVR |
| | | TIDKPAWLGFLGPVIK |
| | | TYTWQIPER |
| | | TYYVAAVEVEWDYSPSR |
| | | VFFEQGATR |
| | | VKDLYSGLIGPLIVCR cam |
| | | VNKDNEEFLESNK |
| | | YTVNQCQR cam |
| gi\|19388017 | Cpn2 protein | AAHSQCAYSNPEGTVLLACEESR cam cam |
| | | ALGLDEGEPAGSWDLTVEGR |
| | | CRWLNIQLSSR cam |
| | | DLRTLNLAQNLLTQLPK |
| | | GAFQSLTGLQMLK |
| | | GQLVPNLKQEQLICPVNPGHLSFR cam |
| | | HLEPDAFGGLPR |
| | | LFQSLRDLR |
| | | LVSLTLSHNAITDLPEHVFR |
| | | LVSLTLSHNAITDLPEHVFRNLEQLVK |
| | | QEQLICPVNPGHLSFR cam |
| | | TLNLAQNLLTQLPK |
| | | TLPGRLFQSLR |
| | | VVFLNTQVR |
| | | WLNIQLSSR |
| gi\|11055360 | Epidermal growth | ACGPDYYEVEEDGIR cam |
| | factor receptor | ACGPDYYEVEEDGIRK cam |
| | isoform 2 | AVNHVCNPLCSSEGCWGPEPR cam cam cam |
| | | CNILEGEPR cam |
| | | FSNNPILCNMDTIQWR cam |
| | | GPDNCIQCAHYIDGPHCVK cam cam cam |
| | | GRSPSDCCHNQCAAGCTGPR cam cam cam cam |
| | | IICAQQCSHR cam cam |
| | | IPLENLQIIR |
| | | KLFGTPNQK |
| | | LTQLGTFEDHFLSLQR |
| | | MYNNCEVVLGNLEITYVQR ox cam |
| | | NLCYANTINWK cam |
| | | NLCYANTINWKK cam |
| | | NLQEILIGAVR |
| | | NYVVTDHGSCVR cam |
| | | SLKEISDGDVIISGNR |
| | | SPSDCCHNQCAAGCTGPR cam cam cam cam |
| | | TIQEVAGYVLIALNTVER |
| | | TPPLDPRELEILK |
| | | YCTAISGDLHILPVAFKGDSFTR cam |
| | | YSFGATCVKK cam |
| gi\|6679689 | Esterase 1 | AISESGVVINTNVGKK |
| | | FAPPQPAEPWSFVK |
| | | ILSDMFSTEKEILPLK ox |
| | | ISEDCLYLNIYSPADLTK cam |
| | | KSELILDMFGDIFFGIPAVLLSR |
| | | LKAEEVAFWTELLAK |
| | | MNEETASLLLR |
| | | MNEETASLLLRR |
| | | MVMKFWANFAR |
| | | NGNPNGEGLPHWPEYDEQEGYLQIGATTQQAQR |
| | | QKTESELLEISGK |
| | | RPQTVEGDHGDEIFFVFGAPLLK |
| | | SELILDMFGDIFFGIPAVLLSR ox |
| | | SFNTVPYIVGFNK |
| | | SLRDAGVSTYMYEFR |
| | | YISLEGFEQPVAVFLGVPFAKPPLGSLR |
| | | YRPSFVSDK |
| | | YRPSFVSDKRPQTVEGDHGDEIFFVFGAPLLK |
| gi\|33563252 | Fibrinogen, alpha | ALTEMRQMR ox ox |
| | polypeptide | AQQIQALQSNVR |
| | | AVNREINLQDYEGHQK |
| | | DSDWPFCSDDDWNHKCPSGCR cam cam cam |
| | | EINLQDYEGHQK |
| | | EINLQDYEGHQKQLQQVIAK |
| | | GDFANANNFDNTYGQVSEDLR |
| | | GDFANANNFDNTYGQVSEDLRR |
| | | GDFATRGPGSK |
| | | GDSRGDFATR |
| | | GLIDEANQDFTNR |
| | | GLIDEANQDFTNRINK |
| | | HPDLSGFFDNHFGLISPNFKEFGSK |
| | | LKNSLFDFQR |
| | | MADEAGSEAHREGETR |
| | | MKGLIDEANQDFTNR |
| | | MSPVPDLVPGSFK ox |
| | | MSPVPDLVPGSFKSQLQEAPPEWK ox |
| | | NNKDSNSLTR |
| | | QYLPALKMSPVPDLVPGSFK ox |
| | | RLEVDIDIK |
| | | SQLQEAPPEWK |
| | | SQLQEAPPEWKALTEMR |
| | | THSDSDILTNIEDPSSHVPEFSSSSK |
| | | VIEKAQQIQALQSNVR |
| | | VTSSGTSTTHR |
| gi\|33859809 | Fibrinogen, B beta | AHYGGFTVQNEASK |
| | polypeptide | AHYGGFTVQNEASKYQVSVNK |
| | | EDGGGWWYNR |
| | | EEPPSLRPAPPPISGGGYR |
| | | ENENVINEYSSILEDQR |
| | | GFGNIATNEDAK |
| | | GFGNIATNEDAKK |
| | | GGETSEMYLIQPDTSIKPYR |
| | | GSWYSMRR ox |
| | | HGTDDGWWMNWK |
| | | IQKLESDISAQMEYCR cam |
| | | IRPFFPQQ |
| | | KEEPPSLRPAPPPISGGGYR |
| | | KGGETSEMYLIQPDTSIKPYR |
| | | LYIDETVNDNIPLNLR |
| | | LYIDETVNDNIPLNLRVLR |
| | | MGPTELLIEMEDWKGDK |
| | | MSMKIRPFFPQQ ox |
| | | QAQVKENENVINEYSSILEDQR |
| | | QCSKEDGGGWWYNR cam |
| | | QDGSVDFGRK |
| | | SILEDLRSK |
| | | TENGGWTVIQNR |
| | | TPCTVSCNIPVVSGK cam cam |
| | | TPCTVSCNIPVVSGKECEEIIR cam cam cam |
| | | VYCDMKTENGGWTVIQNR cam |
| | | YCGLPGEYWLGNDK cam |
| | | YCGLPGEYWLGNDKISQLTR cam |
| | | YYWGGLYSWDMSK |
| gi\|19527078 | Fibrinogen, gamma | AIQVYYNPDQPPKPGMIDSATQK |
| | polypeptide | CHAGHLNGVYHQGGTYSK cam |
| | | DNCCILDER cam cam |
| | | EGFGHLSPTGTTEFWLGNEK |
| | | ESGLYFIRPLK |
| | | ESGLYFIRPLKAK |
| | | FGSFCPTTCGIADFLSSYQTDVDNDLR cam cam |
| | | GAKESGLYFIRPLK |
| | | IHLISMQSTIPYALR |
| | | IIPFNRLSIGEGQQHHMGGSK |
| | | IQLKDWNGR |
| | | LSIGEGQQHHMGGSK |
| | | LSIGEGQQHHMGGSKQAGDV |
| | | MVEEIVKYEALLLTHETSIR |
| | | QQFLVYCEIDGSGNGWTVLQK cam |
| | | QQFLVYCEIDGSGNGWTVLQKR cam |
| | | RIDGSLDFK |
| | | SSTTNGFDDGIIWATWK |
| | | SSTTNGFDDGIIWATWKSR |
| | | TSTADYAMFR |
| | | WYSMKETTMK |
| | | YLQEIYNSNNQK |
| gi\|1181242 | Fibronectin | ATGVFTTLQPLR |
| | | DGQERDAPIVNR |
| | | DTLTSRPAQGVITTLENVSPPRR |
| | | FLTTTPNSLLVSWQAPR |
| | | FTNIGPDTMR |
| | | GGQPKQYNVGPLASK |
| | | GVTYNIIVEALQNQR |
| | | GVTYNIIVEALQNQRR |
| | | NLQPGSEYTATLVAVK |
| | | HHAEHSVGRPR |
| | | IAGYRLTAGLTR |
| | | IHLYTLNDNAR |
| | | ITGYIIKYEKPGSPPR |
| | | ITYGETGGNSPVQEFTVPGSK |
| | | LGVRPSQGGEAPR |
| | | LTCQCLGFGSGHFR cam cam |
| | | NTFAEITGLSPGVTYLFK |
| | | QYNVGPLASKYPLR |
| | | SSPVIIDASTAIDAPSNLR |
| | | STTPDITGYR |
| | | TKTETITGFQVDAIPANGQTPVQR |
| | | VTDATETTITISWR |
| | | VTWAPPPSIELTNLLVR |
| | | WLPSTSPVTGYR |
| | | TVLVTWTPPR |
| gi\|46849812 | Fibronectin 1 | APITGYIIR |
| | | ATGVFTTLQPLR |
| | | CDPIDQCQDSETR cam cam |
| | | CHEGGQSYK cam |
| | | DGQERDAPIVNR |
| | | DQCIVDDITYNVNDTFHK cam |
| | | EYLGAICSCTCFGGQR cam cam cam |
| | | FLTTTPNSLLVSWQAPR |
| | | FTNIGPDTMR |
| | | GDSPASSKPVSINYK |
| | | GEWACIPYSQLR cam |
| | | GGNSNGALCHFPFLYNNR cam |
| | | GGQPKQYNVGPLASK |
| | | GLTPGVIYEGQLISIQQYGHR |
| | | GNLLQCVCTGNGR cam cam |
| | | GRISCTIANR cam |
| | | GVTYNIIVEALQNQR |
| | | GVTYNIIVEALQNQRR |
| | | HALQSASAGSGSFTDVR |
| | | HHAEHSVGRPR |
| | | HYQINQQWER |
| | | IAGYRLTAGLTR |
| | | IGDQWDKQHDLGHMMR ox |
| | | IGDTWSK |
| | | IGEKWDR |
| | | IHLYTLNDNAR |
| | | ISCTIANR cam |
| | | ITGYIIKYEKPGSPPR |
| | | ITYGETGGNSPVQEFTVPGSK |
| | | LGVRPSQGGEAPR |
| | | LTCQCLGFGSGHFR cam cam |
| | | MSCTCLGNGKGEFK cam cam |
| | | NLQPGSEYTVTLVAVKGNQQSPK |
| | | NRCNDQDTR cam |
| | | NTFAEITGLSPGVTYLFK |
| | | QDRVPPSR |
| | | QYNVGPLASKYPLR |
| | | RPGAAEPSPDGTTGHTYNQYTQR |
| | | SDNVPPPTDLQFVELTDVK |
| | | SSPVIIDASTAIDAPSNLR |
| | | STATINNIKPGADYTITLYAVTGR |
| | | STTPDITGYR |
| | | TEIDKPSQMQVTDVQDNSISVR ox |
| | | TFYQIGDSWEK |
| | | TFYSCTTEGR cam |
| | | TKTETITGFQVDAIPANGQTPVQR |
| | | VEVLPVSLPGEHGQR |
| | | VFAVHQGR |
| | | VTDATETTITISWR |
| | | VTWAPPPSIELTNLLVR |
| | | WCHDNGVNYK cam |
| | | WKCDPIDQCQDSETR cam cam |
| | | WKEATIPGHLNSYTIK |
| | | WLPSTSPVTGYR |
| | | WSRPQAPITGYR |
| | | YEKPGSPPR |
| | | YIVNVYQISEEGK |
| | | YSFCTDHAVLVQTR cam |
| | | YTGNTYK |
| gi\|28916693 | Gelsolin | AGKEPGLQIWR |
| | | AVQHREVQGFESSTFSGYFK |
| | | DGGQTAPASIR |
| | | DPDQTDGPGLGYLSSHIANVER |
| | | EPAHLMSLFGGKPMIIYK |
| | | EVQGFESSTFSGYFK |
| | | FDLVPVPPNLYGDFFTGDAYVILK |
| | | GGVASGFKHVVPNEVVVQR |
| | | HVVPNEVVVQR |
| | | IEGSNKVPVDPATYGQFYGGDSYIILYNYR |
| | | MDAHPPRLFACSNR ox cam |
| | | NWRDPDQTDGPGLGYLSSHIANVER |
| | | QTQVSVLPEGGETPLFKQFFK |
| | | SEDCFILDHGR cam |
| | | SEDCFILDHGRDGK cam |
| | | SGALNSNDAFVLK |
| | | SQHVQVEEGSEPDAFWEALGGK |
| | | TASDFISKMQYPR ox |
| | | TPSAAYLWVGAGASEAEK |
| | | TPSAAYLWVGAGASEAEKTGAQELLK |
| | | VEKFDLVPVPPNLYGDFFTGDAYVILK |
| | | VPFDAATLHTSTAMAAQHGMDDDGTGQK |
| | | VPVDPATYGQFYGGDSYIILYNYR |
| | | VSEARPSTMVVEHPEFLK |
| | | VSNGAGSMSVSLVADENPFAQGALR |
| | | VVQGKEPAHLMSLFGGKPMIIYK ox |
| | | YIETDPANR |
| | | YIETDPANRDR |
| gi\|52843238 | Glutathione | FLVGPDGIPVMR ox |
| | peroxidase 3 | LFWEPMKIHDIR |
| | | MDILSYMRR ox |
| | | NSCPPTAELLGSPGR cam |
| | | QEPGENSEILPSLK |
| | | TTVSNVKMDILSYMR ox |
| | | WNFEKFLVGPDGIPVMR ox |
| | | YVRPGGGFVPNFQLFEK |
| | | YVRPGGGFVPNFQLFEKGDVNGEK |
| gi\|17512357 | Gpld1 protein | AQYVLTSPEASSR |
| | | GIVATFYSHPR |
| | | GKYHDVSER |
| | | GRNQVVVAAGR |
| | | IYDNLYGRK |
| | | LGTSLSSGYVR |
| | | LSGALHVYSFSSD |
| | | NDFHRNLTMFISR ox |
| | | NLRLMLAGSSQK ox |
| | | NLTMFISRDIR |
| | | SLGKVYGYFLPNR |
| | | SSWGARLSGALHVYSFSSD |
| | | TQPALLSTFSGDRR |
| | | VYGYFLPNR |
| | | WYVPVRDLLR |
| | | YHDVSERTHWTPFLNASIHYIR |
| gi\|1694789 | GRS protein | LAQDYLQCVLQIPQPGSGPSK cam |
| | | QNGGWENGFVKK |
| | | SGWMTFLEVTGKICEMLSLLK cam |
| | | TLFTQVMEK |
| | | VLQNVAFSVQKEVEK |
| gi\|37719755 | GUGU alpha | AMFHINKPR |
| | | AMFHINKPRR |
| | | AMNQWVSGPAYYVEYLIK |
| | | DGYMLSLNR ox |
| | | DQKDGYMLSLNR |
| | | EHYQEDMGSLFYLTLDVLETDCHVLSR ox cam |
| | | GSIQHLPELDDEKPEESK |
| | | HVPLIQPVEK |
| | | ISAFDRFGR |
| | | KTHTTCPDCPSPIDLSNPSALEAATESLAK cam cam |
| | | LVVLPFPGKEQR |
| | | MFYESVYGQCK cam |
| | | MISAFDRFGR |
| | | SAECPGPEKENNPLVLPP cam |
| | | SQASCSLQHSDSEPVGICQGSTVQSSLR cam cam |
| | | THTTCPDCPSPIDLSNPSALEAATESLAK cam cam |
| gi\|23956086 | Hemopexin | DYFVSCPGR cam |
| | | ELGSPPGISLETIDAAFSCPGSSR cam |
| | | FNPVTGEVPPR |
| | | FNPVTGEVPPRYPLDAR |
| | | GATYAFTGSHYWR |
| | | GECQSEGVLFFQGNR cam |
| | | GECQSEGVLFFQGNRK cam |
| | | GGNNLVSGYPK |
| | | GPDSVFLIK |
| | | LFQEEFPGIPYPPDAAVECHR cam |
| | | LWWLDLK |
| | | LYVSSGRR |
| | | RLWWLDLK |
| | | SGAQATWTEVSWPHEK |
| | | SGAQATWTEVSWPHEKVDGALCLDK cam |
| | | SLGPNTCSSNGSSLYFIHGPNLYCYSSIDKLNAAK |
| | | cam cam |
| | | SLPQPQKVNSILGCSQ cam |
| | | VWVYPPEKK |
| | | WFWDFATR |
| | | WKNPITSVDAAFR |
| | | WLERYYCFQGNK cam |
| | | YYCFQGNK cam |
| | | YYCFQGNKFLR cam |
| gi\|150082914 | High molecular | ATSQVVAGTKYVIEFIAR |
| | weight kininogen I | AYFPCIGCVHAISTDSPDLEPVLK cam cam |
| | isoform DeltaD5 | CQALDMTEMAR ox cam |
| | | ENEFFIVTQTCK cam |
| | | FPSLHGDCVALPNGDDGECR cam cam |
| | | LISDFPEATSPK |
| | | RENEFFIVTQTCK cam |
| | | RPPGFSPFR |
| | | SGNQYMLHR |
| | | TDGSPTFYSFK |
| | | VIEGTKTDGSPTFYSFK |
| gi\|40715898 | HMW-kininogen-II | AKMDGSATFYSFNYQIK ox |
| | variant | ATSQVVAGTNYVIEFIAR |
| | | CQALDKTIPIR cam |
| | | EGNCSAQRGLAWQDCDFK cam cam |
| | | ENKFFIVTQTCK cam |
| | | FFIVTQTCK cam |
| | | GDCVALPNGDGGECR cam cam |
| | | KATSQVVAGTNYVIEFIAR |
| | | MDGSATFYSFNYQIK ox |
| | | QLPRNVLGAHGSQLAGR |
| | | RPPGFSPFR |
| | | RRPPGFSPFR |
| gi\|31615671 | Ig (A Chain A, Crystal | ASQSISNNLHWYQQK |
| | Structure Of Fab | DIVLTQSPATLSVTPGDSVSLSCR cam |
| | Fragment Of | DSTYSMSSTLTLTKDEYER |
| | Antibody Hyhel-26 | HNSYTCEATHK cam |
| | Complexed With | QNGVLNSWTDQDSK |
| | Lysozyme) | YASQSISGIPSR |
| gi\|4930001 | Ig (A Chain A, | IAVAWYQQKPGQSPK |
| | Idiotope-Anti-Idiotope | DSTYSMSSTLTLTKDEYER ox |
| | Fab-Fab Complex) | FMSTSVGDRVSITCK cam |
| | | HNSYTCEATHK cam |
| | | LLIYWASTR |
| | | QNGVLDSWTDQDSK |
| | | VSITCKASQDVR cam |
| gi\|11514687 | Ig (A Chain A, Lyme | ASQDINKYIAWYQHKPGK |
| | Disease Antigen | DSTYSMSSTLTLTKDEYER ox |
| | Ospa In Complex | HNSYTCEATHK cam |
| | With Neutralizing | HNSYTCEATHKTSTSPIVK cam |
| | Antibody Fab La-2) | QNGVLNSWTDQDSK |
| | | RADAAPTVSIFPPSSEQLTSGGASVVCFLNNFYPK |
| | | cam |
| | | YIAWYQHKPGK |
| gi\|42543442 | Ig (A Chain A, S25-2- | DIVMSQSPSSLAVSAGEK ox |
| | Kdo Monosaccharide | DSTYSMSSTLTLTKDEYER ox |
| | Complex) | HNSYTCEATHK cam |
| | | LLIYWASTR |
| | | NYLAWYQQKPGQSPK |
| | | QNGVLNSWTDQDSK |
| | | TSTSPIVKSFNR |
| gi\|7766934 | Ig (A Chain A, | ADAAPTVSIFPPSSEQLTSGGASVVCFLNNFYPK |
| | Structure Of An | cam |
| | Activity Suppressing | DIVLTQSPASLAVSLGQR |
| | Fab Fragment To | DIVLTQSPASLAVSLGQRATISCR cam |
| | Cytochrome P450 | HNSYTCEATHK cam |
| | Aromatase) | HNSYTCEATHKTSTSPIVK cam |
| | | LLIYLVSNLESGVPAR |
| gi\|27373551 | Ig (antibody variable | DFQPGYNTGLK |
| | domain) | FTISKTSTTVDLK |
| | | GLEYIGFINPRGTPYYASWAK |
| | | GTPYYASWAK |
| | | IWGPGTLVTVSS |
| gi\|1870378 | Ig (Anti-DNA | ASQSISDYLHWYQQK |
| | immunoglobulin light | ASQSISDYLHWYQQKSHESPR |
| | chain IgG) | DIVMTQSPATLSVTPGDR ox |
| | | DIVMTQSPATLSVTPGDRVSLSCR ox cam |
| | | LLIKYASQSISGIPSR |
| | | YASQSISGIPSR |
| gi\|12002896 | Ig (anti-human | ADAAPTVSIFPPSSEQLTSGGASVVCFLNNFYPK |
| | apolipoprotein A | cam |
| | monoclonal antibody | HNSYTCEATHK cam |
| | mAb(a)23L kappa | HNSYTCEATHKTSTSPIVK cam |
| | light chain) | LLIHYTSTLQPGIPSR |
| | | RADAAPTVSIFPPSSEQLTSGGASVVCFLNNFYPK |
| | | cam |
| | | TFGGGTKLEIK |
| | | YIAWYQHKPGKGPR |
| gi\|349893 | Ig (C Chain C, Fab | DSTYSMSSTLTLTKDEYER |
| | (Igg2a,Kappa) | DVVMTQTPLSLPVSLGDQASISCR cam |
| | Fragment (26-10) | FSGSGSGTDFTLK |
| | Complex With | FSGVPDRFSGSGSGTDFTLK |
| | Digoxin) | HNSYTCEATHK cam |
| | | HNSYTCEATHKTSTSPIVK cam |
| | | QNGVLNSWTDQDSK |
| | | TSTSPIVKSFNR |
| | | VSNRFSGVPDR |
| gi\|47059057 | Ig (gamma-2b- | APQVYILPPPAEQLSR |
| | immunoglobulin) | CPAPNLEGGPSVFIFPPNIKDVLMISLTPK cam ox |
| | | LEPSGPISTINPCPPCKECHK cam cam cam |
| | | SEDTAMYYCAR cam |
| | | TTPPSVYPLAPGCGDTTGSSVTLGCLVK cam cam |
| | | VNNKDLPSPIER |
| | | VVSALPIQHQDWMSGKEFK ox |
| gi\|1806128 | Ig (immunoglobulin | ALPSPIEKTISKPR |
| | constant heavy chain) | APQVYVLPPPAEEMTKK |
| | | ECPPCAAPDLLGGPSVFIFPPK cam cam |
| | | GPVRAPQVYVLPPPAEEMTK |
| | | GSLFACSVVHEGLHNHLTTK cam |
| | | NTATVLDSDGSYFMYSK |
| | | TEQNYKNTATVLDSDGSYFMYSK ox |
| | | TTAPSVYPLAPVCGGTTGSSVTLGCLVK cam cam |
| | | VPITQNPCPPLK cam |
| | | VPITQNPCPPLKECPPCAAPDLLGGPSVFIFPPK cam |
| | | cam cam |
| | | WSALPIQHQDWMSGK |
| | | VVSALPIQHQDWMSGKEFK |
| gi\|10121892 | Ig (immunoglobulin | ALIYSASYR |
| | IgM MP-18-3-117 | ASQNVGTNVAWYQQKPGQSPK |
| | kappa light chain) | FMSTSVGDRVSVTCK cam |
| | | FMSTSVGDRVSVTCK ox cam |
| | | HNSYTCEATHK cam |
| | | HNSYTCEATHKTSTSPIVK cam |
| | | RADAAPTVSIFPPSSEQLTSGGASVVCFLNNFYPK |
| | | cam |
| gi\|196723 | Ig (immunoglobulin | DVQITQSPSYLAASPGETITINCR cam |
| | kappa-chain VK-1) | DVQITQSPSYLAASPGETITINCRASK cam |
| | | LLIYSGSTLQSGIPSR |
| | | SISKYLAWYQEKPGK |
| | | TNKLLIYSGSTLQSGIPSR |
| | | YLAWYQEKPGK |
| | | YLAWYQEKPGKTNK |
| gi\|18033701 | Ig (immunoglobulin | ANGTPITQGVDTSNPTK |
| | light chain constant | EGNKFMASSFLHLTSDQWR |
| | region) | FMASSFLHLTSDQWR |
| | | SHNSFTCQVTHEGDTVEK cam |
| | | STPTLTVFPPSSEELKENK |
| gi\|13399686 | Ig (L Chain L, 64m-2 | ADAAPTVSIFPPSSEQLTSGGASVVCFLNNFYPK |
| | Antibody Fab | cam |
| | Complexed With | DSTYSMSSTLTLTKDEYER |
| | D(5ht)(6-4)t) | DVLMTQTPLSLPVSLGDQASISCR cam |
| | | FSGSGSGTDFTLK |
| | | FSGVPDRFSGSGSGTDFTLK |
| | | HNSYTCEATHK cam |
| | | QNGVLNSWTDQDSK |
| | | RADAAPTVSIFPPSSEQLTSGGASVVCFLNNFYPK |
| | | cam |
| | | SSQSIVHSNGNTYLEWYLQKPGQSPK |
| | | VSNRFSGVPDR |
| gi\|3212470 | Ig (L Chain L, Anti | ADAAPTVSIFPPSSEQLTSGGASVVCFLNNFYPK |
| | Taq Fab Tp7) | cam |
| | | DEYERHNSYTCEATHK cam |
| | | DIQMTQSPAIMSASPGEK ox ox |
| | | DSTYSMSSTLTLTKDEYER |
| | | FSGSGSGTSYSLTISR |
| | | HNSYTCEATHK cam |
| | | HNSYTCEATHKTSTSPIVK cam |
| | | LLIYDSTNLASGVPVR |
| | | RADAAPTVSIFPPSSEQLTSGGASVVCFLNNFYPK |
| | | cam |
| gi\|5542523 | Ig (L Chain L, | ADAAPTVSIFPPSSEQLTSGGASVVCFLNNFYPK |
| | Bactericidal Antibody | cam |
| | Against Neisseria | DIVMTQTPLSLPVSLGDKASISCR cam |
| | Meningitidis) | DIVMTQTPLSLPVSLGDKASISCR ox cam |
| | | DSTYSMSSTLTLTKDEYER ox |
| | | FSGSGSGTDFTLK |
| | | FSGVPDRFSGSGSGTDFTLK |
| | | HNSYTCEATHK cam |
| | | HNSYTCEATHKTSTSPIVK cam |
| | | QNGVLNSWTDQDSK |
| | | TFGGGTKLEIK |
| | | VSNRFSGVPDR |
| gi\|16975338 | Ig (L Chain L, Crystal | ADAAPTVSIFPPSSEQLTSGGASVVCFLNNFYPK |
| | Structure Of | cam |
| | Immunoglobulin Fab | ASGNIHNYLAWYQQK |
| | Fragment Complexed | DEYERHNSYTCEATHK cam |
| | With 17-Beta- | DIQMTQSPASLSASVGETVTITCR ox cam |
| | Estradiol) | DSTYSMSSTLTLTKDEYER |
| | | DSTYSMSSTLTLTKDEYER ox |
| | | FSGSGSGTQYSLK |
| | | HNSYTCEATHK cam |
| | | HNSYTCEATHKTSTSPIVK cam |
| | | QNGVLNSWTDQDSK |
| | | SPQLLVYNAK |
| gi\|18655521 | Ig (L Chain L, Crystal | ADAAPTVSIFPPSSEQLTSGGASVVCFLNNFYPK |
| | Structure Of The Fab | cam |
| | Fragment Of The | ASQSVDYDGDSYMNWYQQKPGQPPK |
| | Mouse Anti- Human | ASQSVDYDGDSYMNWYQQKPGQPPK ox |
| | Fas Antibody Hfe7a) | DIVLTQSPASLAVSLGQR |
| | | DSTYSMSSTLTLTKDEYER |
| | | DSTYSMSSTLTLTKDEYER ox |
| | | HNSYTCEATHK cam |
| | | LLIYAASNLESGIPAR |
| gi\|1942810 | Ig (L Chain L, Fab | ADAAPTVSIFPPSSEQLTSGGASVVCFLNNFYPK |
| | Fragment Of A | cam |
| | Neutralizing Antibody | DVVMTQTPLTLSVTIGQPASISCK cam |
| | Directed Against An | DVVMTQTPLTLSVTIGQPASISCK ox cam |
| | Epitope Of Gp41 | FTGSGSGTDFTLK |
| | From Hiv-1) | HNSYTCEATHK cam |
| | | HNSYTCEATHKTSTSPIVK cam |
| | | LDSGVPDRFTGSGSGTDFTLK |
| | | LIYLVSKLDSGVPDR |
| | | QNGVLNSWTDQDSK |
| | | SSQSLLDSDGKTYLNWLLQRPGQSPK |
| | | TFGGGTKLEIK |
| | | VEAEDLGVYYCWQGTHFPR cam |
| gi\|7546516 | Ig (L Chain L, Fab | ADAAPTVSIFPPSSEQLTSGGASVVCFLNNFYPK |
| | Fragment Of | cam |
| | Neutralising | DIVLTQSPASLAVSLGQR |
| | Monoclonal Antibody | HNSYTCEATHK cam |
| | 4c4 Complexed With | LLIYRASNLESGIPAR |
| | G-H Loop From | RADAAPTVSIFPPSSEQLTSGGASVVCFLNNFYPK |
| | Fmdv) | cam |
| gi\|17943084 | Ig (L Chain L, | DSTYSMSSTLTLTKDEYER |
| | Structural Basis For | DSTYSMSSTLTLTKDEYER ox |
| | Disfavored | EVVMTQSPLSLPVSLGDQASISCR cam |
| | Elimination Reaction | EVVMTQSPLSLPVSLGDQASISCR ox cam |
| | In Catalytic Antibody | FSGSGSGTDFTLK |
| | 1d4) | FSGVPDRFSGSGSGTDFTLK |
| | | HNSYTCEATHK cam |
| | | HNSYTCEATHKTSTSPIVK cam |
| | | QNGVLNSWTDQDSK |
| | | SSQSLVHSNGNTYLHWYLQKPGQSPK |
| | | TSTSPIVKSFNR |
| | | VSNRFSGVPDR |
| gi\|2624743 | Ig (L Chain L, | DEYERHNSYTCEATHK cam |
| | Structure Of A | DIVMTQSPLTLSVTIGQPASISCKSSQSLLYSNGK ox |
| | Catalytic Antibody, | cam |
| | Igg2a Fab Fragment) | DSTYSMSSTLTLTKDEYER |
| | | DSTYSMSSTLTLTKDEYER ox |
| | | FTGSGSGTDFTLK |
| | | HNSYTCEATHK cam |
| | | LDSGVPDRFTGSGSGTDFTLK |
| | | QNGVLNSWTDQDSK |
| gi\|32263981 | Ig (mAb | FSGSGSGTSYSLTISR |
| | immunoglobulin light | FSGSGSGTSYSLTISRMEAEDAATYYCQQR ox cam |
| | chain variable region) | MEAEDAATYYCQQR cam |
| | | MEAEDAATYYCQQR ox cam |
| | | QIVLTQSPAIMSASPGEK ox |
| | | SSYPFTFGSGTK |
| | | SSYPFTFGSGTKLEIK |
| gi\|18568342 | Ig (monoclonal | CDIQMTQTTSALSASLGDR cam |
| | antibody BBK-2 light | CDIQMTQTTSALSASLGDR cam ox |
| | chain) | DSTYSMSSTLTLTKDEYER ox |
| | | HNSYTCEATHK cam |
| | | HNSYTCEATHKTSTSPIVK cam |
| | | MRFSAQFLGLLLLCFQGTR ox cam |
| | | NLEQEDVATYFCQQGYTLPYTFGGGTKLEIK cam |
| | | QNGVLNSWTDQDSK |
| gi\|52382 | Ig (mu(b) | DGKLVESGFTTDPVTIENK |
| | immunoglobulin | DLHVPIPAVAEMNPNVNVFVPPR ox |
| | heavy chain) | EFVCTVTHR cam |
| | | EQLNLRESATVTCLVK cam |
| | | FISKPNEVHK |
| | | GFSPADISVQWLQR |
| | | GVASVCVEDWNNR cam |
| | | GVASVCVEDWNNRK cam |
| | | HPPAVYLLPPAR |
| | | KEFVCTVTHR cam |
| | | LICEATNFTPKPITVSWLK cam |
| | | LVESGFTTDPVTIENK |
| | | LVESGFTTDPVTIENKGSTPQTYK |
| | | NKDLHVPIPAVAEMNPNVNVFVPPR |
| | | NKDLHVPIPAVAEMNPNVNVFVPPR ox |
| | | NLVAMGCLAR cam |
| | | NLVAMGCLAR ox cam |
| | | SILEGSDEYLVCK cam |
| | | TGGKYLATSQVLLSPK |
| | | YLATSQVLLSPK |
| gi\|7439167 | Ig (PC4436 | APQVYTIPPPKEQMAK |
| | monoclonal antibody | APQVYTIPPPKEQMAK ox |
| | 13-1 heavy chain) | CRVNSAAFPAPIEK cam |
| | | DCGCKPCICTVPEVSSVFIFPPKPK cam cam cam |
| | | cam |
| | | DDSKSSVYLQMNR |
| | | GRPKAPQVYTIPPPK |
| | | SNWEAGNTFTCSVLHEGLHNHHTEK cam |
| | | TTPPSVYPLAPGSAAQTNSMVTLGCLVK ox cam |
| | | VNSAAFPAPIEK |
| | | VNSAAFPAPIEKTISK |
| gi\|15824610 | Ig (Pterin-mimicking | DIVLTQSPASLAVSLGQR |
| | anti-idiotope kappa | FSGSGSGTDFTLNIHPVEEEDAATYYCQHSR cam |
| | chain variable region) | LLIYLASNLESGVPAR |
| | | ELPYTFGGGTKLEIK |
| | | DIVLTQSPASLAVSLGQRATISCR cam |
| gi\|780265 | Ig gamma-1 chain C | APQVYTIPPPKEQMAK ox |
| | region (15C5) | CRVNSAAFPAPIEK cam |
| | (fragment) | NTQPIMDTDGSYFVYSKLNVQK ox |
| | | SNWEAGNTFTCSVLHEGLHNHHTEK cam |
| | | SVSELPIMHQDWLNGK |
| | | SVSELPIMHQDWLNGK ox |
| | | VNSAAFPAPIEK |
| | | VNSAAFPAPIEKTISK |
| gi\|1799551 | Ig gamma-chain, | AQTPQVYTIPPPR |
| | secrete-type | AQTPQVYTIPPPREQMSK |
| | | AQTPQVYTIPPPREQMSK ox |
| | | DALMISLTPK ox |
| | | EVHTAWTQPR |
| | | GYFPEPVTVK |
| | | GYFPEPVTVKWNYGALSSGVR |
| | | NTPPILDSDGTYFLYSK |
| | | VNNKALPAPIER |
| | | WSALPIQHQDWMR |
| | | WSALPIQHQDWMR ox |
| | | WNYGALSSGVR |
| gi\|223428 | Ig H-C allotype | APQVYILPPPAEQLSR |
| | gamma2b | APQVYILPPPAEQLSRK |
| | | CPAPNLEGGPSVFIFPPNIK cam |
| | | CPAPNLEGGPSVFIFPPNIKDVLMISLTPK cam |
| | | CPAPNLEGGPSVFIFPPNIKDVLMISLTPK cam ox |
| | | GLVRAPQVYILPPPAEQLSR |
| | | HEGLKNYYLK |
| | | ISWFVNNVEVHTAQTQTHR |
| | | LEPSGPISTINPCPPCKECHK cam cam cam |
| | | TDSFSCNVR cam |
| | | TDSFSCNVRHEGLK cam |
| | | TTPPSVYPLAPGCGDTTGSSVTLGCLVK cam cam |
| | | VNNKDLPSPIER |
| | | VTCVVVDVSEDDPDVR cam |
| | | WSALPIQHQDWMSGK |
| | | WSALPIQHQDWMSGK ox |
| | | WSALPIQHQDWMSGKEFK |
| | | WSALPIQHQDWMSGKEFK ox |
| | | WEKTDSFSCNVR cam |
| gi\|224008 | Ig J chain | CYTTMVPLR cam |
| | | CYTTMVPLR cam ox |
| | | CYTTMVPLRYHGETK cam |
| | | CYTTMVPLRYHGETK cam ox |
| | | IIPSTEDPNEDIVER |
| | | IIPSTEDPNEDIVERNIR |
| | | MVQAALTPDSCYPD ox cam |
| | | NFVYHLSDVCK cam |
| | | NFVYHLSDVCKK cam |
| gi\|12832551 | Ig K (unnamed | ADAAPTVSIFPPSSEQLTSGGASVVCFLNNFYPK |
| | protein product) | cam |
| | | ASENIYSNLAWYQQK |
| | | ASENIYSNLAWYQQKQGK |
| | | HNSYTCEATHK cam |
| | | HNSYTCEATHKTSTSPIVK cam |
| | | QGKSPQLLVYAATNLADGVPSR |
| | | RADAAPTVSIFPPSSEQLTSGGASVVCFLNNFYPK |
| | | cam |
| | | SPQLLVYAATNLADGVPSR |
| gi\|896077 | Ig kappa chain | ASSSVSSSYLHWYQQK |
| | | ENVLTQSPAIMSASPGEK ox |
| | | ENVLTQSPAIMSASPGEKVTMTCR ox cam |
| | | ENVLTQSPAIMSASPGEKVTMTCR ox ox cam |
| | | LWIYSTSNLASGVPAR |
| | | SGASPKLWIYSTSNLASGVPAR |
| gi\|896089 | Ig kappa chain | DIVMSQSPSSLAVSVGEK ox |
| | | ESGVPDRFTGSGSGTDFTLTISSVK |
| | | FTGSGSGTDFTLTISSVK |
| | | LLIYWASTR |
| | | NYLAWYQQKPGQSPK |
| | | SSQSLLYSSNQK |
| gi\|896103 | Ig kappa chain | ASQDIGSSLNWLQQEPDGTIKR |
| | | DIQMTQSPSSLSASLGER |
| | | DIQMTQSPSSLSASLGER ox |
| | | LIYATSSLDSGVPK |
| | | LIYATSSLDSGVPKR |
| gi\|896107 | Ig kappa chain | ADAAPTVSIFPPSSEQLTSGGASVVCFLNNFYPK |
| | | cam |
| | | ASENIYSNLAWYQQK |
| | | ASENIYSNLAWYQQKQGK |
| | | HNSYTCEATHK cam |
| | | HNSYTCEATHKTSTSPIVK cam |
| | | QGKSPQLLVYAATNLADGVPSR |
| | | RADAAPTVSIFPPSSEQLTSGGASVVCFLNNFYPK |
| | | cam |
| | | SPQLLVYAATNLADGVPSR |
| gi\|7513693 | Ig kappa chain | ADAAPTVSIFPPSSEQLTSGGASVVCFLNNFYPK |
| | (Mab03-1) | cam |
| | | DEYERHNSYTCEATHK cam |
| | | DIVMTQSPSSLAMSVGQK |
| | | DIVMTQSPSSLAMSVGQK ox |
| | | DIVMTQSPSSLAMSVGQK ox ox |
| | | DSTYSMSSTLTLTKDEYER ox |
| | | HNSYTCEATHK cam |
| | | HNSYTCEATHKTSTSPIVK cam |
| | | LLVYFASTR |
| | | NYLAWYQQKPGQSPK |
| | | SSQSLLNSRNQK |
| gi\|110429 | Ig kappa chain V | ASQDINKYIAWYQHKPGK |
| | region (9.42 | DIQMTQSPSSLSASLGGK |
| | | DIQMTQSPSSLSASLGGK ox |
| | | LLIHYTSTLQPGIPSR |
| | | YIAWYQHKPGK |
| | | YIAWYQHKPGKGPR |
| gi\|49258884 | Ig kappa chain V | ADAAPTVSIFPPSSEQLTSGGASVVCFLNNFYPK |
| | region (D444) | cam |
| | | ASQSIGTDIHWYQQR |
| | | DILLTQSPAILSVSPGER |
| | | HNSYTCEATHK cam |
| | | LLIKYASESISGIPSR |
| | | RADAAPTVSIFPPSSEQLTSGGASVVCFLNNFYPK |
| | | cam |
| | | YASESISGIPSR |
| gi\|930226 | Ig kappa chain V | ASQDIKSYLSWYQQKPWK |
| | region (hybridoma | DIKMTQSPSSMYASLGER ox |
| | NC19-E11) | DIKMTQSPSSMYASLGER ox ox |
| | | MTQSPSSMYASLGER ox |
| | | MTQSPSSMYASLGER ox ox |
| | | SYLSWYQQKPWK |
| | | SYLSWYQQKPWKSPK |
| | | TLIYYATSLADGVPSR |
| gi\|125796 | Ig kappa chain V-III | ASESVDNYGISFMNWFQQKPGQPPK |
| | region PC 2880/PC | ASESVDNYGISFMNWFQQKPGQPPK ox |
| | 1229 | DIVLTQSPASLAVSLGQR |
| | | EVPWTFGGGTK |
| | | EVPWTFGGGTKLEIK |
| | | LLIYAASNQGSGVPAR |
| gi\|197064 | Ig kappa chain V- | ASQDISNYLNWYQQKPDGTVK |
| | region (V-Jk1) protein | DIQMTQTTSSLSASLGDR ox |
| | | DIQMTQTTSSLSASLGDRVTISCR cam |
| | | DIQMTQTTSSLSASLGDRVTISCR ox cam |
| | | FSGSGSGTDYSLTISNLEQEDIATYFCQQGNTLPR |
| | | cam |
| | | LLIYYTSRLHSGVPSR |
| | | TFGGGTKLEIK |
| gi\|1613779 | Ig kappa light chain | ADAAPTVSIFPPSSEQLTSGGASVVCFLNNFYPK |
| | | cam |
| | | ANRLVDGVPSR |
| | | DIKMTQSPSSMYASLGER |
| | | DIKMTQSPSSMYASLGER ox |
| | | DIKMTQSPSSMYASLGER ox ox |
| | | HNSYTCEATHK cam |
| | | HNSYTCEATHKTSTSPIVK cam |
| | | MTQSPSSMYASLGER |
| | | MTQSPSSMYASLGER ox |
| | | MTQSPSSMYASLGER ox ox |
| gi\|284924 | Ig light chain V region | ASENIYSYLAWYQQK |
| | (clone 185-c1) | DIQMTQSPASLSASVGETVTITCR ox cam |
| | | FSGSGSGTQFSLK |
| | | QGKSPQLLVYNAK |
| | | SPQLLVYNAK |
| | | TLAEGVPSRFSGSGSGTQFSLK |
| gi\|494164 | Igg2a Fab Fragment | ADAAPTVSIFPPSSEQLTSGGASVVCFLNNFYPK |
| | (Fab 179) | cam |
| | | DIVMTQSPSSLTVTAGEK ox |
| | | HNSYTCEATHK cam |
| | | HNSYTCEATHKTSTSPIVK cam |
| | | NYLTWYQQKPGQPPK |
| gi\|229901 | Igg2b, Kappa | ADAAPTVSIFPPSSEQLTSGGASVVCFLNNFYPK |
| | | cam |
| | | ASQDISNYLNWYQQKPDGTVK |
| | | DIQMTQTTSSLSASLGDR |
| | | DIQMTQTTSSLSASLGDR ox |
| | | DIQMTQTTSSLSASLGDRVTISCR cam |
| | | DIQMTQTTSSLSASLGDRVTISCR ox cam |
| | | DSTYSMSSTLTLTKDEYER ox |
| | | HNSYTCEATHK cam |
| | | HNSYTCEATHKTSTSPIVK cam |
| | | LLIYYTSR |
| | | QNGVLNSWTDQDSK |
| | | TFGGGTKLEIK |
| gi\|62204734 | Inter alpha-trypsin | ADAVQEATFQVELPR |
| | inhibitor, heavy chain | AHGGTNINNAVLLAVELLDR |
| | 4 | AHGGTNINNAVLLAVELLDRSNQAELLPSK |
| | | DIVWEPPVEPDNTKR |
| | | FKPTLSQQQK |
| | | GSEMVVAGKLQDQGPDVLLAK ox |
| | | ILKDLSPQDQFNLIEFSGEANQWK |
| | | IRAHGGTNINNAVLLAVELLDR |
| | | ITFELIYQELLQR |
| | | ITFELIYQELLQRR |
| | | LFVDPSQGLEVTGK |
| | | LPLAAQAHPFRPPVR |
| | | LWALLTIQQQLEQR |
| | | MLSLPSVAQYPADPHLVVTEK ox |
| | | NTEYKWK |
| | | QSLVQATEENLNKAVNYASR |
| | | QTEKFEVSVNVAPGSK |
| | | QYSAAVGRGESAGIVK |
| | | RLGMYELLLK |
| | | RLGMYELLLK ox |
| | | SQSEQDTVLNGDFIVR |
| | | SVSLIILLTDGDPTVGETNPTIIQNNVR |
| | | TLFSVLPGLK |
| | | TVKVQGVDYLATR |
| | | VQGVDYLATR |
| | | VVNRADAVQEATFQVELPR |
| | | YIFHNFMER |
| | | YIFHNFMER ox |
| | | YKFQHHFK |
| gi\|16418335 | Leucine-rich alpha-2- | CAGPEAMKGQR cam |
| | glycoprotein | CAGPEAMKGQR cam ox |
| | | DMQDGFDISHNPWICDKNLADLCR ox cam cam |
| | | ELHLSSNRLQALSPELLAPVPR |
| | | LEDSLLAPQPFLR |
| | | LHLEGNRLQR |
| | | LQALSPELLAPVPR |
| | | LQRLEDSLLAPQPFLR |
| gi\|21594125 | Lifr protein (leukemia | AEIQLSKNDYIISVVAR |
| | inhibitory factor | GAEDSTYHVAVDKLNPYTAYTFR |
| | receptor) | HLTTEATPSKGPDTWR |
| | | IASMEIPNDDITVEQAVGLGNR ox |
| | | INLLCQIEICK cam cam |
| | | LSCETHDLKEIICSWNPGR cam cam |
| | | NDYIISVVAR |
| | | NTEYTLFESISGK |
| | | SEPCLLDWR cam |
| | | YNFYLYGCTNQGYQLLR cam |
| gi\|8569601 | Major urinary protein | AGEYSVTYDGFNTFTIPK |
| | 1 | DGETFQLMGLYGR ox |
| | | EEASSTGRNFNVEK |
| | | EKIEDNGNFR |
| | | ENIIDLSNANR |
| | | FAQLCEEHGILR cam |
| | | FAQLCEEHGILRENIIDLSNANR cam |
| | | IEDNGNFRLFLEQIHVLEK |
| | | INGEWHTIILASDKR |
| | | LFLEQIHVLEK |
| | | TDYDNFLMAHLINEK ox |
| gi\|6754654 | Mannose binding | AIEEKLANMEAEIR |
| | lectin (A) | AIEEKLANMEAEIR ox |
| | | AIQEVATGIAFLGITDEATEGQFMYVTGGR ox |
| | | GEPGQGLRGLQGPPGK |
| | | LANMEAEIR ox |
| | | LFVTNHEKMPFSK ox |
| | | LQLTNKLHAFSMGK ox |
| | | SLCTELQGTVAIPR cam |
| | | SLCTELQGTVAIPRNAEENK cam |
| | | VKSLCTELQGTVAIPR cam |
| gi\|233018 | Mannose binding | AEFDTSEIDSEIAALR |
| | protein C | AEFDTSEIDSEIAALRSELR |
| | | ALCSEFQGSVATPR cam |
| | | ALRNWVLFSLSEK |
| | | DIAYLGITDVR |
| | | DIAYLGITDVRVEGSFEDLTGNR |
| | | NWVLFSLSEK |
| | | VAKDIAYLGITDVR |
| | | VEGSFEDLTGNR |
| | | VEGSFEDLTGNRVR |
| | | VKALCSEFQGSVATPR cam |
| | | WNDVPCSDSFLAICEFSD cam cam |
| | | YTNWNDGEPNNTGDGEDCVVILGNGK cam |
| gi\|2118830 | MHC class I | AKGNEQSFHVSLR |
| | histocompatibility | APWMEQEGPEYWER |
| | antigen H-2 Q10 | APWMEQEGPEYWER ox |
| | alpha chain | APWMEQEGPEYWERETQR |
| | | APWMEQEGPEYWERETQR ox |
| | | AYLEAECVEWLLR cam |
| | | FDSDAETPRMEPR |
| | | FDSDAETPRMEPR ox |
| | | FIIVGYVDDTQFVR |
| | | FIIVGYVDDTQFVRFDSDAETPR |
| | | GNEQSFHVSLR |
| | | GYLQYAYDGR |
| | | GYLQYAYDGRDYIALNEDLK |
| | | KWEQAGAAEYYR |
| | | TDPPKTHVTHHPGSEGDVTLR |
| | | THVTHHPGSEGDVTLR |
| | | TWTAADVAAIITR |
| | | WASVVVPLGK |
| | | WEQAGAAEYYR |
| | | YFETSVSRPGLGEPR |
| | | YLELGKETLLR |
| gi\|387437 | MHC factor B | AEGIPEFYDYDVALVK |
| | | ALFVSEQGK |
| | | ALLDIGRDPK |
| | | ALRLPQTATCK cam |
| | | CLTNLIEK cam |
| | | DNEHHVFK |
| | | FIQVGVISWGVVDVCR cam |
| | | FLCTGGVDPYADPNTCK cam cam |
| | | GDSGGPLIVHKR |
| | | GNDYHKQPWQAK |
| | | HVIIIMTDGLHNMGGNPVTVIQDIR ox |
| | | HVIIIMTDGLHNMGGNPVTVIQDIR ox ox |
| | | ISVTRPLK |
| | | KAEGIPEFYDYDVALVK |
| | | KDNEHHVFK |
| | | LKDEDLGFL |
| | | LKYGQTLRPICLPCTEGTTR cam cam |
| | | LNQISYEDHKLK |
| | | RCLTNLIEK cam |
| | | RDLEIEEVLFHPK |
| | | SLSLCGMVWEHK cam |
| | | SLSLCGMVWEHK cam ox |
| | | SLSLCGMVWEHKK cam |
| | | SLSLCGMVWEHKK cam ox |
| | | VASYGVRPR |
| | | VKDASEVVTPR |
| | | VKDMEDLENVFYQMIDETK ox ox |
| | | VSVGGQRR |
| | | YGLLTYATVPK |
| | | YGQTLRPICLPCTEGTTR cam cam |
| gi\|55217 | Murine valosin- | EAVCIVLSDDTCSDEKIR cam cam |
| | containing protein | ELQELVQYPVEHPDKFLK |
| | | EVDIGIPDATGRLEILQIHTK |
| | | GILLYGPPGTGK |
| | | GVLFYGPPGCGK cam |
| | | IVSQLLTLMDGLK |
| | | IVSQLLTLMDGLKQR |
| | | IVSQLLTLMDGLKQR ox |
| | | KYEMFAQTLQQSR ox |
| | | MTNGFSGADLTEICQR ox cam |
| | | QAAPCVLFFDELDSIAK cam |
| | | WALSQSNPSALR |
| gi\|6679182 | Orosomucoid 1 | AVTHVGMDESEIIFVDWK ox |
| | | AVTHVGMDESEIIFVDWKK |
| | | AVTHVGMDESEIIFVDWKK ox |
| | | HGAFMLAFDLKDEK |
| | | HGAFMLAFDLKDEK ox |
| | | RPDITPELR |
| | | RPDITPELREVFQK |
| | | WFFMGAAFR |
| | | WFFMGAAFR ox |
| | | YEGGVETFAHLIVLR |
| | | YEGGVETFAHLIVLRK |
| gi\|31982113 | Plasminogen [Mus | CEGETDFVCR cam cam |
| | musculus] | CQSWAAMFPHR cam |
| | | CQSWAAMFPHR cam ox |
| | | CQSWAAMFPHRHSK cam |
| | | CQSWAAMFPHRHSK cam ox |
| | | CTTPPPPPSPTYQCLK cam cam |
| | | FTGQHFCGGTLIAPEWVLTAAHCLEK cam cam |
| | | FVDWIEREMR |
| | | FVDWIEREMR ox |
| | | GENYRGTVSVTVSGK |
| | | GKTAVTAAGTPCQGWAAQEPHR cam |
| | | HNRTPENFPCK cam |
| | | HSIFTPQTNPR |
| | | HSKTPENFPDAGLEMNYCR cam |
| | | HSKTPENFPDAGLEMNYCR ox cam |
| | | KCQSWAAMFPHR cam |
| | | KCQSWAAMFPHR cam ox |
| | | LILEPNNRDIALLK |
| | | LKEAQLPVIENK |
| | | LSRPATITDK |
| | | LSRPATITDKVIPACLPSPNYMVADR cam |
| | | LSRPATITDKVIPACLPSPNYMVADR cam ox |
| | | MRDVILFEK |
| | | MRDVILFEK ox |
| | | NLEENYCR cam |
| | | NLEENYCRNPDGETAPWCYTTDSQLR cam cam |
| | | NPDGDKGPWCYTTDPSVR cam |
| | | NPDGDVNGPWCYTTNPR cam |
| | | NPDGDVNGPWCYTTNPRK cam |
| | | NPDGEPRPWCFTTDPTK cam |
| | | NPDGEPRPWCFTTDPTKR cam |
| | | NPDNDEQGPWCYTTDPDKR cam |
| | | NYCRNPDGDVNGPWCYTTNPR cam cam |
| | | QLAAGGVSDCLAK cam |
| | | QLAAGGVSDCLAKCEGETDFVCR cam cam cam |
| | | RVYLSECK cam |
| | | SFQYHSKEQQCVIMAENSK cam |
| | | TAVTAAGTPCQGWAAQEPHR cam |
| | | TGIGNGYRGTMSR ox |
| | | TICYITGWGETQGTFGAGR cam |
| | | TPENFPCKNLEENYCR cam cam |
| | | TPENFPDAGLEMNYCR cam |
| | | TPENFPDAGLEMNYCR ox cam |
| | | VCNRVEYLNNR cam |
| | | VILGAHEEYIR |
| | | VILGAHEEYIRGSDVQEISVAK |
| | | VIPACLPSPNYMVADR cam |
| | | VIPACLPSPNYMVADR cam ox |
| | | VSRFVDWIER |
| | | WEYCDIPR cam |
| | | WEYCNLKR cam |
| | | WGATFPHVPNYSPSTHPNEGLEENYCR cam |
| | | WSEQTPHRHNR |
| | | YDYCNIPECEEECMYCSGEKYEGK cam cam cam ox |
| | | cam |
| gi\|2144495 | PLMS plasmin | CEGETDFVCR cam cam |
| | | CQSWAAMFPHR cam |
| | | CQSWAAMFPHR cam ox |
| | | CTTPPPPPSPTYQCLK cam cam |
| | | EQQCVIMAENSK cam ox |
| | | FTGQHFCGGTLIAPEWVLTAAHCLEK cam cam |
| | | FVDWIEREMR ox |
| | | GENYRGTVSVTVSGK |
| | | GKTAVTAAGTPCQGWAAQEPHR cam |
| | | HSIFTPQTNPR |
| | | KCQSWAAMFPHR cam |
| | | KCQSWAAMFPHR cam ox |
| | | LI LEPNNRDIALLK |
| | | LKEAQLPVIENK |
| | | LSRPATITDK |
| | | LYDYCDIPLCASASSFECGKPQVEPKK cam cam |
| | | cam |
| | | MRDVILFEK |
| | | MRDVILFEK ox |
| | | NLEENYCR cam |
| | | NPDGDKGPWCYTTDPSVR cam |
| | | NPDGDVNGPWCYTTNPR cam |
| | | NPDGETAPWCYTTDSQLR cam |
| | | NPDNDEQGPWCYTTDPDKR cam |
| | | NYCRNPDGDVNGPWCYTTNPR cam cam |
| | | QLAAGGVSDCLAKCEGETDFVCR cam cam cam |
| | | TAVTAAGTPCQGWAAQEPHR cam |
| | | TICYITGWGETQGTFGAGR cam |
| | | TPENFPDAGLEMNYCR cam |
| | | TPENFPDAGLEMNYCR ox cam |
| | | VCNRVEYLNNR cam |
| | | VILGAHEEYIR |
| | | VILGAHEEYIRGLDVQEISVAK |
| | | VIPACLPSPNYMVADR cam |
| | | VIPACLPSPNYMVADR cam ox |
| | | VSRFVDWIER |
| | | WEYCDIPR cam |
| | | WEYCNLKR cam |
| | | WSEQTPHRHNR |
| gi\|6680608 | Pregnancy zone | AAPLSLCALTAVDQSVLLLKPEAK cam |
| | protein | AESPVFVQTDKPIYKPGQIVKFR |
| | | AINYLISGYQR |
| | | ALLAYAFALAGNK |
| | | ALLAYAFALAGNKAK |
| | | AMGVPMMGLDYSDEINQVVEVR ox ox |
| | | EEGTGIELTGIGSCEIANALSKLK cam |
| | | HSLGDNDAHSIFQSVGINIFTNSK |
| | | HSLGDNDAHSIFQSVGINIFTNSKIHKPR |
| | | LSPQSIYNLLPGK |
| | | SKAINYLISGYQR |
| | | SVIVEPEGIEK |
| | | VKALSFYQPR |
| | | VNTNYRPGLPFSGQVLLVDEK |
| gi\|53787 | Properdin (AA 5 - | CGGHCPGEAQQSQACDTQK cam cam cam |
| | 441) | DIRVEDCCLNAAYAFQEHDGGLCQACR cam cam |
| | | cam cam |
| | | HCYNIHNCIMK cam cam |
| | | HCYNIHNCIMK cam cam ox |
| | | HGGPFCAGDATR cam |
| | | HGGPFCAGDATRNQMCNK cam ox cam |
| | | LQDIRHCYNIHNCIMK cam cam |
| | | LQDIRHCYNIHNCIMK cam cam ox |
| | | LRMSINCEGTPGQQSR cam |
| | | LRMSINCEGTPGQQSR ox cam |
| | | MSINCEGTPGQQSR cam |
| | | MSINCEGTPGQQSR ox cam |
| | | QRLCTPLLPK cam |
| | | QRVCDNPAPK cam |
| | | SCSAPAPSHQPPGKPCSGPAYEHK cam cam |
| | | SRSCSAPAPSHQPPGKPCSGPAYEHK cam cam |
| gi\|49868 | Put. beta-actin (aa | DLYANTVLSGGTTMYPGIADR |
| | 27-375) | DLYANTVLSGGTTMYPGIADR ox |
| | | DSYVGDEAQSK |
| | | DSYVGDEAQSKR |
| | | GYSFTTTAER |
| | | GYSFTTTAEREIVR |
| | | HQGVMVGMGQK ox ox |
| | | IWHHTFYNELR |
| | | KDLYANTVLSGGTTMYPGIADR ox |
| | | LCYVALDFEQEMATAASSSSLEK cam ox |
| | | LDLAGRDLTDYLMK |
| | | LDLAGRDLTDYLMK ox |
| | | QEYDESGPSIVHR |
| | | QEYDESGPSIVHRK |
| | | SYELPDGQVITIGNER |
| | | VAPEEHPVLLTEAPLNPK |
| gi\|34785996 | Pzp protein | AAPLSLCALTAVDQSVLLLKPEAK cam |
| | | AESPVFVQTDKPIYKPGQIVK |
| | | AESPVFVQTDKPIYKPGQIVKFR |
| | | AFAQAQSHIFIEK |
| | | AINYLISGYQR |
| | | ALLAYAFALAGNK |
| | | APSAEVEMTAYVLLAYLTSESSRPTR ox |
| | | ATVLNYMSHCIQIR ox cam |
| | | DAVKEEDSLHWQRPGDVQK |
| | | DLTFYYLIK |
| | | EEGTGIELTGIGSCEIANALSKLK cam |
| | | ENGCFQQSGYLLNNAMK cam |
| | | ENGCFQQSGYLLNNAMK cam ox |
| | | GSGSGCVYLQTSLK cam |
| | | GSIFNLGSHVLSLEQGNMK |
| | | GSIFNLGSHVLSLEQGNMK ox |
| | | HSLGDNDAHSIFQSVGINIFTNSK |
| | | IHYLLNEDIMKNEK ox |
| | | KIEHSFEVK |
| | | KLQDQPNIQR |
| | | LPDLPGNYVTK |
| | | LSPQSIYNLLPGK |
| | | LTEVPALVHKDTVVK |
| | | NEKDLTFYYLIK |
| | | SKAINYLISGYQR |
| | | SQKEVLVTIESSGTFSK |
| | | TEVNTNHVLIYIEK |
| | | THITNAFNWLSMK ox |
| | | TVQGAFFGVPVYK |
| | | VKALSFYQPR |
| | | VNTNYRPGLPFSGQVLLVDEK |
| | | YNILPVADGK |
| gi\|33859612 | Retinol binding | DPNGLSPETR |
| | protein 4 | DPNGLSPETRR |
| | | FSGLWYAIAK |
| | | FSGLWYAIAKK |
| | | LLSNWEVCADMVGTFTDTEDPAKFK cam ox |
| | | LQNLDGTCADSYSFVFSR cam |
| | | LQNLDGTCADSYSFVFSRDPNGLSPETR cam |
| | | MKYWGVASFLQR |
| | | MKYWGVASFLQR ox |
| | | QEELCLER cam |
| | | QRQEELCLER cam |
| | | QYRWIEHNGYCQSRPSR cam |
| | | WIEHNGYCQSRPSR cam |
| | | YWGVASFLQR |
| gi\|6678083 | Serine (or cysteine) | AVLTMDETGTEAAAATVLLAVPYSMPPIVR |
| | proteinase inhibitor, | AVLTMDETGTEAAAATVLLAVPYSMPPIVR ox |
| | clade A, member 1 a | AVLTMDETGTEAAAATVLLAVPYSMPPIVR ox ox |
| | | DQSPASHEIATNLGDFAISLYR |
| | | FDHPFLFIIFEEHTQSPLFVGK |
| | | FLEEAKNHYQAEVFSVNFAESEEAK |
| | | IFNNGADLSGITEENAPLK |
| | | IFNNGADLSGITEENAPLKLSQAVHK |
| | | KLDQDTVFALANYILFK |
| | | KPFDPENTEEAEFHVDESTTVK |
| | | LAQIHFPRLSISGEYNLK |
| | | LDQDTVFALANYILFK |
| | | LSISGEYNLK |
| | | MQHLEQTLSKELISK |
| | | NHYQAEVFSVNFAESEEAK |
| | | NHYQAEVFSVNFAESEEAKK |
| | | RLAQIHFPR |
| | | SFQHLLQTLNRPDSELQLSTGNGLFVNNDLK |
| | | SFQHLLQTLNRPDSELQLSTGNGLFVNNDLKLVEK |
| | | TLMSPLGITR ox |
| | | VINDFVEKGTQGK |
| | | WKKPFDPENTEEAEFHVDESTTVK |
| gi\|15029662 | Serine (or cysteine) | DQSPASHEIATNLGDFAISLYR |
| | proteinase inhibitor, | FDHPFLFIIFEEHTQSPIFVGK |
| | clade A, member 1 a | IFNNGADLSGITEENAPLK |
| | | IFNNGADLSGITEENAPLKLSQAVHK |
| | | KLDQDTVFALANYILFK |
| | | KPFDPENTEEAEFHVDESTTVK |
| | | LDQDTVFALANYILFK |
| | | LSISGEYNLK |
| | | LSQAVHKAVLTIDETGTEAAAVTVLQMVPMSMPPILR |
| | | ox ox ox |
| | | MQHLEQTLSK |
| | | NHYQAEVFSVNFAESEEAK |
| | | NHYQAEVFSVNFAESEEAKK |
| | | RLAQIHFPR |
| | | TLMSPLGITR |
| | | TLMSPLGITR ox |
| | | VINDFVEKGTQGK |
| | | WKKPFDPENTEEAEFHVDESTTVK |
| gi\|6678085 | Serine (or cysteine) | DQSPASHEIATNLGDFALR |
| | proteinase inhibitor, | ELISQFLLNRR |
| | clade A, member 1d | FDHPFLFIIFEEHTQSPIFVGK |
| | | FLEEAKNHYQAEVFSVNFAESEEAK |
| | | IFNNGADLSGITEENAPLK |
| | | IFNNGADLSGITEENAPLKLSK |
| | | KLDQDTVFALANYILFK |
| | | KVINDFVEK |
| | | LDQDTVFALANYILFK |
| | | LSISGNYNLK |
| | | MQHLEQTLNKELISQFLLNR |
| | | NHYQAEVFSVNFAESEEAK |
| | | NHYQAEVFSVNFAESEEAKK |
| | | QPFDPENTEEAEFHVDESTTVK |
| | | RSDAQIHIPR |
| | | SDAQIHIPR |
| | | TLMSPLGITR |
| | | TLMSPLGITR ox |
| | | TLMSPLGITRIFNNGADLSGITEENAPLK ox |
| | | VINDFVEKGTQGK |
| | | WKQPFDPENTEEAEFHVDESTTVK |
| gi\|6678087 | Serine (or cysteine) | DQSPASHEIATNLGDFAISLYR |
| | proteinase inhibitor, | FLEEAKNHYQAEVFSVNFAESEEAK |
| | clade A, member 1e | IFNSGADLSGITEENAPLK |
| | | IFNSGADLSGITEENAPLKLSQAVHK |
| | | KLEQDTVFVLANYILFK |
| | | KPFDPENTK |
| | | KVINDFVEK |
| | | LAQIHIPRLSISGNYNLETLMSPLGITR |
| | | LAQIHIPRLSISGNYNLETLMSPLGITR ox |
| | | LEQDTVFVLANYILFK |
| | | LSISGNYNLETLMSPLGITR |
| | | LSISGNYNLETLMSPLGITR ox |
| | | MQHLEQTLNKELISK |
| | | MQHLEQTLNKELISK ox |
| | | NHYQAEVFSVNFAESEEAKK |
| | | QAEFHVDESTTVK |
| | | RLAQIHIPR |
| | | VINDFVEKGTQGK |
| | | WKKPFDPENTK |
| gi\|18044689 | Serine (or cysteine) | ALYQTEAFTADFQQPTEAK |
| | proteinase inhibitor, | AVLDVAETGTEAAAATGVIGGIR |
| | clade A, member 3K | AVLDVAETGTEAAAATGVIGGIRK |
| | | DLQILAEFHEK |
| | | FSIASNYR |
| | | HFRDEELSCSVLELK cam |
| | | ISFDPQDTFESEFYLDEKR |
| | | KTLFPSQIEELNLPK |
| | | LALKNPDTNIVFSPLSISAALALVSLGAK |
| | | MQQVEASLQPETLR ox |
| | | MQQVEASLQPETLRK ox |
| | | TLFPSQIEELNLPK |
| | | TLMVLVNYIYFK ox |
| gi\|18252782 | Serine (or cysteine) | ANRPFLVLIR |
| | proteinase inhibitor, | ANRPFLVLIREVALNTIIFMGR ox |
| | clade C | ATEEDGSEQKVPEATNR |
| | (antithrombin), | DIPVNPLCIYR cam |
| | member 1 | DIPVNPLCIYRSPGK cam |
| | | ENPEQSRVTINNWVANK |
| | | EQLQDMGLIDLFSPEK ox |
| | | EVALNTIIFMGR |
| | | EVALNTIIFMGR ox |
| | | FATNFYQHLADSK |
| | | FRTEDGFSLK |
| | | IKDVIPQGAINELTALVLVNTIYFK |
| | | LQPLDFKENPEQSR |
| | | NDNDNIFLSPLSISTAFAMTK ox |
| | | QLMEVFKFDTISEK |
| | | QLMEVFKFDTISEK ox |
| | | SKFSPENTR |
| | | SLNPNRVTFK |
| | | SQLPGIVAGGRDDLYVSDAFHK |
| | | SSDLVSANRLFGDK |
| | | TSDQIHFFFAK |
| | | VAEGTQVLELPFKGDDITMVLILPKPEK |
| | | VAEGTQVLELPFKGDDITMVLILPKPEK ox |
| | | VDGQSCPVPMMYQEGK cam ox ox |
| | | VDGQSCPVPMMYQEGK ox cam |
| gi\|6679383 | Serine (or cysteine) | DFFHLDER |
| | proteinase inhibitor, | FDPSLTQKDFFHLDER |
| | clade F, member 2 | FTVSVDMMHAVSYPLR ox |
| | | FTVSVDMMHAVSYPLR ox ox |
| | | GFPIKDDFLEQSER |
| | | LAPRMEEDYPQFSSPK ox |
| | | LDNQDFGDHATLK |
| | | LDNQDFGDHATLKR |
| | | LFGAKPVK |
| | | LIGQNDKADFHGGK |
| | | LPLPALFK |
| | | LTGKQEEDLANINQWVK |
| | | NPNPSALPQLQEQR |
| | | NPNPSALPQLQEQRDSPDNR |
| | | QEEDLANINQWVK |
| | | SVPTAEETRR |
| | | WFLLEQPEIQVAHFPFK |
| gi\|15929675 | Serpina 1a protein | AVLTIDETGTEAAAVTVLLAVPYSMPPILR |
| | | AVLTIDETGTEAAAVTVLLAVPYSMPPILR ox |
| | | DQSPASHEIATNLGDFAISLYR |
| | | FDHPFLFIIFEEHTQSPLFVGK |
| | | FLEEAKNHYQAEVFSVNFAESEEAK |
| | | IFNNGADLSGITEENAPLK |
| | | IFNNGADLSGITEENAPLKLSQAVHK |
| | | KLDQDTVFALANYILFK |
| | | KPFDPENTEEAEFHVDESTTVK |
| | | KVINDFVEK |
| | | LDQDTVFALANYILFK |
| | | LSISGEYNLK |
| | | MQHLEQTLSK |
| | | MQHLEQTLSK ox |
| | | NHYQAEVFSVNFAESEEAK |
| | | NHYQAEVFSVNFAESEEAKK |
| | | RLAQIHFPR |
| | | SFQHLLQTLNRPDSELQLSTGNGLFVNNDLK |
| | | SFQHLLQTLNRPDSELQLSTGNGLFVNNDLKLVEK |
| | | TLMSPLGITR |
| | | TLMSPLGITR ox |
| | | VINDFVEKGTQGK |
| | | WKKPFDPENTEEAEFHVDESTTVK |
| gi\|14602605 | Serpina1a protein | AVLTIDETGTEAAAVTVLLAVPYSMPPILR |
| | | AVLTIDETGTEAAAVTVLLAVPYSMPPILR ox |
| | | DQSPASHEIATNLGDFAISLYR |
| | | FDHPFLFIIFEEHTQSPLFVGK |
| | | FLEEAKNHYQAEVFSVNFAESEEAK |
| | | FLLNRR |
| | | IFNNGADLSGITEENAPLK |
| | | IFNNGADLSGITEENAPLKLSQAVHK |
| | | KLDQDTVFALANYILFK |
| | | KPFDPENTEEAEFHVDESTTVK |
| | | KVINDFVEK |
| | | LAQIHFPR |
| | | LDQDTVFALANYILFK |
| | | LSISGEYNLK |
| | | MQHLEQTLSK ox |
| | | NHYQAEVFSVNFAESEEAK |
| | | NHYQAEVFSVNFAESEEAKK |
| | | RLAQIHFPR |
| | | SFQHLLQTLNRPDSELQLSTGNGLFVNNDLK |
| | | TLMSPLGITR |
| | | TLMSPLGITR ox |
| | | TLMSPLGITRIFNNGADLSGITEENAPLK |
| | | VINDFVEK |
| | | VINDFVEKGTQGK |
| | | WKKPFDPENTEEAEFHVDESTTVK |
| gi\|38174334 | Serum amyloid P- | APPSIVLGQEQDNYGGGFQR |
| | component | EKVGEYSLYIGQSK |
| | | EYTVKAPPSIVLGQEQDNYGGGFQR |
| | | GRDNELLIYK |
| | | SQSLFSYSVKGR |
| | | TYSDLSRSQSLFSYSVK |
| | | VFVFPRESETDHVK |
| | | VGEYSLYIGQSK |
| | | VGEYSLYIGQSKVTVR |
| gi\|20330802 | Transferrin | ADRDQYELLCLDNTR cam |
| | | AVLTSQETLFGGSDCTGNFCLFK cam cam |
| | | AVSSFFSGSCVPCADPVAFPK cam cam |
| | | CAPNNKEEYNGYTGAFR cam |
| | | CFVKLPEGTTPEK cam |
| | | CLKDGGGDVAFVK cam |
| | | CLVEKGDVAFVK cam |
| | | DFASCHLAQAPNHVVVSR cam |
| | | DFASCHLAQAPNHVVVSRK cam |
| | | DFQLFSSPLGK |
| | | DGGGDVAFVK |
| | | DLLFKDSAFGLLR |
| | | DLLFRDDTK |
| | | DQYELLCLDNTR cam |
| | | DSAFGLLR |
| | | DSAFGLLRVPPR |
| | | FDEFFSQGCAPGYEK cam |
| | | GTDFQLNQLEGKK |
| | | GYYAVAVVK |
| | | HQTVLDNTEGKNPAEWAK |
| | | IPSHAVVAR |
| | | KPVKDFASCHLAQAPNHVVVSR cam |
| | | KSCHTGLGR cam |
| | | KSCHTGVDR cam |
| | | LCQLCPGCGCSSTQPFFGYVGAFK cam cam cam |
| | | cam |
| | | LLEACTFHKH cam |
| | | LYLGHNYVTAIR |
| | | NLKQEDFELLCPDGTR cam |
| | | NQQEGVCPEGSIDNSPVK cam |
| | | NQQEGVCPEGSIDNSPVKVVCALSHLER cam cam |
| | | SAGWVIPIGLLFCK cam |
| | | SCHTGLGR cam |
| | | SCHTGVDR cam |
| | | SKDFQLFSSPLGK |
| | | TAGWNIPMGMLYNR ox |
| | | TAGWNIPMGMLYNR ox ox |
| | | TSYPDCIK cam |
| | | TVLPPDGPR |
| | | VAQEHFGK |
| | | VPPRMDYR ox |
| | | WCALSHLER cam |
| | | WCAVSEHENTK cam |
| | | YLGAEYMQSVGNMR |
| | | YLGAEYMQSVGNMR ox |
| | | YLGAEYMQSVGNMRK |
| | | YLGAEYMQSVGNMRK ox |
| | | YLGAEYMQSVGNMRK ox ox |
| gi\|19354093 | Transthyretin | FVEGVYRVELDTK |
| | | GSPAVDVAVKVFK |
| | | HYTIAALLSPYSYSTTAVVSNPQN |
| | | KTSEGSWEPFASGK |
| | | LFLLCLAGLVFVSEAGPAGAGESKCPLMVK cam |
| | | cam ox |
| | | TAESGELHGLTTDEK |
| | | TAESGELHGLTTDEKFVEGVYR |
| | | TLGISPFHEFADVVFTANDSGHR |
| | | TSEGSWEPFASGK |
| | | VELDTKSYWK |
| | | VLDAVRGSPAVDVAVK |
| gi\|111243 | Vitamin D-binding | CCESTSEDCMASELPEHTIK cam cam cam ox |
| | protein | DLCGQSTTQAMDQYTFELSR cam |
| | | DLCGQSTTQAMDQYTFELSR cam ox |
| | | DLCGQSTTQAMDQYTFELSRR cam |
| | | DLCGQSTTQAMDQYTFELSRR cam ox |
| | | ECCDTQDSVACFSTQSPLLKR cam cam cam |
| | | EWSLTEECCAEGADPTCYDTR cam cam cam |
| | | FSSSTFEQVNQLVK |
| | | GFADQFLYEYSSNYGQAPLPLLVAYTK |
| | | GQEMCADYSENTFTEYK cam |
| | | GQEMCADYSENTFTEYKK ox cam |
| | | HLSLLTTMSNR |
| | | HLSLLTTMSNR ox |
| | | KFSSSTFEQVNQLVK |
| | | KLCMAALSHQPQEFPTYVEPTNDEICEAFR cam ox |
| | | cam |
| | | LAQKVPTANLENVLPLAEDFTEILSR |
| | | LPTGKDLCGQSTTQAMDQYTFELSR cam ox |
| | | LQMKHLSLLTTMSNR ox |
| | | NYLSMVGSCCTSANPTVCFVK cam cam cam |
| | | NYLSMVGSCCTSANPTVCFVK ox cam cam cam |
| | | QLTSFIEKGQEMCADYSENTFTEYK ox cam |
| | | RTQVPEVFLSK |
| | | SCESDAPFPVHPGTPECCTK cam cam cam |
| | | SCESDAPFPVHPGTPECCTKEGLER cam cam cam |
| | | SLSLILYSR |
| | | TLRECCDTQDSVACFSTQSPLLK cam cam cam |
| | | TQVPEVFLSK |
| | | VCSQYAAYGK cam |
| | | VCSQYAAYGKEK cam |
| | | VPTANLENVLPLAEDFTEILSR |
| gi\|1083568 | Zinc-alpha 2- | AREEIFLVTLK |
| | glycoprotein | AYLEEECPEMLKR cam |
| | | AYLEEECPEMLKR cam ox |
| | | CLAYGFYPQR cam |
| | | FQATAFLNDQAFFHYNSNSGK |
| | | GFSQSLSVQWDR |
| | | SHLDRIDPPTVTITSR |
| | | VIPGGNRIFK |
| | | WEAEKVYVQR |
| | | YAYDGEDFIEFNK |

| | | |
|---|---|---|
| Legenda ox = methionin oxidation cam = carbamidomethyl oxidation | | |

### References

(1) Zerbini, A.; Pilli, M.; Ferrari, C.; Missale, G. Dig. Liver Dis. 2006, 38(4), 221-5.
(2) Jemal, A.; Murray, T.; Ward, E.; Samuels, A.; Tiwari, R. C.; Ghafoor, A.; Feuer, E. J.; Thun, M. J. CA Cancer J. Clin. 2005, 55(1),10-30.
(3) Chen, C. J.; Yu, M. W.; Liaw, Y. F. J. Gastroenterol. Hepatol. 1997, 12(9-10), S294-308.
(4) Santella, R. M.; Zhang, Y. J.; Young, T. L.; Lee, B. M.; Lu, X. Q. Adv. Exp. Med. Biol. 1991, 283,165-81.
(5) Chen, S. Y.; Wang, L. Y.; Lunn, R. M.; Tsai, W. Y.; Lee, P. H.; Lee, C. S.; Ahsan, H.; Zhang, Y. J.; Chen, C. J.; Santella, R. M. Int J Cancer 2002, 99(1), 14-21.
(6) Grizzi, F.; Franceschini, B.; Hamrick, C.; Frezza, E. E.; Cobos, E.; Chiriva-Internati, M. J. Transl. Med. 2007, 5 3
(7) Zhou, L.; Liu, J.; Luo, F. World J Gastroenterol 2006, 12(8), 1175-1181.
(8) Borlak, J.; Meier, T.; Halter, R.; Spanel, R.; Spanel-Borowski, K. Oncogene 2005, 24(11), 1809-1819.
(9) Sebastian, S.; Settleman, J.; Reshkin, S. J.; Azzariti, A.; Bellizzi, A.; Paradiso, A. Biochim.Biophys.Acta 2006, 1766(1), 120-139.
(10) Yano, S.; Kondo, K.; Yamaguchi, M.; Richmond, G.; Hutchison, M.; Wakeling, A.; Averbuch, S.; Wadsworth, P. Anticancer Res 2003, 23(5A), 3639-3650.
(11) Tonjes, R.R.; Lohler, J.; O'Sullivan, J. F.; Kay, G. F.; Schmidt, G. H.; Dalemans, W.; Pavirani, A.; Paul, D. Oncogene 1995, 10(4),765-8.
(12) Shen, H; Cheng, G; Fan, H; Zhang, J; Zhang, X; Lu, H; Liu, C; Sun, F; Jin, H; Xu, X; Xu, G; Wang, S; Fang, C; Bao, H; Wang, Y; Wang, J; Zhong, H; Yu, Z; Liu, Y; Tang, Z; Yang, P. Proteomics, 2006, 6(2), 528-37.
(13) Cui, F; Wang, Y; Wang, J; Wei, K; Hu, J; Liu, F; Wang, H; Zhao, X; Zhang, X; Yang, X. Proteomics, 2006, 6(2), 498-504.
(14) Wisniewski, T.; Golabek, A. A.; Kida, E.; Wisniewski, K. E.; Frangione, B. Am J Pathol. 1995, 147(2), 238-244.
(15) Gerner, C.; Steinkellner, W.; Holzmann, K.; Gsur, A.; Grimm, R.; Ensinger, C.; Obrist, P.; Sauermann, G. Thromb Haemost. 2001, 85(3), 494-501.
(16) Vejda, S.; Posovszky, C.; Zelzer, S.; Peter, B.; Bayer, E.; Gelbmann, D.; Schulte-Hermann, R.; Gerner, C. Mol Cell Proteomics. 2002, 1(5), 387-393.
(17) Fan, B. L.; Zhu, W. L.; Zou, G. L.; Luo, G. S.; Xu, C. L.; Zhao, W. X. Ai.Zheng. 2004, 23(3), 249-253.
(18) Vassalli, J. D.; Sappino, A. P.; Belin, D. J Clin Invest 1991, 88 (4), 1067-1072.
(19) Sudhoff, T.; Schneider, W. Clin Investig. 1992, 70(8), 631-636.
(20) Brunner, G.; Preissner, K. T. Blood Coagul.Fibrinolysis 1994, 5(4), 625-639.
(21) Falleti, E.; Pirisi, M.; Fabris, C.; Bortolotti, N.; Soardo, G.; Toniutto, P.; Gonano, F.; Bartoli, E. EurJ Clin Chem.Clin Biochem. 1993, 31(7), 407-411.
(22) Perier, C.; Chamson, A.; Engler, R.; Frey, J. Clin Chem. 1983, 29 (1), 45-47.
(23) Chio, L. F.; Oon, C. J. Cancer 1979, 43(2), 596-604.
(24) Lichtor, T. Neurosci Lett. 1992, 138(2), 287-90.
(25) Tanne, J. H. BMJ. 1999, 319(7211), 662.
(26) Hough, C. D.; Sherman-Baust, C. A.; Pizer, E. S.; Montz, F. J.; Im, D. D.; Rosenshein, N. B.; Cho, K. R.; Riggins, G. J.; Morin, P. J. Cancer Res. 2000, 60(22), 6281-7.
(27) Niemi, M.; Kervinen, K.; Kiviniemi, H.; Lukkarinen, O.; Kyllonen, A. P.; Apaja-Sarkkinen, M.; Savolainen, M. J.; Kairaluoma, M. I.; Kesaniemi, Y. A. J Epidemiol Community Health. 2000, 54(12), 938-9.
(28) Yokoyama, Y.; Kuramitsu, Y.; Takashima, M.; lizuka, N.; Terai, S.; Oka, M.; Nakamura, K.; Okita, K.; Sakaida, I. Int J Oncol. 2006, 28(3), 625-631.
(29) Xu, N.; Hurtig, M.; Zhang, X. Y.; Ye, Q.; Nilsson-Ehle, P. Biochim.Biophys.Acta 2004, 1683(1-3), 33-37.
(30) Luo, G.; Zhang, X.; Nilsson-Ehle, P.; Xu, N. Lipids Health Dis 2004, 3 21.
(31) Richter, S.; Shih, D. Q.; Pearson, E. R.; Wolfrum, C.; Fajans, S. S.; Hattersley, A. T.; Stoffel, M. Diabetes 2003, 52(12), 2989-2995.
(32) Karlsson, H.; Leanderson, P.; Tagesson, C.; Lindahl, M. Proteomics. 2005, 5(5), 1431-1445.
(33) Ruiu, G.; Gambino, R.; Veglia, F.; Pagano, G.; Cassader, M. Clin Genet 1997, 52(3), 167-172.
(34) Chiang, M. T.; Otomo, M. I.; Itoh, H.; Furukawa, Y.; Kimura, S.; Fujimoto, H. Lipids 1991, 26(1), 46-52.
(35) Averna, M.; Paravizzini, G.; Marino, G.; Lanteri, E.; Cavera, G.; Barbagallo, C. M.; Petralia, S.; Cavallaro, S.; Magro, G.; Grasso, S.; Notarbartolo, A.; Travali, S. Int J Clin Lab Res 1997, 27(3), 207-212.
(36) Okkels, H.; Rasmussen, T. E.; Sanghera, D. K.; Kamboh, M. I.; Kristensen, T. Eur J Biochem. 1999, 259(1-2), 435-440.
(37) Petersen, C. M.; Jensen, P. H.; Bukh, A.; Sunde, L.; Lamm, L. U.; Ingerslev, J. Scand J Clin Lab Invest 1990, 50(5), 479-485.
(38) Teng, H.; Zhang, W. Y.; Zhu, F. Q. Chin Med J (Engl.) 1994, 107(12), 910-914.
(39) Bhattacharya, M.; Barlow, J. J. Int Adv.Surg.Oncol. 1979, 2 155-176.
(40) Bierl, C.; Voetsch, B.; Jin, R. C.; Handy, D. E.; Loscalzo, J. J Biol Chem. 2004, 279(26), 26839-26845.
(41) Giannattasio, A.; De Rosa, M.; Smeraglia, R.; Zarrilli, S.; Cimmino, A.; Di Rosario, B.; Ruggiero, R.; Colao, A.; Lombardi, G. J Endocrinol Invest 2002, 25(11), 983-986.
(42) Burk, R. F.; Early, D. S.; Hill, K. E.; Palmer, I. S.; Boeglin, M. E. Hepatology 1998, 27(3), 794-8.
(43) Reszka, E.; Gromadzinska, J.; Stanczyk, M.; Wasowicz, W. Biol Trace Elem. Res 2005, 104(2), 165-172.
(44) Chignard, N; Shang, S; Wang, H; Marrero, J; Bréchot, C; Hanash, S; Beretta L. Gastroenterology, 2006, 130(7), 2010-22.
(45) Lauer, G.; Sollberg, S.; Cole, M.; Flamme, I.; Sturzebecher, J.; Mann, K.; Krieg, T.; Eming, S. A. J Invest Dermatol. 2000, 115(1), 12-18.
(46) Bhat, VB; Choi, MH; Wishnok, JS; Tannenbaum, SR. J Proteome Res., 2005, 4(5), 1814-25.
(47) Andersson, C.; Gelin, J.; Iresjo, B. M.; Lundholm, K. J Surg.Res 1993, 55(6), 607-614.
(48) Van Molle, W.; Libert, C.; Fiers, W.; Brouckaert, P. J Immunol 1997, 159(7), 3555-3564.
(49) Agrawal, R. S.; Karhu, K.; Laukkanen, J.; Kirkinen, P.; Yla-Herttuala, S.; Agrawal, Y. P. Gene Ther. 1999, 6(1), 146-148.
(50) De Masi, S.; Tosti, M. E.; Mele, A. Dig Liver Dis. 2005, 37(4), 260-8.

The characteristics of the invention being disclosed in the preceding description, the subsequent drawings and claims can be of importance both singularly and in arbitrary combination for the implementation of the invention in its different embodiments.
The foregoing description of preferred embodiments of the invention has been presented for the purposes of illustration and description. It is not intended to be exhaustive or to limit the invention to the precise form described, and many modifications and variations are possible in light of the teaching above. The embodiments were chosen and described in order to best explain the principles of the invention and its practical applications to thereby enable others skilled in the art to best utilize the invention in various embodiments and with various modifications as are suited to the particular use contemplated invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. A biomarker selected from a first group consisting of Amy 1, Apo Al, Carbx, Casp, AFP, ApoM, SAP, Fib-a, Fib-b, Fib-g, ApoE, A2MG, A2MG isoform, Serpin, Clusterin, MHC-fB, SAP isoform, or from a second group consisting of Gpx3, properidin, MUP1, HMW-K, Lifr-p, Orm 1, MBL-A, MBP-C,
wherein the biomarker is regulated by EGF overexpression in a subject.

2. Biomarker as claimed in claim 1 selected from a first group consisting of Amy 1, Apo Al, Carbx, Casp, Fib-a, Fib-b, Fib-g, Clusterin, MHC-fB, SAP isoform or from a second group consisting of HMW-K, Lifr-p, Orm 1, MBL-A, MBP-C.

3. Biomarker as claimed in claim 1 selected from a first group consisting of AFP, ApoE, ApoM, or from a second group consisting of Gpx3, A2MG, A2MG isoform, SAP.

4. A composition for qualifying the EGFR kinase activity in a subject suffering from or being susceptible to cancer, in particular by an *in vitro* body fluid analysis, comprising an effective amount of at least one biomarker selected from the first group according to one of the claims 1-3 or an effective amount of at least one biomarker selected from the second group according to one of the claims 1-3.

5. Composition as claimed in claim 4 comprising an effective amount of at least one biomarker selected from the first group according to one of the claims 1-3 and an effective amount of at least one biomarker selected from the second group according to one of the claims 1-3.

6. Use of a composition as claimed in one of the claims 4-5 for the production of a diagnostic agent, in particular of a diagnostic standard for body fluid analysis.

7. Use as claimed in claim 6 for the production of a diagnostic agent for qualifying the EGFR kinase activity in a subject suffering from or being susceptible to cancer, in particular cancer of the liver, lung, breast, colon, prostate, bladder, head and neck, ovary or brain.

8. Use as claimed in one of the claims 6-7 for the production of a diagnostic agent for predicting or monitoring the response of a cancer patient to a method of treating cancer comprising administering an EGFR kinase modulator.

9. A kit for qualifying the EGFR kinase activity in a subject suffering from or being susceptible to cancer, in particular for predicting or monitoring the response of a cancer patient to a method of treating cancer comprising administering an EGFR kinase modulator, comprising at least one standard (1) indicative of the body fluid level of a biomarker selected from the first group according to one of the claims 1-3 in normal individuals or individuals having cancer associated with increased EGFR kinase activity and/or at least one standard (2) indicative of the body fluid level of a biomarker selected from the second group according to one of the claims 1-3 in normal individuals or individuals having cancer associated with increased EGFR kinase activity, and instructions for the use of the kit.

10. The kit as claimed in claim 9, wherein the at least one standard (1) comprises an indicative amount of at least one biomarker selected from the first group according to one of the claims 1-3 and/or wherein the at least one standard (2) comprises an indicative amount of at least one biomarker selected from the second group according to one of the claims 1-3.

11. The kit as claimed in one of the claims 9-10, comprising a mixture of the at least one standard (1) and the at least one standard (2), in particular the composition according to claim 5.

12. The kit as claimed in one of the claims 9-11, further comprising a lysis buffer according to one of the claims 23-25 and/or a digesting buffer according to one of the claims 23-25.

13. The kit as claimed in one of the claims 9-12, further comprising at least one antibody specific for a biomarker selected from the first group according to one of the claims 1-3 and/or at least one antibody specific for a biomarker selected from the second group according to one of the claims 1-3, and reagents effective to detect said biomarker(s) in a serum sample.

14. The kit as claimed in claim 13, wherein the at least one antibody is polyclonal.

15. The kit as claimed in one of the claims 13-14 comprising at least one labelled secondary antibody specific for the at least one antibody of claim 9-10.

16. A method of qualifying the EGFR kinase activity in a subject, comprising determining in a body fluid sample of a subject suffering from or being susceptible to cancer at least one biomarker selected from the first group according to one of the claims 1-3 and/or at least one biomarker selected from the second group according to one of the claims 1-3,
wherein the body fluid level of the at least one biomarker of said first group being significantly higher and/or the body fluid level of the at least one biomarker of said second group being significantly lower than the level of said biomarker(s) in the body fluid of subjects without cancer associated with increased activity of EGFR is indicative of induced EGFR kinase activity in the subject.

17. Method as claimed in claim 16 for predicting the response of a cancer patient to a method of treating cancer comprising administering an EGFR kinase modulator,
wherein the body fluid level of the at least one biomarker of said first group being significantly higher and/or the body fluid level of the at least one biomarker of said second group being significantly lower than the level of said biomarker(s) in the body fluid of subjects without cancer associated with increased activity of EGFR is indicative that the subject will respond therapeutically to a method of treating cancer comprising administering an EGFR kinase modulator.

18. Method as claimed in claim 16 for monitoring the therapeutically response of a cancer patient to a method of treating cancer comprising administering an EGFR kinase modulator, wherein the body fluid level of the at least one biomarker of said first group before and after the treatment and/or the body fluid level of the at least one biomarker of said second group before and after the treatment is determined, and a significant decrease of said body fluid level(s) of the at least one biomarker of said first group and/or a significant increase of said body fluid level(s) of the at least one biomarker of said second group after the treatment is indicative that the cancer patient therapeutically responds to the administration of the EGFR kinase modulator.

19. The method as claimed in one of the claims 16-18, wherein an immunoassay is performed, in particular by using the kit as claimed in one of the claims 9-15.

20. The method as claimed in claim 19, wherein at least one antibody specific for a biomarker selected from the first group according to claim 2 and/or at least one antibody specific for a biomarker selected from the second group according to claim 2, and reagents effective to detect said biomarker(s) in a serum sample is used for the immunoassay.

21. The method as claimed in one of the claims 16-18, wherein a peptide mass fingerprinting is performed, in particular by using the kit as claimed in one of the claims 9-12.

22. The method as claimed in claim 21, comprising the steps of
- isolating a serum sample from a blood sample of a subject suffering from or being susceptible to cancer;
- adding lysis buffer to the serum sample;
- separating the proteins of the lysed serum sample by 2-DE gel electrophoresis;
- excising from the gel at least one sample containing a protein of interest;
- adding digesting buffer to the at least one excised sample;
- determining the amount of the at least one protein of interest by analyzing the at least one digest mixture by mass spectrometry.

23. The method as claimed in claim 22, wherein
- the subject is a human patient or non-human transgenic animal; and/or
- the serum sample is isolated by centrifuging the blood sample; and/or
- the 2-DE is performed by using two different pH gradients; and/or
- the lysis buffer comprises (a) at least one buffer component, (b) at least one chaotrope, (c) at least one detergens, (d) at least one reducing agent (e) at least one carrier ampholyte, (f) at least one ribonuclease; and/or
- the protein of interest is a biomarker selected from the first group according to one of the claims 1-3 or a biomarker selected from the second group according to one of the claims 1-3; and/or
- the digesting buffer comprises a bicarbonate compound and a protease; and/or
- wherein the mass spectrometry is selected from the group consisting of MALDI-TOF and ESI-TOF.

24. The method as claimed in one of the claims 22- 23, wherein
- the subject is suffering from or is susceptible to cancer in particular cancer of the liver, lung, breast, colon, prostate, bladder, head and neck, ovary or brain.; and/or
- the 2-DE is performed by using the pH gradients 3-10 and 4-7; and/or
- the lysis buffer is an aqueous solution of (a) at least one buffer compound selected from the group consisting of Tris and HEPES, (b) at least one chaotrope selected from the group consisting of urea and thiourea, (c) at least one detergens selected from the group consisting of CHAPS and SDS, (d) at least one reducing agent selected from the group consisting of DTT and TCEP, (e) at least one carrier ampholyte selected from the group consisting of biolyte 5-7 and biolyte 3-10, (f) at least one ribonuclease selected from the group consisting of endonuclease and exonuclease; and/or
- the protein of interest is a biomarker selected from the first group according to one of the claims 2-3 or a biomarker selected from the second group according to one of the claims 2-3; and/or
- the digesting buffer is an aqueous solution of at least one bicarbonate compound selected from the group consisting of ammonium bicarbonate and sodium bicarbonate and of at least one serine protease, in particular selected from the group consisting of trypsin, chymotrypsin and elastase; and/or
- the mass spectrometry is performed by MALDI-TOF; and/or
- a tandem mass spectrometer is used; and/or
- a matrix is used for the mass spectrometry selected from the group consisting of 3,5-dimethoxy-4-hydroxycinnamic acid, α-cyano-4-hydroxycinnamic acid and 2,5-dihydroxybenzoic acid.

25. The method as claimed in one of the claims 22-24, wherein
- the subject is a transgenic mouse, in particular a mouse whose genome comprises a non natural IgEGF sequence; and/or
- the lysis buffer is an aqueous solution of (a) Tris; (b) urea and thiourea, (c) CHAPS, (d) DTT, (e) biolyte 3-10, (f) endonuclease; and/or
- the serum sample is calibrated or the serum samples are equilibrated to a predefined protein concentration by adding the lysis buffer; and/or
- the protein of interest is a biomarker selected from the first group according to claim 3 or a biomarker selected from the second group according to claim 3; and/or
- the digesting buffer is an aqueous solution of ammonium bicarbonate and trypsin; and/or
- a MALDI-TOF/TOF spectrometry is performed; and/or
- a matrix is used for the mass spectrometry selected from the group consisting of α-cyano-4-hydroxycinnamic acid.

26. The method as claimed in one of the claims 22-25, further comprising the steps of
- determining the protein concentration of the serum sample, in particular by the Bradford method; and/or
- freezing and thawing the serum sample before the lysis buffer is added; and/or
- staining the gel after the 2-DE, in particular by using coomassie blue; and/or
- destaining the exised sample; and/or
- shrinking, in particular by adding acetonitrile, and drying of the excised sample before the digesting buffer is added; and/or
- using a peptide calibration standard for the mass spectrometry.

27. A procedure to screen for and to identify drugs against cancer associated with an increased EGFR kinase activity comprising determining in a body fluid sample of a transgenic cancer mouse being treated with a compound to be tested, in particular of a mouse whose genome comprises a non natural IgEGF sequence, at least one biomarker selected from the first group according to one of the claims 1-3 and/or at least one biomarker selected from the second group according to one of the claims 1-3, wherein the body fluid level of the at least one biomarker of said first group being significantly lower and/or the body fluid level of the at least one biomarker of said second group being significantly higher than the level of said biomarker(s) in the body fluid of an untreated transgenic cancer mouse is indicative of the therapeutic effect of said compound as a EGFR kinase modulator.

28. The procedure as claimed in claim 27, wherein the method as claimed in claim 18, in particular according to one of the claims 19-26, is used.
